# EUROPEAN PATENT SPECIFICATION

(11) **EP 1 045 926 B1**
(45) Date of publication and mention of the grant of the patent: **14.06.2006**
(21) Application number: 98962084.4
(22) Date of filing: 11.12.1998
(51) Int. Cl.: C12Q 1/68, C07K 7/08

(54) **GENE FAMILY WITH TRANSFORMATION MODULATING ACTIVITY**
EINE FAMILIE VON GENEN MIT TRANSFORMATIONSMODULIERENDER AKTIVITÄT
FAMILLE DE GENES POSSEDANT UNE ACTIVITE DE MODULATION DE LA TRANSFORMATION

(30) Priority: 12.12.1997 US 69677 P
(43) Date of publication of application: 25.10.2000
(73) Proprietor: THE JOHNS HOPKINS UNIVERSITY, Baltimore, MD 21205-2109 (US)
(72) Inventor: PASTERNACK, Gary, R., Baltimore, MD 21210-1913 (US); KOCHEAVAR, Gerald, J., College Station, TX 77845 (US); BRODY, Jonathan, R., Potomac, MD 20854 (US); KADKOL, Shrihari, S., Ellicott City, MD 21043 (US)
(74) Representative: Tombling, Adrian George
(86) International application number: PCT/US1998/026433
(87) International publication number: WO 1999/029906

(56) References cited:
- WO-A-94/03594
- WO-A-96/10092
- DATABASE EMBL(EMHUM) EMBL,Heidelberg Accession Number U71084, 3 April 1997 REBEL J.M.J. ET AL.: "Human pp32 pseudogene" XP002106402 cited in the application
- DATABASE EMBL(EMHUM) EMBL, Heidelberg Accession Number AF008216, 8 January 1998 KOCHEVAR G.J. ET AL.: "Homo sapiens candidate tumor suppressor pp32r1 gene" XP002106403

### SEQUENCE LISTING

<110> Pasternack, Gary R.
   Kocheavar, Gerald J.
   Brody, Jonathan R.
   Kodkol, Shrihari S.
   The Johns Hopkins University
<120> GENE FAMILY WITH TRANSFORMATION MODULATING ACTIVITY
<130> 062482.0168
<140> PCT/US98/26433
   <141> 1998-12-11
<150> 60/069,677
   <151> 1997-12-12
<160> 51
<170> PatentIn Ver. 2.0 **·** beta
<210> 1
   **<211> 5785**
   <212> DNA
   <213> Homo sapiens
<220>
   <221> CDS
   <222> (4453)..(5154)
<400> 1
   aagctttcct gatctctaaa tcaaggtcag ctccctaagc tcttggctcc cgtactgaaa 60
   cttttcttta tgtaactctc ataaacacat agcataatgt tttgcatgtt tttcttccct 120
   atcagttgca agttccagca gagctgatat attttcattt cattcgctac tatagcccta 180
   gagcctgaca tagtttctgg ctgtgaatgc tcaataaata tttgtttaat tgagtagaaa 240
   cataaagtat ctatttcatt gaaggaaaga ataattagct acatttttct ttttcttgcc 300
   ttaatatttg aggaatttgc ttatatgtca taataaaaaa gttaaagcct tatacattat 360
   actaaggaat ttggacatta aattcaagct agcctttcta taaacaaaat actgaatttc 420
   tgtccctaaa tttgttcctt ccctattctt ccccattgag atgacaccaa atccctctag 480
   ctgctcaaac caagtacccg tatgttattc ttaattatct ctttaccttg cttctcatat 540
   gcaatttgtt aacaagtcat cttcagtctg tatccattat tctccctttc cagaccacca 600
   acatgtcttg actatactgc tacaatagcc tcccaactct tgtcctactt aaaattcatt 660
   gtaaaaaatc agtcttggcc gggcacggtg gctcacacct ataatcccag cactttggga 720
   gtcccaggcg ggcgggtcac gaggtcaaga gatggagacc atcatggcca acatggtgaa 780
   accctgtctc tactataaat acaaaaaaat tatctgggtg tggtggcaca tgcctgtaat 840
   cccaactact agggaggctg aggcaggaga atcgcttgaa cctgggaggc ggaggttgca 900
   gtgagccgag atcgcaccat tgcactccag cctggcaaca gagcgagact ccatcccaaa 960
   acaaaacaaa acaaaaccat gtaaaacatg tctgtaaaac atgtcagatt tcgtgttcag 1020
   aagtcttaca tgtcttttca ttatgctaag ataaaaccca aatgcatttt cttggtttct 1080
   aaagccaaga aaataagagt tgctttcagc aaccttgttt cttccgccat gcttttccct 1140
   agctcactct ttttaggcaa gtcgacctga ttttctttct gttagtctgt ttctgcctcg 1200
   tggtctggct ttctttctgt tagtctgttt ccacctcgtg gtcttggtcc tggctcttca 1260
   ttctgcctgg aatgctctcc actccagatc cttactagat cttagctcag tcatcaccct 1320
   cgcaggaaga tcttccaacc attcacctgc atacacctat ggctgctccc tagagaacat 1380
   cattctgttt tcttcacttc ctagcactta ctgctttctg aaattatcta ctttgattgt 1440
   ttatttcttt ctttactctt actaggatac ctgggtcatt aaaggaggga tatttctctc 1500
   ttatttactg ttataaactt aatgcttagg ctgtagaagt tatacaatat ttgaagaata 1560
   aatcgttaaa tgtataacat ttttgaagaa agataattgt gggatccatt tagtttgcaa 1620
   acatttgatc tgtgtgttag acagaaggcc atggtaaagg acaaagacat attttatagg 1680
   actgtaccct gaaaaataaa taaacttgaa ccagttatac aagacttatg tgcaggaaac 1740
   aggtaccagt tatatttaga aatggtaaat caccttctaa gcataactca gagcacaata 1800
   tattagaggg tagagagaga agtgcgtctt agatattggt aatcatatta ggactgacgc 1860
   catccttgat ttttcttctg ggaaacagct caaaatgact atttaatgtt tacaatgata 1920
   tcttgcatct tgccagtaaa taatataata gacactagga atccaaattg taagatgaac 1980
   aagtctttat agagggagag ccaaatacac aataaataac acaaggtggt aaatgcagta 2040
   atacaaacat acataccatg cataggagtg cagagaaggt gtgcttctcc gaatgcagtc 2100
   acccagaaag tccttctgta gaaagggata tcttaaatgg tgcttaaagg aaaagtaacc 2160
   aaaggcaact aaagattgca aggaggtccc aggaaaaagc aaaagaacca aaggtacata 2220
   ggcacaaaag tagcctgcct tcctgggaac ttccaatagt ttgctggagc acacagttag 2280
   aagtactgtg ccatgggagc aaagactgaa gacatatgca ggttcaaggg cacagagccc 2340
   catatatgtc atgataagat attgggaagc cactggggag ctactgaaac tttaagcagg 2400
   gaaataaaat tgtcatatct acaccttaga aatttgattt ttttctcttc ttttatcttc 2460
   tcttctcctc tcttctctct ctctctctct ctctctctct gtgtgtgtgt gtgtgtgtgt 2520
   gtgtgtgtgt gacagagtcc tgctctgtca cccaggctgg agtgtagtgg agtgatctcc 2580
   gcttactgca gtctctgcct ctcaagcgat tccctgcctc agcctcccga gtagctggga 2640
   ttacaggcgg gctctacaac agctggctaa cttttgtatt ttttggtaac aaccaggttt 2700
   taccatgttg gccaggctgg tcttgaactc ctgacctcag gtgatctgcc tgccttggct 2760
   ttccaaagtg ctgggattac aggcgtgagc caccctgcct ggtgtagaag tttgattttg 2820
   atgtcagtgt ggtagatgaa tttgtgggaa gcaaaacaag atagagttca atgacagtga 2880
   aaagtttatt gtataagcta tataaaagaa aatgttgaag gtttgaaatc cattagtggc 2940
   agtaagggtg tacagaacga aactatttga gaagtacaca aggcaagtct tactttcaag 3000
   gcagtttatg taagctcatt caattgtctc agtgttcttg ctatgtgtgg gttataggat 3060
   ttggaacata tgatcaatct gagcacacat cagtaaactg aataggatta ttaaaatcca 3120
   caagcatttt actagtggaa tctgtgatat tttctagcta ctcttgcttg ttttatttga 3180
   atcttttgct catatcctat agtaaagatt tcaggaaata tatttttatt tgcctagaat 3240
   tttagccttt tagttttttg aatctattgc tcatattctt atagtaagag tttcagggaa 3300
   tgtatttcta tttgtctgga attttagcct ttcaggtttt tgagcccctc ttttgcttat 3360
   gggacatagt atgagacaag atgaaatgat acttctattc ccaattcact gatggggaaa 3420
   atgaagcaaa aaatgttatt cactcaaggc ttctgccatg tttcctggtg gaattacggc 3480
   tcagacacaa atttcctaat gcctgtgctg ctaacttctc aatagaacac tatattaatt 3540
   tatcttcttc ctgagtgttt ttccacaaat cccatagcct gtgaaaagat tgttttaggg 3600
   aaatattatt tttaatatag catattttgt caatgtggga cataggacta gtacctgctg 3660
   aaaaccatct catgatcctt gtgtaagaac taattcacac tagaaatact attttccttg 3720
   ctcattaaaa acataaatgt ctcagaaagt aaaaaattat tcctctctaa ataaacatac 3780
   atgccactca aattttattc ctctaccact tgccgtatct aaacctagtt agatactttg 3840
   gttttaggta taatctgaca gaacagatac aaccaagatc acattgtgag tcagaagtgg 3900
   aaaattcata attcatgatg ataccaataa aagatagatt tagcttttta caggatgttt 3960
   ttggcatttt attctttcat ttgaggggag atctcaccaa aatatgtctt tcatggttca 4020
   ttgtgttatt taatttctgt gatgcatatt ctcaggttac tttaaaccta gtctatagat 4080
   tcaaagatat cccgtgtcag gtctctaaaa gtaaaaagaa aaatgggtac ttgtgaaggc 4140
   tgattcacag taagtagtgt agaggggagt gccttgtgta ttcacaaatt atcaacgtga 4200
   gcatcagata agattttctt tagtcacaca cacctacctt cttactagga agatccatat 4260
   acttgaataa ttgttctgct tgacccaggt tacttatcag tccctttatt ataatatttg 4320
   taaatattgg ggctcgagaa ccgagcggag ctggttgagt cttcaaagtc ctaaaacgtg 4380
   cggccgtggg ttcgaggttt attgattgaa ttcggctggc acgagagcct ctgcagacag 4440
   agagcgcgag ag atg gag atg ggc aga cgg att cat tca gag ctg cgg aac 4491
   Met Glu Met Gly Arg Arg Ile His Ser Glu Leu Arg Asn
   1 5 10
   agg gcg ccc tct gat gtg aaa gaa ctt gcc ctg gac aac agt cgg tcg 4539
   Arg Ala Pro Ser Asp Val Lys Glu Leu Ala Leu Asp Asn Ser Arg Ser
   15 20 25
   aat gaa ggc aaa ctc gaa gcc ctc aca gat gaa ttt gaa gaa ctg gaa 4587
   Asn Glu Gly Lys Leu Glu Ala Leu Thr Asp Glu Phe Glu Glu Leu Glu
   30 35 40 45
   ttc tta agt aaa atc aac gga ggc ctc acc tca atc tca gac tta cca 4635
   Phe Leu Ser Lys Ile Asn Gly Gly Leu Thr Ser Ile Ser Asp Leu Pro
   50 55 60
   aag tta aag ttg aga aag ctt gaa cta aga gtc tca ggg ggc ctg gaa 4683
   Lys Leu Lys Leu Arg Lys Leu Glu Leu Arg Val Ser Gly Gly Leu Glu
   65 70 75
   gta ttg gca gaa aag tgt cca aac ctc acg cat cta tat tta agt ggc 4731
   Val Leu Ala Glu Lys Cys Pro Asn Leu Thr His Leu Tyr Leu Ser Gly
   80 85 90
   aac aaa att aaa gac ctc agc aca ata gag cca ctg aaa cag tta gaa 4779
   Asn Lys Ile Lys Asp Leu Ser Thr Ile Glu Pro Leu Lys Gln Leu Glu
   95 100 105
   aac ctc aag agc tta gac ctt ttc aat tgc gag gta acc aac ctg aac 4827
   Asn Leu Lys Ser Leu Asp Leu Phe Asn Cys Glu Val Thr Asn Leu Asn
   110 115 120 125
   gac tac gga gaa aac gtg ttc aag ctt ctc ctg caa ctc aca tat ctc 4875
   Asp Tyr Gly Glu Asn Val Phe Lys Leu Leu Leu Gln Leu Thr Tyr Leu
   130 135 140
   gac agc tgt tac tgg gac cac aag gag gcc cct tac tca gat att gag 4923
   Asp Ser Cys Tyr Trp Asp His Lys Glu Ala Pro Tyr Ser Asp Ile Glu
   145 150 155
   gac cac gtg gag ggc ctg gat gac gag gag gag ggt gag cat gag gag 4971
   Asp His Val Glu Gly Leu Asp Asp Glu Glu Glu Gly Glu His Glu Glu
   160 165 170
   gag tat gat gaa gat gct cag gta gtg gaa gat gag gag ggc gag gag 5019
   Glu Tyr Asp Glu Asp Ala Gln Val Val Glu Asp Glu Glu Gly Glu Glu
   175 180 185
   gag gag gag gaa ggt gaa gag gag gac gtg agt gga ggg gac gag gag 5067
   Glu Glu Glu Glu Gly Glu Glu Glu Asp Val Ser Gly Gly Asp Glu Glu
   190 195 200 205
   gat gaa gaa ggt tat aac gat gga gag gta gat ggc gag gaa gat gaa 5115
   Asp Glu Glu Gly Tyr Asn Asp Gly Glu Val Asp Gly Glu Glu Asp Glu
   210 215 220
   gaa gag ctt ggt gaa gaa gaa agg ggt cag aag cga aaa tgagaacctg 5164
   Glu Glu Leu Gly Glu Glu Glu Arg Gly Gln Lys Arg Lys
   225 230
   aagatgaggg agaagatgat gactaagtag aataacctat tttgaaaaat tcctattgtg 5224
   atttgactgt ttttacccat atcccctccc ccctccaatc ctgccccctg aaacttactt 5284
   ttttctgatt gtaacattgc tgtgggaatg agacgggaaa agtgtactgg gggttgtgga 5344
   gggagggagg gcaggaggcg gtggactaaa atactatttt tactgccaaa taaaataata 5404
   tttgtaaata ttaactggga tactagcttt gtagaatgat tactattaat tattctctct 5464
   ctctttttat ttttttacac attctattct tttaagtata gtccttttag tccaaggaaa 5524
   aggcactaca atccacttat taatgcttgc tactgtgttc aagtaaaata agctccagga 5584
   tttaacaaaa agaggaaaga aaatatttac aatgaaaatg ttgctaaaaa tttaaaacaa 5644
   attacagtaa atgtattgtt aaagcaaatt ctatttttaa aatttattaa taaggaaata 5704
   atttgctaaa gcaaattttt ggaaaaataa taatgcactt tatacttgat tttatttatt 5764
   aaaacaatga tttataagct t 5785
<210> 2
   <211> 234
   <211> 234
   <212> PRT
   <213> Homo sapiens
<400> 2
   Met Glu Met Gly Arg Arg Ile His Ser Glu Leu Arg Asn Arg Ala Pro
   1 5 10 15
   Ser Asp Val Lys Glu Leu Ala Leu Asp Asn Ser Arg Ser Asn Glu Gly
   20 25 30
   Lys Leu Glu Ala Leu Thr Asp Glu Phe Glu Glu Leu Glu Phe Leu Ser
   35 40 45
   Lys Ile Asn Gly Gly Leu Thr Ser Ile Ser Asp Leu Pro Lys Leu Lys
   50 55 60
   Leu Arg Lys Leu Glu Leu Arg Val Ser Gly Gly Leu Glu Val Leu Ala
   65 70 75 80
   Glu Lys Cys Pro Asn Leu Thr His Leu Tyr Leu Ser Gly Asn Lys Ile
   85 90 95
   Lys Asp Leu Ser Thr Ile Glu Pro Leu Lys Gln Leu Glu Asn Leu Lys
   100 105 110
   Ser Leu Asp Leu Phe Asn Cys Glu Val Thr Asn Leu Asn Asp Tyr Gly
   115 120 125
   Glu Asn Val Phe Lys Leu Leu Leu Gln Leu Thr Tyr Leu Asp Ser Cys
   130 135 140
   Tyr Trp Asp His Lys Glu Ala Pro Tyr Ser Asp Ile Glu Asp His Val
   145 150 155 160
   Glu Gly Leu Asp Asp Glu Glu Glu Gly Glu His Glu Glu Glu Tyr Asp
   165 170 175
   Glu Asp Ala Gln Val Val Glu Asp Glu Glu Gly Glu Glu Glu Glu Glu
   180 185 190
   Glu Gly Glu Glu Glu Asp Val Ser Gly Gly Asp Glu Glu Asp Glu Glu
   195 200 205
   Gly Tyr Asn Asp Gly Glu Val Asp Gly Glu Glu Asp Glu Glu Glu Leu
   210 215 220
   Gly Glu Glu Glu Arg Gly Gln Lys Arg Lys
   225 230
<210> 3
   <211> 889
   <212> DNA
   <213> Homo sapiens
<400> 3
   gggttcgagg tttattgatt gaattcggct ggcacgagag cctctgcaga cagagagcgc 60
   gagagatgga gatgggcaga cggattcatt cagagctgcg gaacagggcg ccctctgatg 120
   tgaaagaact tgccctggac aacagtcggt cgaatgaagg caaactcgaa gccctcacag 180
   atgaatttga agaactggaa ttcttaagta aaatcaacgg aggcctcacc tcaatctcag 240
   acttaccaaa gttaaagttg agaaagcttg aactaagagt ctcagggggc ctggaagtat 300
   tggcagaaaa gtgtccaaac ctcacgcatc tatatttaag tggcaacaaa attaaagacc 360
   tcagcacaat agagccactg aaacagttag aaaacctcaa gagcttagac cttttcaatt 420
   gcgaggtaac caacctgaac gactacggag aaaacgtgtt caagcttctc ctgcaactca 480
   catatctcga cagctgttac tgggaccaca aggaggcccc ttactcagat attgaggacc 540
   acgtggaggg cctggatgac gaggaggagg gtgagcatga ggaggagtat gatgaagatg 600
   ctcaggtagt ggaagatgag gagggcgagg aggaggagga ggaaggtgaa gaggaggacg 660
   tgagtggagg ggacgaggag gatgaagaag gttataacga tggagaggta gatggcgagg 720
   aagatgaaga agagcttggt gaagaagaaa ggggtcagaa gcgaaaatga gaacctgaag 780
   atgagggaga agatgatgac taagtagaat aacctatttt gaaaaattcc tattgtgatt 840
   tgactgtttt tacccatatc ccctcccccc tccaatcctg ccccctgaa 889
<210> 4
   <211> 907
   <212> DNA
   <213> Homo sapiens
<220>
   <221> CDS
   <222> (66)..(455)
<400> 4
   gggttcgggg tttattgatt gaattcggct ggcgcgggag cctctgcaga gagagagcgc 60
   gagag atg gag atg ggc aga cgg att cat tta gag ctg cgg aac ggg acg 110
         Met Glu Met Gly Arg Arg Ile His Leu Glu Leu Arg Asn Gly Thr
           1               5                  10                  15
   ccc tct gat gtg aaa gaa ctt gtc ctg gac aac agt cgg tcg aat gaa   158
   Pro Ser Asp Val Lys Glu Leu Val Leu Asp Asn Ser Arg Ser Asn Glu
                   20                  25                  30
   ggc aaa ctc gaa ggc ctc aca gat gaa ttt gaa gaa ctg gaa ttc tta   206
   Gly Lys Leu Glu Gly Leu Thr Asp Glu Phe Glu Glu Leu Glu Phe Leu
               35                  40                  45
   agt aca atc aac gta ggc ctc acc tca atc gca aac tta cca aag tta   254
   Ser Thr Ile Asn Val Gly Leu Thr Ser Ile Ala Asn Leu Pro Lys Leu
           50                  55                  60
   aac aaa ctt aag aag ctt gaa cta agc agt aac aga gcc tca gtg ggc   302
   Asn Lys Leu Lys Lys Leu Glu Leu Ser Ser Asn Arg Ala Ser Val Gly
       65                  70                  75
   cta gaa gta ttg gca gaa aag tgt cca aac ctc ata cat cta aat tta   350
   Leu Glu Val Leu Ala Glu Lys Cys Pro Asn Leu Ile His Leu Asn Leu
   80                  85                  90                  95
   agt ggc aac aaa att aaa gac ctc agc aca ata gag ccc ctg aaa aag   398
   Ser Gly Asn Lys Ile Lys Asp Leu Ser Thr Ile Glu Pro Leu Lys Lys
                   100                 105                 110
   tta gaa aac ctc gag agc tta gac ctt ttc act tgc gag gta acc aac   446
   Leu Glu Asn Leu Glu Ser Leu Asp Leu Phe Thr Cys Glu Val Thr Asn
               115                 120                 125
   ctg aac aac tactgagaga agatgttcaa gctcctcctg caactcacat 495
   Leu Asn Asn
   130
   atctcaacgg ctgtgacccg gatgacaagg aggcccctaa ctcggatggt gagggctttg 555
   tggagtgcct ggatgacaag gaggaggatg aggatgagga ggagtatgat gaagatgctc 615
   aggtaatgga agatgaggag gacgaggatg aggaggagga acgtgaagag gaggacgtga 675
   gtggagacga ggaggagaag gatgaaggtt ataacaatgg agaggtagat gatgaggaag 735
   atgaagaaga gcttggtgaa gaagaaaggg gtcagaagcg aaaataagaa actgaagatg 795
   agggagaaga cgatgcctaa gtggaataat ctattttgaa aaattccttt tgtgatttta 855
   ctgtttttag ccgtaccccc tctccccccc cactctaatc ctgccccctg aa 907
<210> 5
   <211> 130
   <212> PRT
   <213> Homo sapiens
<400> 5
   Met Glu Met Gly Arg Arg Ile His Leu Glu Leu Arg Asn Gly Thr Pro
   1 5 10 15
   Ser Asp Val Lys Glu Leu Val Leu Asp Asn Ser Arg Ser Asn Glu Gly
   20 25 30
   Lys Leu Glu Gly Leu Thr Asp Glu Phe Glu Glu Leu Glu Phe Leu Ser
   35 40 45
   Thr Ile Asn Val Gly Leu Thr Ser Ile Ala Asn Leu Pro Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn
   130
<210> 6
   <211> 907
   <212> DNA
   <213> Homo sapiens
<400> 6
   gggttcgggg tttattgatt gaattcggct ggcacgagag cctctgcaga cagagagcgc 60
   gagagacgga gatgggcaga cggattcatc tagagctgcg gaacagggcg ccctctgatg 120
   tgaaagaact tgccctggac aacagtcggt cgaatgaagg caaactcgaa gccctcacag 180
   atgaatttga agaactggaa ttcttaagta aaatcaacgg aggcctcacc tcaatctcag 240
   acttaccaaa gttaaacaag ttgagaaagc ttgaactaag cagtaacaga gtctcagggg 300
   gcctggaagt attggcagaa aagtgtccaa acctcacgca tctatattta agtggcaaca 360
   aaattaaaga cctcagcaca atagagccac tgaaacagtt agaaaacctc aagagcttag 420
   accttttcaa ttgcgaggta accaacctga acgactacgg agaaaacgtg ttcaagcttc 480
   tcctgcaact cacatatctc gacagctgtt actgggacca caaggaggcc ccttactcag 540
   atattgaggc ccacgtggag ggcctggatg acgaggagga gggtgagcat gaggaggagt 600
   atgatgaaga tgctcaggta gtggaagatg aggagggcga ggaggaggag gaggaaggtg 660
   aagaggagga cgtgagtgga ggggacgagg aggatgaaga aggttataac gatggagagg 720
   tagatggcga ggaagatgaa gaagagcttg gtgaagaaga aaggggtcag aagcgaaaat 780
   gagaacctga agatgaggga gaagatgatg actaagtaga ataacctatt ttgaaaaatt 840
   cctattgtga tttgactgtt tttacccata tcccctctcc cccccccctc taatcctgcc 900
   ccctgaa 907
<210> 7
   <211> 905
   <212> DNA
   <213> Homo sapiens
<220>
   <221> CDS
   <222> (64)..(453)
<400> 7
   gggttcgggg tttattggtt gaattccgct ggctcaggag cctctgcaga gaaagcgtga 60
   gag atg gag atg ggc aaa tgg att cat tta gag ctg cgg aac agg acg   108
       Met Glu Met Gly Lys Trp Ile His Leu Glu Leu Arg Asn Arg Thr
        1               5                  10                  15
   ccc tcc gat gtg aaa gaa ctt ttc ctg gac aac agt cag tca aat gaa   156
   Pro Ser Asp Val Lys Glu Leu Phe Leu Asp Asn Ser Gln Ser Asn Glu
                   20                  25                  30
   ggc aaa ttg gaa ggc ctc aca gat gaa ttt gaa gaa ctg gaa tta tta   204
   Gly Lys Leu Glu Gly Leu Thr Asp Glu Phe Glu Glu Leu Glu Leu Leu
               35                  40                  45
   aat aca atc aac ata ggc ctc acc tca att gca aac ttg cca aag tta   252
   Asn Thr Ile Asn Ile Gly Leu Thr Ser Ile Ala Asn Leu Pro Lys Leu
           50                  55                  60
   aac aaa ctt aag aag ctt gaa cta agc agt aac aga gcc tca gtg ggc   300
   Asn Lys Leu Lys Lys Leu Glu Leu Ser Ser Asn Arg Ala Ser Val Gly
       65                  70                  75
   cta gaa gta ttg gca gaa aag tgt cca aac ctc ata cat cta aat tta   348
   Leu Glu Val Leu Ala Glu Lys Cys Pro Asn Leu Ile His Leu Asn Leu
   80                  85                  90                  95
   agt ggc aac aaa att aaa gac ctc agc aca ata gag ccc ctg aaa aag   396
   Ser Gly Asn Lys Ile Lys Asp Leu Ser Thr Ile Glu Pro Leu Lys Lys
                   100                 105                 110
   tta gaa aac ctc gag agc tta gac ctt ttc act tgc gag gta acc aac    444
   Leu Glu Asn Leu Glu Ser Leu Asp Leu Phe Thr Cys Glu Val Thr Asn
               115                     120                 125
   ctg aac aac tactgagaaa agatgttcaa gctcctcctg caactcacat           493
   Leu Asn Asn
           130
   atctcaacgg ctgtgacccg gatgacaagg aggcccctaa ctcggatggt gagggctatg 553
   tggagtgcct ggatgacaag gaggaggatg aggatgagga ggagtatgat gaagatgctc 613
   aggtaatgga agatgaggag gacgaggatg aggaggagga acgtgaagag gaggacgtga 673
   gtggagacga ggaggagaag gatgaaggtt ataacaatgg agaggtagat gatgaggaag 733
   atgaagaaga gcttggtgaa gaagaaaggg gtcagaagcg aaaataagaa actgaagatg 793
   agggagaaga cgatgcctaa gtggaataat ctattttgaa aaattccttt tgtgatttta 853
   ctgtttttag ccgtatcccc tctccccccc cactctaatc ctgccccctg aa          905
<210> 8
   <211> 130
   <212> PRT
   <213> Homo sapiens
<400> 8
   Met Glu Met Gly Lys Trp Ile His Leu Glu Leu Arg Asn Arg Thr Pro
   1 5 10 15
   Ser Asp Val Lys Glu Leu Phe Leu Asp Asn Ser Gln Ser Asn Glu Gly
   20 25 30
   Lys Leu Glu Gly Leu Thr Asp Glu Phe Glu Glu Leu Glu Leu Leu Asn
   35 40 45
   Thr Ile Asn Ile Gly Leu Thr Ser Ile Ala Asn Leu Pro Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn
   130
<210> 9
   <211> 907
   <212> DNA
   <213> Homo sapiens
<220>
   <221> CDS
   <222> (66)..(812)
<400> 9
   gggttcgggg tttattgatt gaattccgcc ggcgcgggag cctctgcaga gagagagcgc 60
   gagag atg gag atg ggc aga cgg att cat tta gag ctg cgg aac agg acg 110
   Met Glu Met Gly Arg Arg Ile His Leu Glu Leu Arg Asn Arg Thr
   1 5 10 15
   ccc tct gat gtg aaa gaa ctt gtc ctg gac aac agt cgg tcg aat gaa 158
   Pro Ser Asp Val Lys Glu Leu Val Leu Asp Asn Ser Arg Ser Asn Glu
   20 25 30
   ggc aaa ctc gaa ggc ctc aca gat gaa ttt gaa gaa ctg gaa ttc tta 206
   Gly Lys Leu Glu Gly Leu Thr Asp Glu Phe Glu Glu Leu Glu Phe Leu
   35 40 45
   agt aca atc aac gta ggc ctc acc tca atc gca aac ttg cca aag tta 254
   Ser Thr Ile Asn Val Gly Leu Thr Ser Ile Ala Asn Leu Pro Lys Leu
   50 55 60
   aac aaa ctt aag aag ctt gaa cta agc agt aac aga gcc tca gtg ggc 302
   Asn Lys Leu Lys Lys Leu Glu Leu Ser Ser Asn Arg Ala Ser Val Gly
   65 70 75
   cta gaa gta ttg gca gaa aag tgt cca aac ctc ata cat cta aat tta 350
   Leu Glu Val Leu Ala Glu Lys Cys Pro Asn Leu Ile His Leu Asn Leu
   80 85 90 95
   agt ggc aac aaa att aaa gac ctc agc aca ata gag cca ctg aaa aag 398
   Ser Gly Asn Lys Ile Lys Asp Leu Ser Thr Ile Glu Pro Leu Lys Lys
   100 105 110
   tta gaa aac ctc aag agc tta gac ctt tcc aat tgc gag gta acc aac 446
   Leu Glu Asn Leu Lys Ser Leu Asp Leu Ser Asn Cys Glu Val Thr Asn
   115 120 125
   ctg aac gac tac cga gaa aat gtg ttc aag ctc ctc ccg caa ctc aca 494
   Leu Asn Asp Tyr Arg Glu Asn Val Phe Lys Leu Leu Pro Gln Leu Thr
   130 135 140
   tat ctc gac ggc tat gac cgg gac gac aag gag gcc cct gac tcg gat 542
   Tyr Leu Asp Gly Tyr Asp Arg Asp Asp Lys Glu Ala Pro Asp Ser Asp
   145 150 155
   gct gag ggc tac gtg gag ggc ctg gat gat gag gag gag gat gag gat 590
   Ala Glu Gly Tyr Val Glu Gly Leu Asp Asp Glu Glu Glu Asp Glu Asp
   160 165 170 175
   gag gag gag tat gat gaa gat gct cag gta gta gaa gat gag gag gac 638
   Glu Glu Glu Tyr Asp Glu Asp Ala Gln Val Val Glu Asp Glu Glu Asp
   180 185 190
   gag gat gag gag gag gaa ggt gaa gag gag gac gtg agt gga gag gag 686
   Glu Asp Glu Glu Glu Glu Gly Glu Glu Glu Asp Val Ser Gly Glu Glu
   195 200 205
   gag gag gat gaa gaa ggt tat aac gat gga gag gta gat gac gag gaa 734
   Glu Glu Asp Glu Glu Gly Tyr Asn Asp Gly Glu Val Asp Asp Glu Glu
   210 215 220
   gat gaa gaa gag ctt ggt gaa gaa gaa agg ggt cag aag cga aaa cga 782
   Asp Glu Glu Glu Leu Gly Glu Glu Glu Arg Gly Gln Lys Arg Lys Arg
   225 230 235
   gaa cct gaa gat gag gga gaa gat gat gac taagtggaat aacctatttt     832
   Glu Pro Glu Asp Glu Gly Glu Asp Asp Asp
   240                 245
   gaaaaattcc tattgtgatt tgactgtttt tacccatatc ccctctcccc cccccctcta 892
   atcctgcccc ctgaa 907
<210> 10
   <211> 249
   <212> PRT
   <213> Homo sapiens
<400> 10
   Met Glu Met Gly Arg Arg Ile His Leu Glu Leu Arg Asn Arg Thr Pro
   1 5 10 15
   Ser Asp Val Lys Glu Leu Val Leu Asp Asn Ser Arg Ser Asn Glu Gly
   20 25 30
   Lys Leu Glu Gly Leu Thr Asp Glu Phe Glu Glu Leu Glu Phe Leu Ser
   35 40 45
   Thr Ile Asn Val Gly Leu Thr Ser Ile Ala Asn Leu Pro Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Lys Ser Leu Asp Leu Ser Asn Cys Glu Val Thr Asn Leu
   115 120 125
   Asn Asp Tyr Arg Glu Asn Val Phe Lys Leu Leu Pro Gln Leu Thr Tyr
   130 135 140
   Leu Asp Gly Tyr Asp Arg Asp Asp Lys Glu Ala Pro Asp Ser Asp Ala
   145 150 155 160
   Glu Gly Tyr Val Glu Gly Leu Asp Asp Glu Glu Glu Asp Glu Asp Glu
   165 170 175
   Glu Glu Tyr Asp Glu Asp Ala Gln Val Val Glu Asp Glu Glu Asp Glu
   180 185 190
   Asp Glu Glu Glu Glu Gly Glu Glu Glu Asp Val Ser Gly Glu Glu Glu
   195 200 205
   Glu Asp Glu Glu Gly Tyr Asn Asp Gly Glu Val Asp Asp Glu Glu Asp
   210 215 220
   Glu Glu Glu Leu Gly Glu Glu Glu Arg Gly Gln Lys Arg Lys Arg Glu
   225 230 235 240
   Pro Glu Asp Glu Gly Glu Asp Asp Asp
   245
<210> 11
   <211> 905
   <212> DNA
   <213> Homo sapiens
<220>
   <221> CDS
   <222> (64)..(810)
<400> 11
   gggttcgggg tttattggtt gaattccgct ggctcaggag cctctgcaga gaaagcgtga 60
   gag atg gag atg ggc aaa tgg att cat tta gag ctg cgg aac agg acg 108
   Met Glu Met Gly Lys Trp Ile His Leu Glu Leu Arg Asn Arg Thr
   1 5 10 15
   ccc tcc gat gtg aaa gaa ctt ttc ctg gac aac agt cag tca aat gaa 156
   Pro Ser Asp Val Lys Glu Leu Phe Leu Asp Asn Ser Gln Ser Asn Glu
   20 25 30
   ggc aaa ttg gaa ggc ctc aca gat gaa ttt gaa gaa ctg gaa tta tta 204
   Gly Lys Leu Glu Gly Leu Thr Asp Glu Phe Glu Glu Leu Glu Leu Leu
   35 40 45
   aat aca atc aac ata ggc ctc acc tca att gca aac ttg cca aag tta 252
   Asn Thr Ile Asn Ile Gly Leu Thr Ser Ile Ala Asn Leu Pro Lys Leu
   50 55 60
   aac aaa ctt aag aag ctt gaa cta agc agt aac aga gcc tca gtg ggc 300
   Asn Lys Leu Lys Lys Leu Glu Leu Ser Ser Asn Arg Ala Ser Val Gly
   65 70 75
   cta gaa gta ttg gca gaa aag tgt cca aac ctc ata cat cta aat tta 348
   Leu Glu Val Leu Ala Glu Lys Cys Pro Asn Leu Ile His Leu Asn Leu
   80 85 90 95
   agt ggc aac aaa att aaa gac ctc agc aca ata gag ccc ctg aaa aag 396
   Ser Gly Asn Lys Ile Lys Asp Leu Ser Thr Ile Glu Pro Leu Lys Lys
   100 105 110
   tta gaa aac ctc gag agc tta gac ctt ttc act tgc gag gta acc aac 444
   Leu Glu Asn Leu Glu Ser Leu Asp Leu Phe Thr Cys Glu Val Thr Asn
   115 120 125
   ctg aac aac tac cga gaa aat gtg ttc aag ctc ctc ccg caa ctc aca 492
   Leu Asn Asn Tyr Arg Glu Asn Val Phe Lys Leu Leu Pro Gln Leu Thr
   130 135 140
   tat ctc gac ggc tat gac cgg gac gac aag gag gcc cct gac tcg gat 540
   Tyr Leu Asp Gly Tyr Asp Arg Asp Asp Lys Glu Ala Pro Asp Ser Asp
   145 150 155
   gct gag ggc tac gtg gag ggc ctg gat gat gag gag gag gat gag gat 588
   Ala Glu Gly Tyr Val Glu Gly Leu Asp Asp Glu Glu Glu Asp Glu Asp
   160 165 170 175
   gag gag gag tat gat gaa gat gct cag gta gtg gaa gac gag gag gac 636
   Glu Glu Glu Tyr Asp Glu Asp Ala Gln Val Val Glu Asp Glu Glu Asp
   180 185 190
   gag gat gag gag gag gaa ggt gaa gag gag gac gtg agt gga gag gag 684
   Glu Asp Glu Glu Glu Glu Gly Glu Glu Glu Asp Val Ser Gly Glu Glu
   195 200 205
   gag gag gat gaa gaa ggt tat aac gat gga gag gta gat gac gag gaa 732
   Glu Glu Asp Glu Glu Gly Tyr Asn Asp Gly Glu Val Asp Asp Glu Glu
   210 215 220
   gat gaa gaa gag ctt ggt gaa gaa gaa agg ggt cag aag cga aaa cga 780
   Asp Glu Glu Glu Leu Gly Glu Glu Glu Arg Gly Gln Lys Arg Lys Arg
   225 230 235
   gaa cct gaa gat gag gga gaa gat gat gac taagtggaat aacctatttt 830
   Glu Pro Glu Asp Glu Gly Glu Asp Asp Asp
   240 245
   gaaaaattcc tattgtgatt tgactgtttt tacccatatc ccctctcccc cccccctcta 890
   atcctgcccc ctgaa 905
<210> 12
   <211> 249
   <212> PRT
   <213> Homo sapiens
<400> 12
   Met Glu Met Gly Lys Trp Ile His Leu Glu Leu Arg Asn Arg Thr Pro
   1 5 10 15
   Ser Asp Val Lys Glu Leu Phe Leu Asp Asn Ser Gln Ser Asn Glu Gly
   20 25 30
   Lys Leu Glu Gly Leu Thr Asp Glu Phe Glu Glu Leu Glu Leu Leu Asn
   35 40 45
   Thr Ile Asn Ile Gly Leu Thr Ser Ile Ala Asn Leu Pro Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn Tyr Arg Glu Asn Val Phe Lys Leu Leu Pro Gln Leu Thr Tyr
   130 135 140
   Leu Asp Gly Tyr Asp Arg Asp Asp Lys Glu Ala Pro Asp Ser Asp Ala
   145 150 155 160
   Glu Gly Tyr Val Glu Gly Leu Asp Asp Glu Glu Glu Asp Glu Asp Glu
   165 170 175
   Glu Glu Tyr Asp Glu Asp Ala Gln Val Val Glu Asp Glu Glu Asp Glu
   180 185 190
   Asp Glu Glu Glu Glu Gly Glu Glu Glu Asp Val Ser Gly Glu Glu Glu
   195 200 205
   Glu Asp Glu Glu Gly Tyr Asn Asp Gly Glu Val Asp Asp Glu Glu Asp
   210 215 220
   Glu Glu Glu Leu Gly Glu Glu Glu Arg Gly Gln Lys Arg Lys Arg Glu
   225 230 235 240
   Pro Glu Asp Glu Gly Glu Asp Asp Asp
   245
<210> 13
   <211> 907
   <212> DNA
   <213> Homo sapiens
<220>
   <221> CDS
   <222> (66)..(812)
<400> 13
   gggttcgggg tttattgatt gaattccgcc ggcgcgggag cctctgcaga gagagagcgc 60
   gagag atg gag atg ggc aga cgg att cat tta gag ctg cgg aac agg acg 110
         Met Glu Met Gly Arg Arg Ile His Leu Glu Leu Arg Asn Arg Thr
         1               5                   10                  15
   ccc tct gat gtg aaa gaa ctt gtc ctg gac aac agt cgg tcg aat gaa   158
   Pro Ser Asp Val Lys Glu Leu Val Leu Asp Asn Ser Arg Ser Asn Glu
                   20                  25                  30
   ggc aaa ctc gaa ggc ctc aca gat gaa ttt gaa gaa ctg gaa ttc tta   206
   Gly Lys Leu Glu Gly Leu Thr Asp Glu Phe Glu Glu Leu Glu Phe Leu
               35                  40                  45
   agt aca atc aac gta ggc ctc acc tca atc gca aac tta cca aag tta   254
   Ser Thr Ile Asn Val Gly Leu Thr Ser Ile Ala Asn Leu Pro Lys Leu
           50                  55                  60
   aac aaa ctt aag aag ctt gaa cta agc gat aac aga gtc tca ggg ggc   302
   Asn Lys Leu Lys Lys Leu Glu Leu Ser Asp Asn Arg Val Ser Gly Gly
       65                  70                  75
   ctg gaa gta ttg gca gaa aag tgt ccg aac ctc acg cat cta aat tta   350
   Leu Glu Val Leu Ala Glu Lys Cys Pro Asn Leu Thr His Leu Asn Leu
   80 85 90 95
   agt ggc aac aaa att aaa gac ctc agc aca ata gag cca ctg aaa aag 398
   Ser Gly Asn Lys Ile Lys Asp Leu Ser Thr Ile Glu Pro Leu Lys Lys
   100 105 110
   tta gaa aac ctc aag agc tta gac ctt ttc aat tgc gag gta acc aac 446
   Leu Glu Asn Leu Lys Ser Leu Asp Leu Phe Asn Cys Glu Val Thr Asn
   115 120 125
   ctg aac gac tac cga gaa aat gtg ttc aag ctc ctc ccg caa ctc aca 494
   Leu Asn Asp Tyr Arg Glu Asn Val Phe Lys Leu Leu Pro Gln Leu Thr
   130 135 140
   tat ctc gac ggc tat gac cgg gac gac aag gag gcc cct gac tcg gat 542
   Tyr Leu Asp Gly Tyr Asp Arg Asp Asp Lys Glu Ala Pro Asp Ser Asp
   145 150 155
   gct gag ggc tac gtg gag ggc ctg gat gat gag gag gag gat gag gat 590
   Ala Glu Gly Tyr Val Glu Gly Leu Asp Asp Glu Glu Glu Asp Glu Asp
   160 165 170 175
   gag gag gag tat gat gaa gat gct cag gta gtg gaa gac gag gag gac 638
   Glu Glu Glu Tyr Asp Glu Asp Ala Gln Val Val Glu Asp Glu Glu Asp
   180 185 190
   gag gat gag gag gag gaa ggt gaa gag gag gac gtg agt gga gag gag 686
   Glu Asp Glu Glu Glu Glu Gly Glu Glu Glu Asp Val Ser Gly Glu Glu
   195 200 205
   gag gag gat gaa gaa ggt tat aac gat gga gag gta gat gac gag gaa 734
   Glu Glu Asp Glu Glu Gly Tyr Asn Asp Gly Glu Val Asp Asp Glu Glu
   210 215 220
   gat gaa gaa gag ctt ggt gaa gaa gaa agg ggt cag aag cga aaa cga 782
   Asp Glu Glu Glu Leu Gly Glu Glu Glu Arg Gly Gln Lys Arg Lys Arg
   225 230 235
   gaa cct gaa gat gag gga gaa gat gat gac taagtggaat aacctatttt 832
   Glu Pro Glu Asp Glu Gly Glu Asp Asp Asp
   240 245
   gaaaaattcc tattgtgatt tgactgtttt tacccatatc ccctctcccc cccccctcta 892
   atcctgcccc ctgaa 907
<210> 14
   <211> 249
   <212> PRT
   <213> Homo sapiens
<400> 14
   Met Glu Met Gly Arg Arg Ile His Leu Glu Leu Arg Asn Arg Thr Pro
   1 5 10 15
   Ser Asp Val Lys Glu Leu Val Leu Asp Asn Ser Arg Ser Asn Glu Gly
   20 25 30
   Lys Leu Glu Gly Leu Thr Asp Glu Phe Glu Glu Leu Glu Phe Leu Ser
   35 40 45
   Thr Ile Asn Val Gly Leu Thr Ser Ile Ala Asn Leu Pro Lys Leu Asn
   50 55 60
   Lys Leu Lys Lys Leu Glu Leu Ser Asp Asn Arg Val Ser Gly Gly Leu
   65 70 75 80
   Glu Val Leu Ala Glu Lys Cys Pro Asn Leu Thr His Leu Asn Leu Ser
   85 90 95
   Gly Asn Lys Ile Lys Asp Leu Ser Thr Ile Glu Pro Leu Lys Lys Leu
   100 105 110
   Glu Asn Leu Lys Ser Leu Asp Leu Phe Asn Cys Glu Val Thr Asn Leu
   115 120 125
   Asn Asp Tyr Arg Glu Asn Val Phe Lys Leu Leu Pro Gln Leu Thr Tyr
   130 135 140
   Leu Asp Gly Tyr Asp Arg Asp Asp Lys Glu Ala Pro Asp Ser Asp Ala
   145 150 155 160
   Glu Gly Tyr Val Glu Gly Leu Asp Asp Glu Glu Glu Asp Glu Asp Glu
   165 170 175
   Glu Glu Tyr Asp Glu Asp Ala Gln Val Val Glu Asp Glu Glu Asp Glu
   180 185 190
   Asp Glu Glu Glu Glu Gly Glu Glu Glu Asp Val Ser Gly Glu Glu Glu
   195 200 205
   Glu Asp Glu Glu Gly Tyr Asn Asp Gly Glu Val Asp Asp Glu Glu Asp
   210 215 220
   Glu Glu Glu Leu Gly Glu Glu Glu Arg Gly Gln Lys Arg Lys Arg Glu
   225 230 235 240
   Pro Glu Asp Glu Gly Glu Asp Asp Asp
   245
<210> 15
   <211> 895
   <212> DNA
   <213> Homo sapiens
<220>
   <221> CDS
   <222> (66)..(767)
<400> 15
   gggttcgggg tttattgatt gaattcggct ggcacgagag cctctgcaga cagagagcgc 60
   gagag atg gag atg ggc aga cgg att cat tca gag ctg cgg aac agg gcg 110
         Met Glu Met Gly Arg Arg Ile His Ser Glu Leu Arg Asn Arg Ala
         1               5                   10                  15
   ccc tct gat gtg aaa gaa ctt gcc ctg gac aac agt cgg tcg aat gaa   158
   Pro Ser Asp Val Lys Glu Leu Ala Leu Asp Asn Ser Arg Ser Asn Glu
                   20                  25                  30
   ggc aaa ctc gaa gcc ctc aca gat gaa ttt gaa gaa ctg gaa ttc tta   206
   Gly Lys Leu Glu Ala Leu Thr Asp Glu Phe Glu Glu Leu Glu Phe Leu
               35                  40                  45
   agt aaa atc aac gga ggc ctc acc tca atc tca gac tta cca aag tta   254
   Ser Lys Ile Asn Gly Gly Leu Thr Ser Ile Ser Asp Leu Pro Lys Leu
           50                  55                  60
   aag ttg aga aag ctt gaa cta aga gtc tca ggg ggc ctg gaa gta ttg   302
   Lys Leu Arg Lys Leu Glu Leu Arg Val Ser Gly Gly Leu Glu Val Leu
   65 70 75
   gca gaa aag tgt cca aac ctc acg cat cta tat tta agt ggc aac aaa 350
   Ala Glu Lys Cys Pro Asn Leu Thr His Leu Tyr Leu Ser Gly Asn Lys
   80 85 90 95
   att aaa gac ctc agc aca ata gag cca ctg aaa cag tta gaa aac ctc 398
   Ile Lys Asp Leu Ser Thr Ile Glu Pro Leu Lys Gln Leu Glu Asn Leu
   100 105 110
   aag agc tta gac ctt ttc aat tgc gag gta acc aac ctg aac gac tac 446
   Lys Ser Leu Asp Leu Phe Asn Cys Glu Val Thr Asn Leu Asn Asp Tyr
   115 120 125
   gga gaa aac gtg ttc aag ctt ctc ctg caa ctc aca tat ctc gac agc 494
   Gly Glu Asn Val Phe Lys Leu Leu Leu Gln Leu Thr Tyr Leu Asp Ser
   130 135 140
   tgt tac tgg gac cac aag gag gcc cct tac tca gat att gag gac cac 542
   Cys Tyr Trp Asp His Lys Glu Ala Pro Tyr Ser Asp Ile Glu Asp His
   145 150 155
   gtg gag ggc ctg gat gac gag gag gag ggt gag cat gag gag gag tat 590
   Val Glu Gly Leu Asp Asp Glu Glu Glu Gly Glu His Glu Glu Glu Tyr
   160 165 170 175
   gat gaa gat gct cag gta gtg gaa gat gag gag ggc gag gag gag gag 638
   Asp Glu Asp Ala Gln Val Val Glu Asp Glu Glu Gly Glu Glu Glu Glu
   180 185 190
   gag gaa ggt gaa gag gag gac gtg agt gga ggg gac ggg gag gat gaa 686
   Glu Glu Gly Glu Glu Glu Asp Val Ser Gly Gly Asp Gly Glu Asp Glu
   195 200 205
   gaa ggt tat aac gat gga gag gta gat ggc gag gaa gat gaa gaa gag 734
   Glu Gly Tyr Asn Asp Gly Glu Val Asp Gly Glu Glu Asp Glu Glu Glu
   210 215 220
   ctt ggt gaa gaa gaa agg ggt cag aag cga aaa tgagaacctg aagatgaggg 787
   Leu Gly Glu Glu Glu Arg Gly Gln Lys Arg Lys
   225 230
   agaagatgat gactaagtag aataacctat tttgaaaaat tcctattgtg atttgactgt 847
   ttttacccat atcccatctc ccccccccct ctaatcctgc cccctgaa 895
<210> 16
   <211> 234
   <212> PRT
   <213> Homo sapiens
<400> 16
   Met Glu Met Gly Arg Arg Ile His Ser Glu Leu Arg Asn Arg Ala Pro
   1 5 10 15
   Ser Asp Val Lys Glu Leu Ala Leu Asp Asn Ser Arg Ser Asn Glu Gly
   20 25 30
   Lys Leu Glu Ala Leu Thr Asp Glu Phe Glu Glu Leu Glu Phe Leu Ser
   35 40 45
   Lys Ile Asn Gly Gly Leu Thr Ser Ile Ser Asp Leu Pro Lys Leu Lys
   50 55 60
   Leu Arg Lys Leu Glu Leu Arg Val Ser Gly Gly Leu Glu Val Leu Ala
   65 70 75 80
   Glu Lys Cys Pro Asn Leu Thr His Leu Tyr Leu Ser Gly Asn Lys Ile
   85 90 95
   Lys Asp Leu Ser Thr Ile Glu Pro Leu Lys Gln Leu Glu Asn Leu Lys
   100 105 110
   Ser Leu Asp Leu Phe Asn Cys Glu Val Thr Asn Leu Asn Asp Tyr Gly
   115 120 125
   Glu Asn Val Phe Lys Leu Leu Leu Gln Leu Thr Tyr Leu Asp Ser Cys
   130 135 140
   Tyr Trp Asp His Lys Glu Ala Pro Tyr Ser Asp Ile Glu Asp His Val
   145 150 155 160
   Glu Gly Leu Asp Asp Glu Glu Glu Gly Glu His Glu Glu Glu Tyr Asp
   165 170 175
   Glu Asp Ala Gln Val Val Glu Asp Glu Glu Gly Glu Glu Glu Glu Glu
   180 185 190
   Glu Gly Glu Glu Glu Asp Val Ser Gly Gly Asp Gly Glu Asp Glu Glu
   195 200 205
   Gly Tyr Asn Asp Gly Glu Val Asp Gly Glu Glu Asp Glu Glu Glu Leu
   210 215 220
   Gly Glu Glu Glu Arg Gly Gln Lys Arg Lys
   225 230
<210> 17
   <211> 905
   <212> DNA
   <213> Homo sapiens
<220>
   <221> CDS
   <222> (64)..(453)
<400> 17
   gggttcgggg tttattggtt gaattccgct ggctcgagag cctctggaga gaaagcgtga 60
   gag atg gag atg ggc aaa tgg att cat tta gag ctg cgg aac agg acg   108
       Met Glu Met Gly Lys Trp Ile His Leu Glu Leu Arg Asn Arg Thr
       1               5                   10                  15
   ccc tcc gat gtg aaa gaa ctt ttc ctg gac aac agt cag tca aat gaa   156
   Pro Ser Asp Val Lys Glu Leu Phe Leu Asp Asn Ser Gln Ser Asn Glu
                   20                  25                  30
   ggc aaa ttg gaa ggc ctc aca gat gaa ttt gag gaa ctg gaa tta tta   204
   Gly Lys Leu Glu Gly Leu Thr Asp Glu Phe Glu Glu Leu Glu Leu Leu
               35                  40                  45
   aat aca atc aac ata ggc ctc acc tca att gca aac ttg cca aag tta   252
   Asn Thr Ile Asn Ile Gly Leu Thr Ser Ile Ala Asn Leu Pro Lys Leu
           50                  55                  60
   aac aaa ctt aag aag ctt gaa cta agc agt aac aga gcc tca gtg ggc   300
   Asn Lys Leu Lys Lys Leu Glu Leu Ser Ser Asn Arg Ala Ser Val Gly
       65                  70                  75
   cta gaa gta ttg gca gaa aag tgt cca aac ctc ata cat cta aat tta   348
   Leu Glu Val Leu Ala Glu Lys Cys Pro Asn Leu Ile His Leu Asn Leu
   80 85 90 95
   agt ggc aac aaa att aaa gac ctc agc aca ata gag ccc ctg aaa aag 396
   Ser Gly Asn Lys Ile Lys Asp Leu Ser Thr Ile Glu Pro Leu Lys Lys
   100 105 110
   tta gaa aac ctt gag agc tta gac ctt ttc act tgc gag gta acc aac 444
   Leu Glu Asn Leu Glu Ser Leu Asp Leu Phe Thr Cys Glu Val Thr Asn
   115 120 125
   ctg aac aac tactgagaaa agatgttcaa gctcctcctg caactcacat 493
   Leu Asn Asn
   130
   atctcaacgg ctgtgacccg gatgacaagg aggcccctaa ctcggatggt gagggctacg 553
   tggagggcct ggacgatgag gaggaggatg aggatgagga ggagtatgat gaagatgctc 613
   aggtagtgga agacgaggag gacgaggatg aggaggagga aggtgaagag gaggacgtga 673
   gtggagagga ggaggaggat gaagaaggtt ataacgatgg agaggtagat gacgaggaag 733
   atgaagaaga gcttggtgaa gaagaaaggg gtcagaagcg aaaacgagaa cctgaagatg 793
   agggagaaga tgatgactaa gtggaataac ctattttgaa aaattcctat tgtgatttga 853
   ctgtttttag ccgtatcccc tctccccccc cactctaatc ctgccccctg aa 905
<210> 18
   <211> 130
   <212> PRT
   <213> Homo sapiens
<400> 18
   Met Glu Met Gly Lys Trp Ile His Leu Glu Leu Arg Asn Arg Thr Pro
   1 5 10 15
   Ser Asp Val Lys Glu Leu Phe Leu Asp Asn Ser Gln Ser Asn Glu Gly
   20 25 30
   Lys Leu Glu Gly Leu Thr Asp Glu Phe Glu Glu Leu Glu Leu Leu Asn
   35 40 45
   Thr Ile Asn Ile Gly Leu Thr Ser Ile Ala Asn Leu Pro Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn
   130
<210> 19
   <211> 905
   <212> DNA
   <213> Homo sapiens
<220>
   <221> CDS
   <222> (64)..(453)
<400> 19
   gggttcgggg tttattggtt gaattccgct ggctcaggag cctctgcaga gaaagcgtga 60
   gag atg gag atg ggc aaa tgg att cat tta gag ctg cgg aac agg acg   108
       Met Glu Met Gly Lys Trp Ile His Leu Glu Leu Arg Asn Arg Thr
       1               5                   10                  15
   ccc tcc gat gtg aaa gaa ctt ttc ctg gac aac agt cag tca aat gaa   156
   Pro Ser Asp Val Lys Glu Leu Phe Leu Asp Asn Ser Gln Ser Asn Glu
                   20                  25                  30
   ggc aaa ttg gaa ggc ctc aca gat gaa ttt gaa gaa ctg gaa tta tta   204
   Gly Lys Leu Glu Gly Leu Thr Asp Glu Phe Glu Glu Leu Glu Leu Leu
               35                  40                  45
   aat aca atc aac ata ggc ctc acc tca att gca aac ttg cca aag tta   252
   Asn Thr Ile Asn Ile Gly Leu Thr Ser Ile Ala Asn Leu Pro Lys Leu
           50                  55                  60
   aac aaa ctt aag aag ctt gaa cta agc agt aac aga gcc tca gtg ggc   300
   Asn Lys Leu Lys Lys Leu Glu Leu Ser Ser Asn Arg Ala Ser Val Gly
       65                  70                  75
   cta gaa gta ttg gca gaa aag tgt cca aac ctc ata cat cta aat tta   348
   Leu Glu Val Leu Ala Glu Lys Cys Pro Asn Leu Ile His Leu Asn Leu
   80                  85                  90                  95
   agt ggc aac aaa att aaa gac ctc agc aca ata gag ccc ctg aaa aag 396
   Ser Gly Asn Lys Ile Lys Asp Leu Ser Thr Ile Glu Pro Leu Lys Lys
   100 105 110
   tta gaa aac ctc gag agc tta gac ctt ttc act tgc gag gta acc aac 444
   Leu Glu Asn Leu Glu Ser Leu Asp Leu Phe Thr Cys Glu Val Thr Asn
   115 120 125
   ctg aac aac tactgagaaa agatgttcaa gctcctcctg caactcacat 493
   Leu Asn Asn
   130
   atctcaacgg ctgtgacccg gatgacaagg aggcccctaa ctcggatggt gagggctttg 553
   tggagtgcct ggatgacaag gaggaggatg aggatgagga ggagtatgat gaagatgctc 613
   aggtaatgga agatgaggag gacgaggatg aggaggagga acgtgaagag gaggacgtga 673
   gtggagacga ggaggagaag gatgaaggtt ataacaatgg agaggtagat gatgaggaag 733
   atgaagaaga gcttggtgaa gaagaaaggg gtcagaagcg aaaataagaa actgaagatg 793
   agggagaaga cgatgcctaa gtggaataat ctattttgaa aaattccttt tgtgatttta 853
   ctgtttttag ccgtatcccc tctccccccc cactctaatc ctgccccctg aa 905
<210> 20
   <211> 130
   <212> PRT
   <213> Homo sapiens
<400> 20
   Met Glu Met Gly Lys Trp Ile His Leu Glu Leu Arg Asn Arg Thr Pro
   1 5 10 15
   Ser Asp Val Lys Glu Leu Phe Leu Asp Asn Ser Gln Ser Asn Glu Gly
   20 25 30
   Lys Leu Glu Gly Leu Thr Asp Glu Phe Glu Glu Leu Glu Leu Leu Asn
   35 40 45
   Thr Ile Asn Ile Gly Leu Thr Ser Ile Ala Asn Leu Pro Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn
   130
<210> 21
   <211> 895
   <212> DNA
   <213> Homo sapiens
<220>
   <221> CDS
   <222> (66) .. (767)
<400> 21
   gggttcgggg tttattgatt gaattcggct ggcacgagag cctctgcaga cagagagcgc 60
   gagag atg gag atg ggc aga cgg att cat tca gag ctg cgg aac agg gcg 110
   ccc tct gat gtg aaa gaa ctt gtc ctg gac aac agt cgg tcg aat gaa   158
   ggc aaa ctc gaa gcc ctc aca gat gaa ttt gaa gaa ctg gaa ttc tta   206
   agt aaa atc aac gga ggc ctc acc tca atc tca gac tta cca aag tta   254
   aag ttg aga aag ctt gaa cta aaa gtc tca ggg ggc ctg gaa gta ttg   302
   gca gaa aag tgt cca aac ctc acg cat cta tat tta agt ggc aac aaa   350
   att aaa gac ctc agc aca ata gag cca ctg aaa cag tta gaa aac ctc   398
   aag agc tta gac ctt ttc aat tgc gag gta acc aac ctg aac gac tac    446
   gga gaa aac gtg ttc aag ctt ctc ctg caa ctc aca tat ctc gac agc    494
   tgt tac tgg gac cac aag gag gcc cct tac tca gat att gag gac cac    542
   gtg gag ggc ctg gat gac gag gag gag ggt gag cat gag gag gag tat    590
   gat gaa gat gct cag gta gtg gaa gat gag gag ggc gag gag gag gag    638
   gag gaa ggt gaa gag gag gac gtg agt gga ggg gac gag gag gat gaa    686
   gaa ggt tat aac gat gga gag gta gat ggc gag gaa gat gaa gaa gag    734
   ctt ggt gaa gaa gaa agg ggt cag aag cga aaa tgagaacctg aagatgaggg 787
   agaagatgat gactaagtag aataacctat tttgaaaaat tcctattgtg atttgactgt 847
   ttttacccat atcccccctc ccccccccct ctaatcctgc cccctgaa 895
<210> 22
   <211> 234
   <212> PRT
   <213> Homo sapiens
<400> 22
<210> 23
   <211> 895
   <212> DNA
   <213> Homo sapiens
<220>
   <221> CDS
   <222> (66)..(767)
<400> 23 agaagatgat gactaagtgg aataacctat tttgaaaaat tcctattgtg atttgactgt 847
   ttttacccat atcccctctc ccccccccct ctaatcctgc cccctgaa 895
<210> 24
   <211> 234
   <212> PRT
   <213> Homo sapiens
<400> 24
   Met Glu Met Gly Arg Arg Ile His Leu Glu Leu Arg Asn Arg Thr Pro
   1 5 10 15
   Ser Asp Val Lys Glu Leu Val Leu Asp Asn Ser Arg Ser Asn Glu Gly
   20 25 30
   Lys Leu Glu Gly Leu Thr Asp Glu Phe Glu Glu Leu Glu Phe Leu Ser
   35 40 45
   Thr Ile Asn Val Gly Leu Thr Ser Ile Ala Asn Leu Pro Lys Leu Lys
   50 55 60
   Leu Arg Lys Leu Glu Leu Arg Val Ser Gly Gly Leu Glu Val Leu Ala
   65 70 75 80
   Glu Lys Cys Pro Asn Leu Thr His Leu Tyr Leu Ser Gly Asn Lys Ile
   85 90 95
   Lys Asp Leu Ser Thr Ile Glu Pro Leu Lys Gln Leu Glu Asn Leu Lys
   100 105 110
   Ser Leu Asp Leu Phe Asn Cys Glu Val Thr Asn Leu Asn Asp Tyr Gly
   115 120 125
   Glu Asn Val Phe Lys Leu Leu Leu Gln Leu Thr Tyr Leu Asp Ser Cys
   130 135 140
   Tyr Trp Asp His Lys Glu Ala Pro Tyr Ser Asp Ile Glu Asp His Val
   145 150 155 160
   Glu Gly Leu Asp Asp Glu Glu Glu Gly Glu His Glu Glu Glu Tyr Asp
   165 170 175
   Glu Asp Ala Gln Val Val Glu Asp Glu Glu Gly Glu Glu Gly Glu Glu
   180 185 190
   Glu Gly Glu Glu Glu Asp Val Ser Gly Gly Asp Glu Glu Asp Glu Glu
   195 200 205
   Gly Tyr Asn Asp Gly Glu Val Asp Asp Glu Glu Asp Glu Glu Glu Leu
   210 215 220
   Gly Glu Glu Glu Arg Gly Gln Lys Arg Lys
   225 230
<210> 25
   <211> 907
   <212> DNA
   <213> Homo sapiens
<400> 25
   gggttcgggg tttattgatt gaattccgcc ggcgcgggag cctctgcaga gagagagcgc 60
   gagagatgga gatgggcaga cggattcatt tagagctgcg gaacaggacg ccctctgatg 120
   tgaaagaact tgtcctggac aacagtcggt cgaatgaagg caaactcgag ggcctcacag 180
   atgaatttga agaactggaa ttcttaagta caatcaacgt aggcctcacc tcaatcgcaa 240
   acttaccaaa gttaaacaaa cttaagaagc ttgaactaag cgataacaga gtctcagggg 300
   gcctggaagt attggcagaa aagtgtccga acctcacgca tctaaattta agtggcaaca 360
   aaattaaaga cctcagcaca atagagccac tgaaaaagtt agaaaacctc aagagcttag 420
   accttttcaa ttgcgaggta accaacctga acgactaccg agaaaatgtg ttcaagctcc 480
   tcccgcaact cacatatctc gacggctatg accgggacga caaggaggcc cctgactcgg 540
   atgctgaggg ctacgtggag ggcctggatg atgaggagga ggatgaggat gaggaggagt 600
   atgatgaaga tgctcaggta gtggaagacg aggaggacga ggatgaggag gaggaaggtg 660
   aagaggagga cgtgagtgga gaggaggagg aggatgaaga aggttataac gatggagagg 720
   tagatgacga ggaagatgaa gaagagcttg gtgaagaaga aaggggtcag aagcgaaaac 780
   gagaacctga agatgaggga gaagatgatg actaagtgga ataacctatt ttgaaaaatt 840
   cctattgtga tttgactgtt tttacccata tcccctctcc cccccccctc taatcctgcc 900
   ccctgaa 907
<210> 26
   <211> 905
   <212> DNA
   <213> Homo sapiens
<220>
   <221> CDS
   <222> (64)..(453)
<400> 26
   gggttcgggg tttattggtt gaattccgct ggctcaggag cctctgcaga gaaagcgtga 60
   gag atg gag atg ggc aaa tgg att cat tta gag ctg cgg aac agg acg 108
   Met Glu Met Gly Lys Trp Ile His Leu Glu Leu Arg Asn Arg Thr
   1 5 10 15
   ccc tcc gat gtg aaa gaa ctt ttc ctg gac aac agt cag tca aat gaa 156
   Pro Ser Asp Val Lys Glu Leu Phe Leu Asp Asn Ser Gln Ser Asn Glu
   20 25 30
   ggc aaa ttg gaa ggc ctc aca gat gaa ttt gaa gaa ctg gaa tta tta 204
   Gly Lys Leu Glu Gly Leu Thr Asp Glu Phe Glu Glu Leu Glu Leu Leu
   35 40 45
   aat aca atc aac ata ggc ctc acc tca att gca aac ttg cca aag tta 252
   Asn Thr Ile Asn Ile Gly Leu Thr Ser Ile Ala Asn Leu Pro Lys Leu
   50 55 60
   aac aaa ctt aag aag ctt gaa cta agc agt aac aga gcc tca gtg ggc 300
   Asn Lys Leu Lys Lys Leu Glu Leu Ser Ser Asn Arg Ala Ser Val Gly
   65 70 75
   cta gaa gta ttg gca gaa aag tgt cca aac ctc ata cat cta aat tta 348
   Leu Glu Val Leu Ala Glu Lys Cys Pro Asn Leu Ile His Leu Asn Leu
   80 85 90 95
   agt ggc aac aaa att aaa gac ctc agc aca ata gag ccc ctg aaa aag 396
   Ser Gly Asn Lys Ile Lys Asp Leu Ser Thr Ile Glu Pro Leu Lys Lys
   100 105 110
   tta gaa aac ctc gag agc tta gac ctt ttc act tgc gag gta acc aac 444
   Leu Glu Asn Leu Glu Ser Leu Asp Leu Phe Thr Cys Glu Val Thr Asn
   115 120 125
   ctg aac aac tactgagaaa agatgttcaa gctcctcctg caactcacat 493
   Leu Asn Asn
   130
   atctcaacgg ctgtgacccg gatgacaagg aggcccctaa ctcggatggt gagggctttg 553
   tggagtgcct ggatgacaag gaggaggatg aggatgagga ggagtatgat gaagatgctc 613
   aggtaatgga agatgaggag gacgaggatg aggaggagga acgtgaagag gaggacgtga 673
   gtggagacga ggaggagaag gatgaaggtt ataacaatgg agaggtagat gatgaggaag 733
   atgaagaaga gcttggtgaa gaagaaaggg gtcagaagcg aaaataagaa actgaagatg 793
   agggagaaga cgatgcctaa gtggaataat ctattttgaa aaattccttt tgtgatttta 853
   ctgtttttag ccgtatcccc tctccccccc cactctaatc ctgccccctg aa 905
<210> 27
   <211> 130
   <212> PRT
   <213> Homo sapiens
<400> 27
   Met Glu Met Gly Lys Trp Ile His Leu Glu Leu Arg Asn Arg Thr Pro
   1 5 10 15
   Ser Asp Val Lys Glu Leu Phe Leu Asp Asn Ser Gln Ser Asn Glu Gly
   20 25 30
   Lys Leu Glu Gly Leu Thr Asp Glu Phe Glu Glu Leu Glu Leu Leu Asn
   35 40 45
   Thr Ile Asn Ile Gly Leu Thr Ser Ile Ala Asn Leu Pro Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn
   130
<210> 28
   <211> 907
   <212> DNA
   <213> Homo sapiens
<220>
   <221> CDS
   <222> (66)..(812)
<400> 28
   gggttcgggg tttattgatt gaattccgcc ggcgcgggag cctctgcaga gagagagcgc 60
   gagag atg gag atg ggc aga cgg att cat cta gag ctg cgg aac agg acg 110
   Met Glu Met Gly Arg Arg Ile His Leu Glu Leu Arg Asn Arg Thr
   1 5 10 15
   ccc tct gat gtg aaa gaa ctt gtc ctg gtc aac agt cgg tcg aat gaa 158
   Pro Ser Asp Val Lys Glu Leu Val Leu Val Asn Ser Arg Ser Asn Glu
   20 25 30
   ggc aaa ctc gaa ggc ctc aca gat gaa ttt gaa gaa ctg gaa ttc tta 206
   Gly Lys Leu Glu Gly Leu Thr Asp Glu Phe Glu Glu Leu Glu Phe Leu
   35 40 45
   agt aca atc aac gta ggc ctc acc tca atc gca aac tta cca aag tta 254
   Ser Thr Ile Asn Val Gly Leu Thr Ser Ile Ala Asn Leu Pro Lys Leu
   50 55 60
   aac aaa ctt aag aag ctt gaa cta agc gat aac aga gtc tca ggg ggc 302
   Asn Lys Leu Lys Lys Leu Glu Leu Ser Asp Asn Arg Val Ser Gly Gly
   65 70 75
   cta gaa gta ttg gca gaa aag tgt ccg aac ctc acg cat cta aat tta 350
   Leu Glu Val Leu Ala Glu Lys Cys Pro Asn Leu Thr His Leu Asn Leu
   80 85 90 95
   agt ggc aac aaa att aaa gac ctc agc aca ata gag cca ctg aaa aag 398
   Ser Gly Asn Lys Ile Lys Asp Leu Ser Thr Ile Glu Pro Leu Lys Lys
   100 105 110
   tta gaa aac ctc aag agc tta gac ctt ttc aat tgc gag gta acc aac 446
   Leu Glu Asn Leu Lys Ser Leu Asp Leu Phe Asn Cys Glu Val Thr Asn
   115 120 125
   ctg aac gac tac cga gaa aat gtg ttc aag ctc ctc ccg caa ctc aca 494
   Leu Asn Asp Tyr Arg Glu Asn Val Phe Lys Leu Leu Pro Gln Leu Thr
   130 135 140
   tat ctc gac ggc tat gac cgg gac gac aag gag gcc cct gac tcg gat 542
   Tyr Leu Asp Gly Tyr Asp Arg Asp Asp Lys Glu Ala Pro Asp Ser Asp
   145 150 155
   gct gag ggc tac gtg gag ggc ctg gat gat gag gag gag gat gag gat 590
   Ala Glu Gly Tyr Val Glu Gly Leu Asp Asp Glu Glu Glu Asp Glu Asp
   160 165 170 175
   gag gag gag tat gat gaa gat gct cag gta gtg gaa gac gag gag gac 638
   Glu Glu Glu Tyr Asp Glu Asp Ala Gln Val Val Glu Asp Glu Glu Asp
   180 185 190
   gag gat gag gag gag gaa ggt gaa gag gag gac gtg agt gga gag gag 686
   Glu Asp Glu Glu Glu Glu Gly Glu Glu Glu Asp Val Ser Gly Glu Glu
   195 200 205
   gag gag gat gaa gaa ggt tat aac gat gga gag gta gat gac gag gaa 734
   Glu Glu Asp Glu Glu Gly Tyr Asn Asp Gly Glu Val Asp Asp Glu Glu
   210 215 220
   gat gaa gaa gag ctt ggt gaa gaa gaa agg ggt cag aag cga aaa cga 782
   Asp Glu Glu Glu Leu Gly Glu Glu Glu Arg Gly Gln Lys Arg Lys Arg
   225 230 235
   gaa cct gaa gat gag gga gaa gat gat gac taagtggaat aacctatttt 832
   Glu Pro Glu Asp Glu Gly Glu Asp Asp Asp
   240 245
   gaaaaattcc tattgtgatt tgactgtttt tacccatatc ccctctcccc cccccctcta 892
   atcctgcccc ctgaa 907
<210> 29
   <211> 249
   <212> PRT
   <213> Homo sapiens
<400> 29
   Met Glu Met Gly Arg Arg Ile His Leu Glu Leu Arg Asn Arg Thr Pro
   1 5 10 15
   Ser Asp Val Lys Glu Leu Val Leu Val Asn Ser Arg Ser Asn Glu Gly
   20 25 30
   Lys Leu Glu Gly Leu Thr Asp Glu Phe Glu Glu Leu Glu Phe Leu Ser
   35 40 45
   Thr Ile Asn Val Gly Leu Thr Ser Ile Ala Asn Leu Pro Lys Leu Asn
   50 55 60
   Lys Leu Lys Lys Leu Glu Leu Ser Asp Asn Arg Val Ser Gly Gly Leu
   65 70 75 80
   Glu Val Leu Ala Glu Lys Cys Pro Asn Leu Thr His Leu Asn Leu Ser
   85 90 95
   Gly Asn Lys Ile Lys Asp Leu Ser Thr Ile Glu Pro Leu Lys Lys Leu
   100 105 110
   Glu Asn Leu Lys Ser Leu Asp Leu Phe Asn Cys Glu Val Thr Asn Leu
   115 120 125
   Asn Asp Tyr Arg Glu Asn Val Phe Lys Leu Leu Pro Gln Leu Thr Tyr
   130 135 140
   Leu Asp Gly Tyr Asp Arg Asp Asp Lys Glu Ala Pro Asp Ser Asp Ala
   145 150 155 160
   Glu Gly Tyr Val Glu Gly Leu Asp Asp Glu Glu Glu Asp Glu Asp Glu
   165 170 175
   Glu Glu Tyr Asp Glu Asp Ala Gln Val Val Glu Asp Glu Glu Asp Glu
   180 185 190
   Asp Glu Glu Glu Glu Gly Glu Glu Glu Asp Val Ser Gly Glu Glu Glu
   195 200 205
   Glu Asp Glu Glu Gly Tyr Asn Asp Gly Glu Val Asp Asp Glu Glu Asp
   210 215 220
   Glu Glu Glu Leu Gly Glu Glu Glu Arg Gly Gln Lys Arg Lys Arg Glu
   225 230 235 240
   Pro Glu Asp Glu Gly Glu Asp Asp Asp
   245
<210> 30
   <211> 907
   <212> DNA
   <213> Homo sapiens
<220>
   <221> CDS
   <222> (66)..(455)
<400> 30
   gggttcgggg tttattgatt gaattccgcc ggcgcgggag cctctgcaga gagagagcgc 60
   gagag atg gag atg ggc aga cgg att cat tta gag ctg cgg aac agg acg 110
   Met Glu Met Gly Arg Arg Ile His Leu Glu Leu Arg Asn Arg Thr
   1 5 10 15
   ccc tct gat gtg aaa gaa ctt gtc ctg gac aac agt cgg tcg aat gaa 158
   Pro Ser Asp Val Lys Glu Leu Val Leu Asp Asn Ser Arg Ser Asn Glu
   20 25 30
   ggc aaa ctc gaa ggc ctc aca gat gaa ttt gaa gaa ctg gaa ttc tta 206
   Gly Lys Leu Glu Gly Leu Thr Asp Glu Phe Glu Glu Leu Glu Phe Leu
   35 40 45
   agt aca atc aac gta ggc ctc acc tca atc gca aac tta cca aag tta 254
   Ser Thr Ile Asn Val Gly Leu Thr Ser Ile Ala Asn Leu Pro Lys Leu
   50 55 60
   aac aaa ctt aag aag ctt gaa cta agc gat aac aga gtc tca ggg ggc 302
   Asn Lys Leu Lys Lys Leu Glu Leu Ser Asp Asn Arg Val Ser Gly Gly
   65 70 75
   cta gaa gta ttg gca gaa aag tgt cca aac ctc ata cat cta aat tta 350
   Leu Glu Val Leu Ala Glu Lys Cys Pro Asn Leu Ile His Leu Asn Leu
   80 85 90 95
   agt ggc aac aaa att aaa gac ctc agc aca ata gag ccc ctg aaa aag 398
   Ser Gly Asn Lys Ile Lys Asp Leu Ser Thr Ile Glu Pro Leu Lys Lys
   100 105 110
   tta gaa aac ctc gag agc tta gac ctt ttc act tgc gag gta acc aac 446
   Leu Glu Asn Leu Glu Ser Leu Asp Leu Phe Thr Cys Glu Val Thr Asn
   115 120 125
   ctg aac aac tactgagaaa agatgttcaa gctcctcctg caactcacat 495
   Leu Asn Asn
   130
   atctcaacgg ctgtgacccg gatgacaagg aggcccctaa ctcggatggt gagggctttg 555
   tggagtgcct ggatgacaag gaggaggatg aggatgagga ggagtatgat gaagatgctc 615
   aggtaatgga agatgaggag gacgaggatg aggaggagga acgtgaagag gaggacgtga 675
   gtggagacga ggaggagaag gatgaaggtt ataacaatgg agaggtagat gatgaggaag 735
   atgaagaaga gcttggtgaa gaagaaaggg gtcagaagcg aaaataagaa actgaagatg 795
   agggagaaga cgatgcctaa gtggaataat ctattttgaa aaattcctat tgtgatttga 855
   ctgtttttac ccatatcccc tctccccccc ccctctaatc ctgccccctg aa 907
<210> 31
   <211> 130
   <212> PRT
   <213> Homo sapiens
<400> 31
   Met Glu Met Gly Arg Arg Ile His Leu Glu Leu Arg Asn Arg Thr Pro
   1 5 10 15
   Ser Asp Val Lys Glu Leu Val Leu Asp Asn Ser Arg Ser Asn Glu Gly
   20 25 30
   Lys Leu Glu Gly Leu Thr Asp Glu Phe Glu Glu Leu Glu Phe Leu Ser
   35 40 45
   Thr Ile Asn Val Gly Leu Thr Ser Ile Ala Asn Leu Pro Lys Leu Asn
   50 55 60
   Lys Leu Lys Lys Leu Glu Leu Ser Asp Asn Arg Val Ser Gly Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn
   130
<210> 32
   <211> 908
   <212> DNA
   <213> Homo sapiens
<400> 32
   gggttcgggg tttattgatt gaattccgcc ggcgcgggag cctctgcaga gagagagcgc 60
   ggagagatgg agatgggcag acggattcat ttagagctgc ggaacaggac gccctctgat 120
   gtgaaagaac ttgtcctgga caacagtcgg tcgaatgaag gcaaactcga aggcctcaca 180
   gatgaatttg aagaactgga attcttaagt acaatcaacg taggcctcac ctcaatcgca 240
   aacttaccaa agttaaacaa acttaagaag cttgaactaa gcgataacag agtctcaggg 300
   ggcctggaag tattggcaga aaagtgtccg aacctcacgc atctaaattt aagtggcaac 360
   aaaattaaag acctcagcac aatagagcca ctgaaaaagt tagaaaacct caagagctta 420
   gaccttttca attgcgaggt aaccaacctg aacgactacc gagaaaatgt gttcaagctc 480
   ctcccgcaac tcacatatct cgacggctat gaccgggacg acaaggaggc ccctgactcg 540
   gatgctgagg gctacgtgga gggcctggat gatgaggagg aggatgagga tgaggaggag 600
   tatgatgaag atgctcaggt agtggaagac gaggaggacg aggatgagga ggaggaaggt 660
   gaagaggagg acgtgagtgg agaggaggag gaggatgaag aaggttataa cgatggagag 720
   gtagatgacg aggaagatga agaagagctt ggtgaagaag aaaggggtca gaagcgaaaa 780
   cgagaacctg aagatgaggg agaagatgat gactaagtgg aataacctat tttgaaaaat 840
   tcctattgtg atttgactgt ttttacccat atcccctctc ccccccccct ctaatcctgc 900
   cccctgaa 908
<210> 33
   <211> 906
   <212> DNA
   <213> Homo sapiens
<220>
   <221> CDS
   <222> (66)..(812)
<400> 33
   gggttcgggg tttattgatt gaattccgct ggcgcgggag cctctgcaga gagagagcgc 60
   gagag atg gag atg ggc aga cgg att cat tta gag ctg cgg aac agg acg 110
         Met Glu Met Gly Arg Arg Ile His Leu Glu Leu Arg Asn Arg Thr
         1               5                   10                  15
   ccc tct gat gtg aaa gaa ctt gtc ctg gac aac agt cgg tcg aat gaa   158
   Pro Ser Asp Val Lys Glu Leu Val Leu Asp Asn Ser Arg Ser Asn Glu
                   20                  25                  30
   ggc aaa ctc gaa ggc ctc aca gat gaa ttt gaa gaa ctg gaa ttc tta   206
   Gly Lys Leu Glu Gly Leu Thr Asp Glu Phe Glu Glu Leu Glu Phe Leu
               35                  40                  45
   agt aca atc aac gta ggc ctc acc tca atc gca aac tta cca aag tta   254
   Ser Thr Ile Asn Val Gly Leu Thr Ser Ile Ala Asn Leu Pro Lys Leu
           50                  55                  60
   aac aaa ctt aag aag ctt gaa cta agc agt aac aga gtc tca ggg ggc   302
   Asn Lys Leu Lys Lys Leu Glu Leu Ser Ser Asn Arg Val Ser Gly Gly
       65                  70                  75
   cta gaa gta ttg gca gaa aag tgt cca aac ctc acg cat cta aat tta 350
   Leu Glu Val Leu Ala Glu Lys Cys Pro Asn Leu Thr His Leu Asn Leu
   80 85 90 95
   agt ggc aac aaa att aaa gac ctc agc aca ata gag cca ctg aaa aag 398
   Ser Gly Asn Lys Ile Lys Asp Leu Ser Thr Ile Glu Pro Leu Lys Lys
   100 105 110
   tta gaa aac ctc aag agc tta gac ctt ttc aat tgc gag gta acc aac 446
   Leu Glu Asn Leu Lys Ser Leu Asp Leu Phe Asn Cys Glu Val Thr Asn
   115 120 125
   ctg aac gac tac cga gaa aat gtg ttc aag ctc ctc ctg caa ctc aca 494
   Leu Asn Asp Tyr Arg Glu Asn Val Phe Lys Leu Leu Leu Gln Leu Thr
   130 135 140
   tat ctc gac ggc tgt gac cgg gac gac aag gag gcc cct gac tcg gat 542
   Tyr Leu Asp Gly Cys Asp Arg Asp Asp Lys Glu Ala Pro Asp Ser Asp
   145 150 155
   gct gag ggc tac gtg gag ggc ctg gat gac gag gag gag gat gag gat 590
   Ala Glu Gly Tyr Val Glu Gly Leu Asp Asp Glu Glu Glu Asp Glu Asp
   160 165 170 175
   gag gag gag tat gat gaa gat gct cag gta gtg gaa gat gag gag gac 638
   Glu Glu Glu Tyr Asp Glu Asp Ala Gln Val Val Glu Asp Glu Glu Asp
   180 185 190
   gag gat gag gag gag gaa ggt gaa gag gag gac gtg agt gga gag gag 686
   Glu Asp Glu Glu Glu Glu Gly Glu Glu Glu Asp Val Ser Gly Glu Glu
   195 200 205
   gag gag gat gaa gaa ggt tat aac gat gga gag gta gat gac gag gaa 734
   Glu Glu Asp Glu Glu Gly Tyr Asn Asp Gly Glu Val Asp Asp Glu Glu
   210 215 220
   gat gaa gaa gag ctt ggt gaa gaa gaa agg ggt cag aag cga aaa gag 782
   Asp Glu Glu Glu Leu Gly Glu Glu Glu Arg Gly Gln Lys Arg Lys Glu
   225 230 235
   aac ctg aag atg agg gag aag atg atg act aagtggaata acctattttg 832
   Asn Leu Lys Met Arg Glu Lys Met Met Thr
   240 245
   aaaaattcct attgtgattt gactgttttt acccatatcc cctctccccc ccccctctaa 892
   tcctgccccc tgaa 906
<210> 34
   <211> 249
   <212> PRT
   <213> Homo sapiens
<400> 34
   Met Glu Met Gly Arg Arg Ile His Leu Glu Leu Arg Asn Arg Thr Pro
   1 5 10 15
   Ser Asp Val Lys Glu Leu Val Leu Asp Asn Ser Arg Ser Asn Glu Gly
   20 25 30
   Lys Leu Glu Gly Leu Thr Asp Glu Phe Glu Glu Leu Glu Phe Leu Ser
   35 40 45
   Thr Ile Asn Val Gly Leu Thr Ser Ile Ala Asn Leu Pro Lys Leu Asn
   50 55 60
   Lys Leu Lys Lys Leu Glu Leu Ser Ser Asn Arg Val Ser Gly Gly Leu
   65 70 75 80
   Glu Val Leu Ala Glu Lys Cys Pro Asn Leu Thr His Leu Asn Leu Ser
   85 90 95
   Gly Asn Lys Ile Lys Asp Leu Ser Thr Ile Glu Pro Leu Lys Lys Leu
   100 105 110
   Glu Asn Leu Lys Ser Leu Asp Leu Phe Asn Cys Glu Val Thr Asn Leu
   115 120 125
   Asn Asp Tyr Arg Glu Asn Val Phe Lys Leu Leu Leu Gln Leu Thr Tyr
   130 135 140
   Leu Asp Gly Cys Asp Arg Asp Asp Lys Glu Ala Pro Asp Ser Asp Ala
   145 150 155 160
   Glu Gly Tyr Val Glu Gly Leu Asp Asp Glu Glu Glu Asp Glu Asp Glu
   165 170 175
   Glu Glu Tyr Asp Glu Asp Ala Gln Val Val Glu Asp Glu Glu Asp Glu
   180 185 190
   Asp Glu Glu Glu Glu Gly Glu Glu Glu Asp Val Ser Gly Glu Glu Glu
   195 200 205
   Glu Asp Glu Glu Gly Tyr Asn Asp Gly Glu Val Asp Asp Glu Glu Asp
   210 215 220
   Glu Glu Glu Leu Gly Glu Glu Glu Arg Gly Gln Lys Arg Lys Glu Asn
   225 230 235 240
   Leu Lys Met Arg Glu Lys Met Met Thr
   245
<210> 35
   <211> 26
   <212> DNA
   <213> Homo sapiens
<400> 35
   tatgctagcg ggttcggggt ttattg 26
<210> 36
   <211> 29
   <212> DNA
   <213> Homo sapiens
<400> 36
   gattctagat ggtaagttta cgattgagg 29
<210> 37
   <211> 29
   <212> DNA
   <213> Homo sapiens
<400> 37
   gaatctagaa ggaggagaaa ggtgaagag 29
<210> 38
   <211> 29
   <212> DNA
   <213> Homo sapiens
<400> 38
   ctatctagat tcagggggca ggattagag 29
<210> 39
   <211> 24
   <212> DNA
   <213> Homo sapiens
<400> 39
   gaggtttatt gattgaattc ggct 24
<210> 40
   <211> 24
   <212> DNA
   <213> Homo sapiens
<400> 40
   ccccagtaca cttttcccgt ctca 24
<210> 41
   <211> 12
   <212> DNA
   <213> Artificial Sequence
<220>
   <223> Description of Artificial Sequence: recognition sequence
<400> 41
   tttttctttt tc 12
<210> 42
   <211> 10
   <212> DNA
   <213> Artificial Sequence
<220>
   <223> Description of Artificial Sequence: recognistion sequence
<400> 42
   ttaaaattoa 10
<210> 43
   <211> 10
   <212> DNA
   <213> Artificial Sequence
<220>
   <223> Description of Artificial Sequence: recognition sequence
<400> 43
   atgtaaaaca 10
<210> 44
   <211> 11
   <212> DNA
   <213> Artificial Sequence
<220>
   <223> Description of Artificial Sequence: recognition sequence
<400> 44
   aagataaaac c 11
**<210> 45**
   <211> 10
   <212> DNA
   <213> Artificial Sequence
<220>
   <223> Description of Artificial Sequence; recognition sequence
<400> 45
   ccactgggga 10
<210> 46
   <211> 13
   <212> DNA
   <213> Artificial Sequence
<220>
   <223> Description of Artificial Sequence: recognition sequence
<400> 46
   ctctctctct ctc 13
<210> 47
   <211> 11
   <212> DNA
   <213> Artificial Sequence
<220>
   <223> Description of Artificial Sequence: recognition sequence
<400> 47
   aaaacataaa t 11
<210> 48
   <211> 131
   <212> PRT
   <213> Homo sapiens
<220>
   <223> Description of Artificial Sequence: recognition sequence
<400> 48
   Met Glu Met Gly Lys Trp Ile His Leu Glu Leu Arg Asn Arg Thr Pro
   1 5 10 15
   Ser Asp Val Lys Glu Leu Phe Leu Asp Asn Ser Gln Ser Asn Glu Gly
   20 25 30
   Lys Leu Glu Gly Leu Ala Asp Glu Phe Glu Glu Leu Glu Leu Leu Asn
   35 40 45
   Thr Ile Asn Ile Gly Leu Ser Ser Ile Ala Asn Leu Ala Lys Leu Asn
   50 55 60
   Lys Leu Lys Lys Leu Glu Leu Ser Ser Asn Arg Ala Ser Val Gly Leu
   65 70 75 80
   Glu Val Leu Ala Glu Lys Cys Pro Asn Leu Ile His Leu Asn Leu Ser
   85 90 95
   Gly Asn Lys Ile Lys Asp Leu Ser Thr Ile Glu Pro Leu Lys Lys Leu
   100 105 110
   Glu Asn Leu Glu Ser Leu Asp Leu Phe Thr Cys Glu Val Thr Asn Leu
   115 120 125
   Asn Asn Tyr
   130
<210> 49
   <211> 234
   <212> PRT
   <213> Homo sapiens
<220>
   <223> Description of Artificial Sequence: recognition sequence
<400> 49
   Met Glu Met Gly Arg Arg Ile His Ser Glu Leu Arg Asn Arg Ala Pro
   1 5 10 15
   Ser Asp Val Lys Glu Leu Ala Leu Asp Asn Ser Arg Ser Asn Glu Gly
   20 25 30
   Lys Leu Glu Ala Leu Thr Asp Glu Phe Glu Glu Leu Glu Phe Leu Ser
   35 40 45
   Lys Ile Asn Gly Gly Leu Thr Ser Ile Ser Asp Leu Pro Lys Leu Lys
   50 55 60
   Leu Arg Lys Leu Glu Leu Arg Val Ser Gly Gly Leu Glu Val Leu Ala
   65 70 75 80
   Glu Lys Cys Pro Asn Leu Thr His Leu Tyr Leu Ser Gly Asn Lys Ile
   85 90 95
   Lys Asp Leu Ser Thr Ile Glu Pro Leu Lys Gln Leu Glu Asn Leu Lys
   100 105 110
   Ser Leu Asp Leu Phe Asn Cys Glu Val Thr Asn Leu Asn Asp Tyr Gly
   115 120 125
   Glu Asn Val Phe Lys Leu Leu Leu Gln Leu Thr Tyr Leu Asp Ser Cys
   130 135 140
   Tyr Trp Asp His Lys Glu Ala Pro Tyr Ser Asp Ile Glu Asp His Val
   145 150 155 160
   Glu Gly Leu Asp Asp Glu Glu Glu Gly Glu His Glu Glu Glu Tyr Asp
   165 170 175
   Glu Asp Ala Gln Val Val Glu Asp Glu Glu Gly Glu Glu Glu Glu Glu
   180 185 190
   Glu Gly Glu Glu Glu Asp Val Ser Gly Gly Asp Glu Glu Asp Glu Glu
   195 200 205
   Gly Tyr Asn Asp Gly Glu Val Asp Gly Glu Glu Asp Glu Glu Glu Leu
   210 215 220
   Gly Glu Glu Glu Arg Gly Gln Lys Arg Lys
   225 230
<210> 50
   <211> 17
   <212> DNA
   <213> Homo sapiens
<400> 50
   gggttcgggg tttattg 17
<210> 51
   <211> 20
   <212> DNA
   <213> Homo sapiens
<400> 51
   ctctaatact gcaccctgaa 20

## Description

### BACKGROUND

### File of the Invention

This invention is directed to various members of a gene family with transformation modulating activity, and to diagnostic and gene therapy techniques based on the variants.

### Review of Related Art

Prostatic adenocarcinoma is the most frequent malignancy in adult men with approximately 317,000 new cases diagnosed each year (Parker, et al., CA, 46:8-27, 1996). In spite of the capabilities for early diagnosis and treatment (Potosky, et al., JAMA, 273:548-552, 1995), it represents the second leading cause of cancer death in men following lung cancer.

To date, the study of alterations in specific genes has not been particularly rewarding in primary prostate cancer. Most alterations in the widely studied oncogenes and tumor suppressor genes occur in only 20 - 30% of primary prostate carcinomas, except for the myc gene, where overexpression has been observed in as many as 50 - 60% of such cases (Fleming, et al., Cancer Res., 46:1535-1538, 1986). Up to 40% of primary prostate cancers studied by comparative genomic hybridization display chromosomal aberrations (Visakorpi, et al., Cancer Res., 55:342-347, 1995), although such alterations occur more frequently as tumors recur and become refractory to hormonal therapy. Characterization of candidate proto-oncogenes or tumor suppressor genes at such altered loci may eventually shed light on tumor progression in the prostate.

pp32 (GenBank HSU73477) is a highly conserved nuclear phosphoprotein. Increased expression of pp32 or closely related species is a frequent feature of clinical cancers. For example, in human prostate cancer, high-level expression of RNA hybridizing with pp32 probes occurs in nearly 90% of clinically significant prostate cancers, in contrast to the substantially lower frequencies of alterations of other oncogenes and tumor suppressors (See U.S. Patent No. 5,726,018, incorporated herein by reference).

### Molecular Features and Activities of pp32.

pp32 is a nuclear phosphoprotein that is differentiation-regulated during differentiation of adult prostatic epithelium (Walensky, et al., Cancer Res. 53:4720-4726, 1993). The human pp32 cDNA sequence (Gen-Bank U73477) is 1052 bp in length and encodes a protein of 249 amino acids. The protein is composed of two domains: an amino terminal amphipathic α-helical region containing a leucine zipper, and a highly acidic carboxyl terminal region. The murine and human forms of pp32 are highly conserved with over 90% nucleic acid homology and over 95% protein-level homology.

Human pp32 has been isolated independently by a number of groups. Vaesen et al. ("Purification and characterization of two putative HLA class II associated proteins: PHAPI and PHAPII." *Biol. Chem. Hoppe-Seyler,* 375:113-126, 1994) cloned an essentially equivalent molecule, termed PHAPI, from an EBV-transformed human B-lymphoblastoid cell line; PHAPII, cloned by the same strategy, is unrelated to pp32. This study identified PHAPI through its association in solution with human HLA class II protein, noting membrane and cytoplasmic localization as well as nuclear; the gene has putatively been localized to chromosome 15q22.3-q23 by fluorescent in situ hybridization (Fink, et al., "Localization of the gene encoding the putative human HLA class II-associated protein (PHAPI) to chromosome 15q22.3-q23 by fluorescence in situ hybridization." *Genomics,* 29:309-310, 1995). More recently, a group studying inhibitors of protein phosphatases identified pp32 as I1PP2a, an inhibitor of protein phosphatase 2a (Li, et al., "Molecular Identification of I1PP2A, a novel potent heat-stable inhibitor protein of protein phosphatase 2A." *Biochemistry* 35:6998-7002, 1996); another phosphatase inhibitor, I2PP2a, is unrelated to pp32. Interestingly, another recent report (Ulitzur, et al., "Biochemical characterization of mapmodulin, a protein that binds microtubule-associated proteins." *Journal of Biological Chemistry* 272:30577-30582, 1997) identified pp32 as a cytoskeletally-associated cytosolic protein in CHO cells. It is not clear whether this finding stems from a difference in system, or whether pp32 can localize to the cytoplasm under certain circumstances. pp32 has also been identified as LANP, a leucine rich nuclear protein in the central nervous system (Matsuoka, et al., "A nuclear factor containing the leucine-rich repeats expressed in murine cerebellar neurons. *Proc Natl Acad Sci USA* 91: 9670-9674, 1994).

There are also a number of reports of gene products bearing lesser degrees of homology to pp32. The Vaesen group has identified a series of unpublished sequences, termed PHAP12a (EMBL Locus HSPHAP12A) and PHAP12b (EMBL Locus HSPHAP12B), also cloned from an EBV-transformed human B-lymphoblastoid cell line. These variant pp32 sequences are distinct from the sequences reported herein, representing the April protein instead. April, cloned from human pancreas, is shorter than PHAP12a by two N-terminal amino acids (Mencinger, et al., "Expression analysis and chromosomal mapping of a novel human gene, APRIL, encoding an acidic protein rich in leucines." *Biochimica et Biophysica Acta.* 1395: 176-180, 1998, see EMBL Locus HSAPRIL); PHAP12b is identical to a subset of APRIL. Silver-stainable protein SSP29 (unpublished GenBank Locus HSU70439) was cloned from HeLa cells and is identical to PHAP12a.

The nuclear phosphoprotein pp32 has been linked to proliferation. Malek and associates reported that various neoplastic cell lines showed markedly elevated expression levels and that bacterial polysaccharide induced expression of pp32 epitopes by B lymphocytes upon polyclonal expansion (Malek, et al., J. Biol. Chem., 265: 13400-13409, 1990). Walensky and associates reported that levels of pp32 expression, measured by *in situ* hybridization, increased in direct relation to increasing Gleason grade of human prostatic cancers.

Pp32 cDNA probes hybridize strongly with prostatic adenocarcinoma, whereas the hybridization signal in normal prostate is confined to basal cells. Polyclonal anti-pp32 antibodies react strongly with sections of human prostatic adenocarcinoma. The antibodies and riboprobes used by the investigators in previous studies are consistent with cross-reactivities of the reagents with all reported members of the pp32 nuclear phosphoprotein family, therefore, while previous descriptions focused upon pp32, it cannot be excluded that homologous proteins were detected.

WO 9403594 describes a DNA sequence cloned from the probasin gene promoter region that confers androgen regulation in cell culture and prostate specific expression in eukaryotes. Alteration of the DNA sequences allows production of prostatic disease models and gene therapy for prostatic disease.

The sequence provided under accession number U71084 has a region of homology with the sequence provided herein but the sequences do not share more than 14 contiguous nucleotides.

WO 9610092 described three proteins useful in the diagnosis and prognosis of lymphoid and epithelial tumors.

### SUMMARY OF THE INVENTION

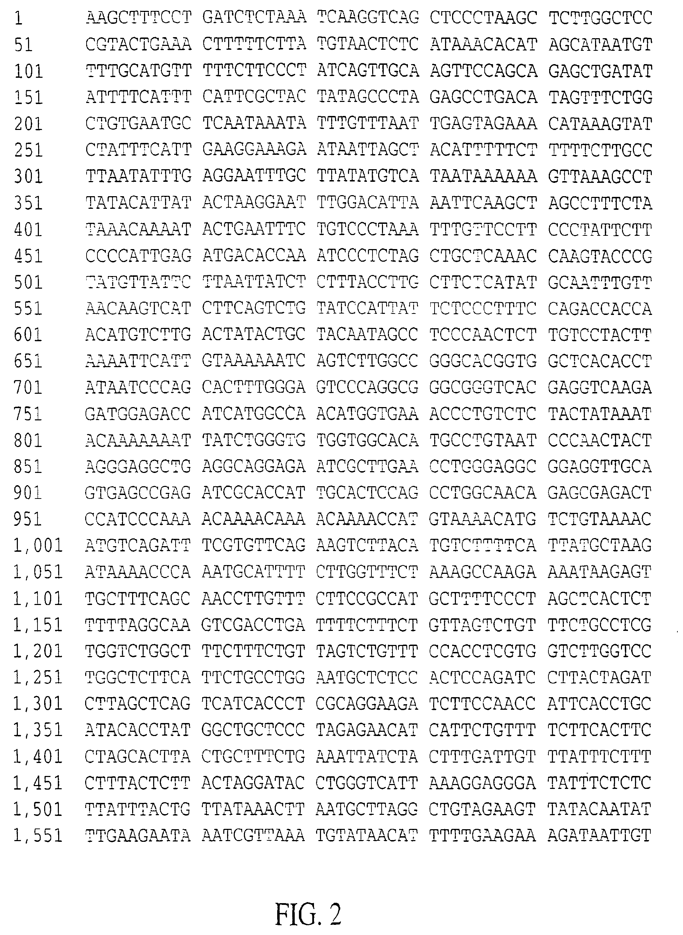

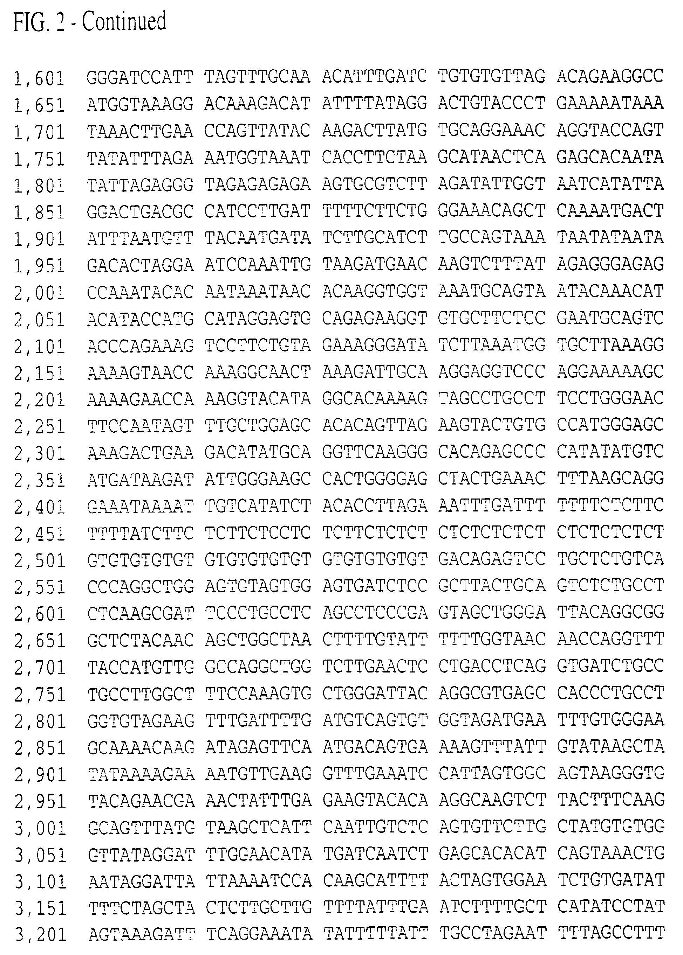

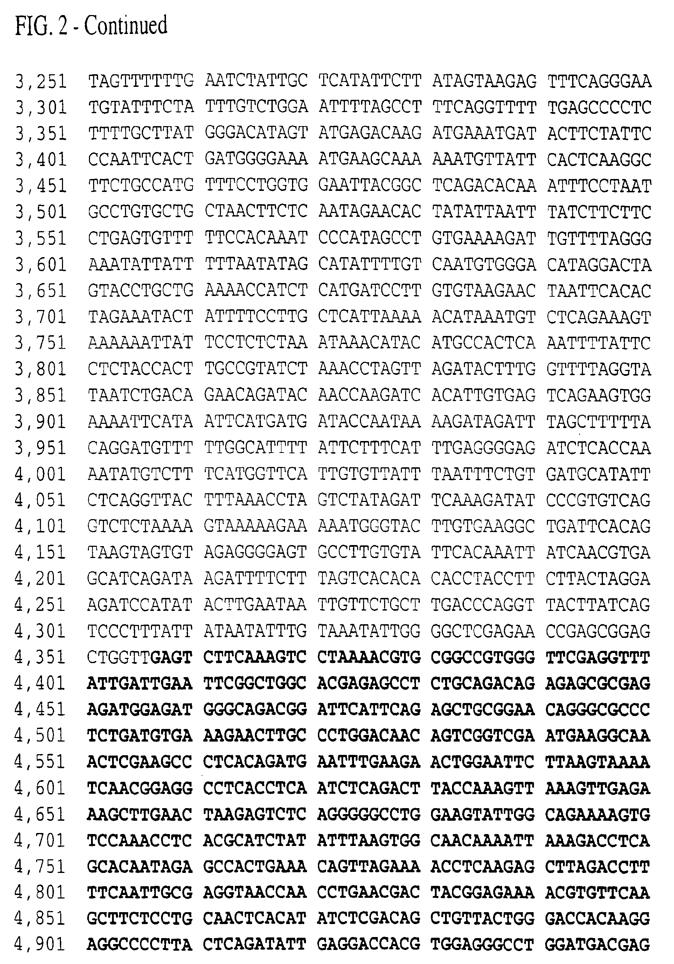

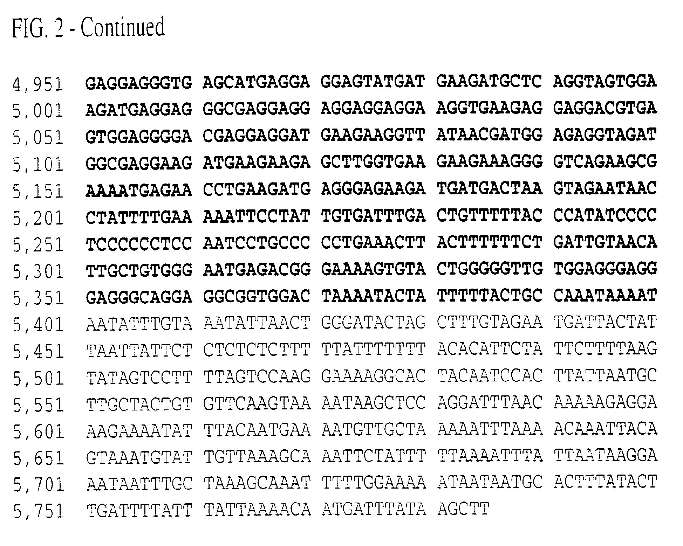

This invention provides an isolated DNA molecule comprising a sequence of 18 contiguous nucleotides selected from the sequence consisting of base pairs 4894-4942 of the sequence shown in Figure 2 or its complement. Alternatively, this invention provides an isolated DNA molecule that encodes amino acid residues 146-163 of tumor-derived pp32r1 sequence (encoded by the sequence of figure 2). In one mode, the DNA molecule is an expression vector which expresses said amino acid sequence and the invention also includes a recombinant cell containing the expression vector. In another mode, the DNA molecule has the particular sequence operatively linked to a promoter in antisense orientation. In another alternative, this invention provides an isolated nucleic acid probe or at least is nucleotides which specifically hybridizes on Northern blot with nucleic acid encoding the amino acids from residue 146-163 of the tumor-derived pp32r1 sequence (encoded by the sequence of figure 2), a preferred probe would have a sequence of at least 8 contiguous nucleotides "unique" to the nucleotide sequence of the pp32r1 variant as described herein. In yet another alternative, the invention provides a pair of nucleic acid primers each of which comprises at least 10 contiguous nucleotides, at least one of the primers binding specifically to the pp32r1 sequence, where if the primers are used in nucleic acid amplification of a suitable source of human nucleic acid the amplification will produce an amplified nucleic acid encoding at least residues 146-163 of the pp32r1 sequence.

In still another aspect, this invention provides antibodies that specifically bind the tumor derived pp32, but do not bind to normal pp32. Preferably, these antibodies are monoclonal antibodies. The invention also provides polypeptides containing epitopes that bind these antibodies.

In yet another aspect this invention provides diagnostic methods for predicting malignant potential of neuroendocrine neural mesenchymal, lymphoid, epithelial or germ cell derived tumors by determining, in a sample of human neuroendocrine, neural, mesenchymal, lymphoid, epithelial or germ cell derived tissue, the level of, or the intracellular sites of expression of a gene product expressed from a gene sequence which encodes, *inter alia,* residues 146-163 of tumor derived pp32r1. Where the gene product is mRNA, the mRNA is extracted from the sample and quantitated, optionally by PCR. or the level of mRNA may be determined by *in situ* hybridization to a section of the tissue sample. Where the gene product is protein, the determination may include reacting the sample with an antibody that specifically binds to tumor derived pp32. but not to normal pp32. Preferably, the tissue sample is carcinoma tissue, e.g., carcinoma or sarcoma of a tissue selected from the group consisting of epithelial, lymphoid, hematopoietic, mesenchymal central nervous system and peripheral nervous system tissues, including colon carcinoma, prostate carcinoma and non-Hodgkin's lymphoma.

pp32 is a member of a highly conserved family of differentiation-regulated nuclear proteins that is highly expressed in nearly all human prostatic adenocarcinomas of Gleason Grade ≥ 5. This contrasts with the low percentage of prostate tumors that express molecular alterations in proto-oncogenes or demonstrate tumor suppressor mutation or loss of heterozygosity. By analysis of specimens of human prostatic adenocarcinoma and paired adjacent normal prostate from three individual patients, the inventors have shown that normal prostate continues to express normal pp32, whereas three of three sets of RT-PCR-amplified transcripts from prostatic adenocarcinomas display multiple *cancer-associated* coding sequence changes. The cancer-associated sequence changes appear to be functionally significant. Normal pp32 exerts antineoplastic effects through suppression of transformation. In contrast, cancer-associated pp32 variants augment, rather than inhibit, transformation.

### BRIEF DESCRIPTION OF THE DRAWINGS

Figure 1A shows detection of pp32-related mRNA in benign prostate and prostate cancer tissue sections by *in situ* hybridization.
Figure 1B shows immunohistochemical stain of prostate cancer sections with anti-pp32 antibodies.
Figure 2 shows the genomic sequence of variant pp32r1 isolated from human placenta.
Figure 3 provides a base-by-base comparison of the sequence of pp32r1 (top) with normal human pp32 (bottom). The numbering system for pp32r1 corresponds to Figure 1. and the numbering system for normal pp32 is taken from Chen, et al. Nucleotide base differences are underlined in the pp32r1 sequence. Sequences within the normal pp32 sequence missing in pp32r1 are represented by dashes. The open reading frame for pp32r1 is indicated by overlining.
Figure 4 shows the alignment of the pp32r1 amino acid sequence (top) with normal human pp32 (bottom). Residue changes are underlined in the pp32r1 sequence. Amino acids missing in the pp32r1 sequence compared to normal pp32 are represented by dashes.
Figure 5 shows the genomic sequence of variant pp32r2.
Figure 6A shows RT-PCR amplification of pp32 and pp32 variants from human prostate cancer and prostate cancer cell line.
Figure 6B shows cleavase fragment length polymorphism analysis of pp32 detects variant pp32 transcripts in human prostate cancer.
Figure 7 shows the alignment of nucleic acid (A) and amino acid (B) sequences from human prostatic adenocarcinoma and prostatic adenocarcinoma cell lines with pp32.
Figure 8 is a bar graph showing ras + myc induced transformed focus formation. Co-transfection with a pp32 expression vector reduces transformation, while co-transfection with a pp32r1 expression vector stimulates transformation.
Figure 9 is a bar graph showing pp32r1 stimulation of ras + myc induced transformed focus formation. Co-transfection with a pp32 expression vector reduces transformation, while co-transfection with expression vectors for pp32r1 sequences from prostate cancer cell lines stimulate transformation.
Figure 10 is a graph of transformation assay results for cells transfected with variant pp32 species, which are shown to stimulate transformation with variable potency.

### DETAILED DESCRIPTION OF THE INVENTION

The inventors have discovered that phenotypic changes in pp32 are a common feature of human prostate cancer. Previous data show that 87% of prostate cancers of Gleason Score 5 and above express pp32 or closely-related transcripts (U.S. Patent No. 5,734,022). This is striking in comparison to the frequency of molecular alterations in other widely studied oncogenes and tumor suppressor genes in primary prostatic adenocarcinoma, which occur in a substantially smaller proportion of cases. For example, myc overexpression (Fleming, et al.) occurs in around 60% of cases, and p53 is abnormal in only around 25% of primary tumors (Isaacs, et al., in "Genetic Alterations in Prostate Cancer." Cold Spring Harbor Symposia on Quantitative Biology, 59:653-659, 1994).

Several lines of evidence suggest that pp32 may act as a tumor suppressor. Functionally, pp32 inhibits transformation *in vitro* by oncogene pairs such as ras with myc, mutant p53, Ela, or jun, or human papilloma virus E6 and E7 (Chen. et al., "Structure of pp32, an acidic nuclear protein which inhibits oncogene-induced formation of transformed foci." *Molecular Biology of the Cell,* 7:2045-2056, 1996). pp32 also inhibits growth of transformed cells in soft agar (Chen, et al.). In another system, ras-transfected NIH3T3 cells previously transfected to overexpress normal human pp32 do not form foci *in vitro* or, preliminarily, do not form tumors in nude mice, unlike control cells. In contrast, knockout of endogenous pp32 in the same system by an antisense pp32 expression construct markedly augments tumorigenesis (Example 12 below).

In clinical prostate cancer, the situation at first appears counterintuitive. Most human prostate cancers seem to express high levels of pp32 by in situ hybridization (see Example 1 below) and stain intensely with anti-pp32 antibodies. Because pp32 inhibits oncogene-mediated transformation (Chen, et al.), its paradoxical expression in cancer was investigated at the sequence level. The paradoxical question of why prostate cancers seem to express high-levels of an anti-oncogenic protein was addressed by comparing the sequence and function of pp32 species from paired normal prostate and adjacent prostatic carcinoma from three patients as well as from four prostate cancer cell lines. It is demonstrated herein that pp32 is a member of a closely-related gene family, and that alternate expression of these closely-related genes located on different chromosomes modulates oncogenic potential in human prostate cancer. The variant pp32 species expressed in prostate cancer are closely related to pp32.

The present data indicate that prostate cancers express variant pp32 transcripts, whereas adjacent normal prostate expresses normal pp32. Two instances clearly show that expression of alternate genes on different chromosomes can lead to the phenotypic switch, rather than mutation or alternate splicing. This switch in molecular phenotype is accompanied by a switch in functional pp32 phenotype. Normal pp32 is anti-oncogenic in character, in contrast to the pro-oncogenic variant transcripts that foster oncogene-mediated transformation. The high frequency of this abnormality suggests that expression of variant pp32 species may play an etiologic role in the development of human prostate cancer. In addition, these findings have significant diagnostic and prognostic implications.

### Definitions

In describing the present invention, the following terminology is used in accordance with the definitions set out below.

### Nucleic Acids

In discussing the structure of particular double-stranded DNA molecules, sequences may be described herein according to the normal convention of giving only the sequence in the 5' to 3' direction along the nontranscribed stand of DNA (i.e., the strand having a sequence homologous to the mRNA).

A DNA sequence "corresponds" to an amino acid sequence if translation of the DNA sequence in accordance with the genetic code yields the amino acid sequence (i.e., the DNA sequence "encodes" the amino acid sequence): one DNA sequence "corresponds" to another DNA sequence if the two sequences encode the same amino acid sequence.

Two DNA sequences are "substantially similar'' when at least about 90% (preferably at least about 94%, and most preferably at least about 96%) of the nucleotides match over the defined length of the DNA sequences. Sequences that are substantially similar can be identified by the assay procedures described below or by isolating and sequencing the DNA molecules. See e.g., Maniatis et al.. *infra,* DNA Cloning, vols. I and II *infra*: Nucleic Acid Hybridization, *infra.*

A "heterologous" region or domain of a DNA construct is an identifiable segment of DNA within a larger DNA molecule that is not found in association with the larger molecule in nature. Thus, when the heterologous region encodes a mammalian gene, the gene will usually be fianked by DNA that does not flank the mammalian genomic DNA in the genome of the source organism. Another example of a heterologous region is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

A "coding sequence" or "open reading frame" is an in-frame sequence of codons that (in view of the genetic code) correspond to or encode a protein or peptide sequence. Two coding sequences correspond to each other if the sequences or their complementary sequences encode the same amino acid sequences. A coding sequence in association with appropriate regulatory sequences may be transcribed and translated into a polypeptide *in vivo.* A polyadenylation signal and transcription termination sequence will usually be located 3' to the coding sequence. A "promoter sequence" is a DNA regulatory region capable of binding RNA polymerase in a cell and initiating transcription of a downstream (3' direction) coding sequence. Promoter sequences typically contain additional sites for binding of regulatory molecules (e.g., transcription factors) which affect the transcription of the coding sequence. A coding sequence is "under the control" of the promoter sequence or "operatively linked" to the promoter when RNA polymerase binds the promoter sequence in a cell and transcribes the coding sequence into mRNA, which is then in turn translated into the protein encoded by the coding sequence.

Vectors are used to introduce a foreign substance, such as DNA, RNA or protein, into an organism. Typical vectors include recombinant viruses (for DNA) and liposomes (for protein). A "DNA vector" is a replicon, such as plasmid, phase or cosmid, to which another DNA segment may be attached so as to bring about the replication of the attached segment. An "expression vector" is a DNA vector which contains regulatory sequences which will direct protein synthesis by an appropriate host cell. This usually means a promoter to bind RNA polymerase and initiate transcription of mRNA, as well as ribosome binding sites and initiation signals to direct translation of the mRNA into a polypeptide. Incorporation of a DNA sequence into an expression vector at the proper site and in correct reading frame, followed by transformation of an appropriate host cell by the vector, enables the production of a protein encoded by said DNA sequence.

An expression vector may alternatively contain an antisense sequence, where a small DNA fragment, corresponding to all or part of an mRNA sequence, is inserted in opposite orientation into the vector after a promoter. As a result, the inserted DNA will be transcribed to produce an RNA which is complementary to and capable of binding or hybridizing with the mRNA. Upon binding to the mRNA, translation of the mRNA is prevented, and consequently the protein coded for by the mRNA is not produced. Production and use of antisense expression vectors is described in more detail in U.S. Patent 5.107,065 (describing and exemplifying antisense regulation of genes in plants) and U.S. Patent 5.190.931 (describing antisense regulation of genes in both prokaryotes and eukaryotes and exemplifying prokaryotes).

"Amplification" of nucleic acid sequences is the *in vitro* production of multiple copies of a particular nucleic acid sequence. The amplified sequence is usually in the form of DNA. A variety of techniques for carrying out such amplification are described in a review article by Van Brunt (1990, *Bio*/*Technol.,* 8(4):291-294). Polymerase chain reaction or PCR is a prototype of nucleic acid amplification, and use of PCR herein should be considered exemplary of other suitable amplification techniques.

### Polypeptides

For the purposes of defining the present invention, two proteins arc homologous if 80% of the amino acids in their respective amino acid sequences are the same; for proteins of differing length, the sequences will be at least 80% identical over the sequence which is in common (i.e., the length of the shorter protein).

Two amino acid sequences are "substantially similar" when at least about 87% of the amino acids match over the defined length of the amino acid sequences, preferably a match of at least about 89%, more preferably a match of at least about 95%. Typically, two amino acid sequences which are similar will differ by only conservative substitutions.

"Conservative amino acid substitutions" are the substitution of one amino acid residue in a sequence by another residue of similar properties, such that the secondary and tertiary structure of the resultant peptides are substantially the same. Conservative amino acid substitutions occur when an amino acid has substantially the same charge or hydrophobicity as the amino acid for which it is substituted and the substitution has no significant effect on the local conformation of the protein. Amino acid pairs which may be conservatively substituted for one another are well-known to those of ordinary skill in the art.

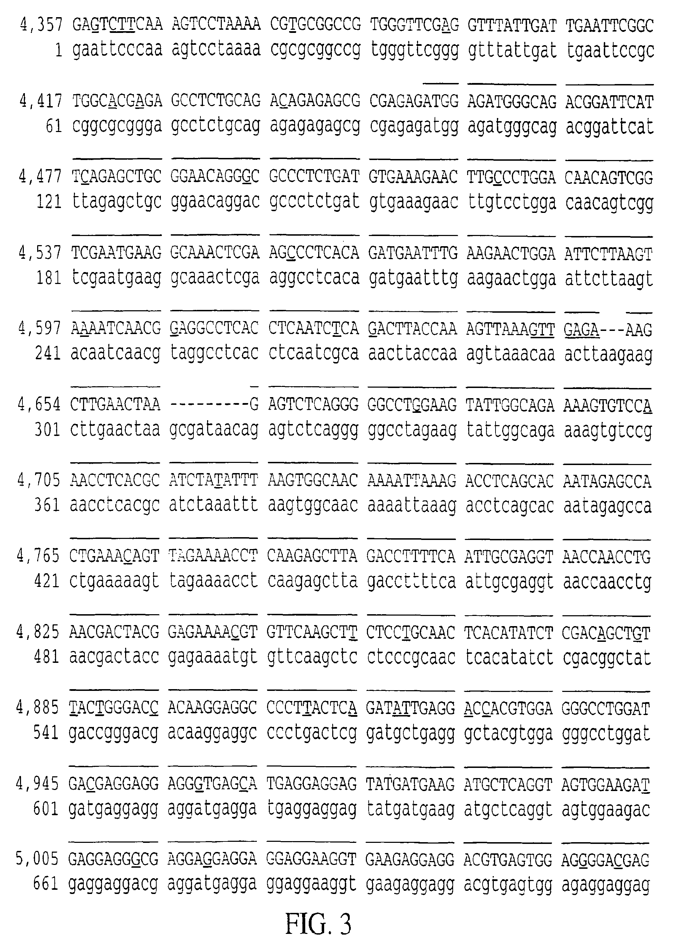

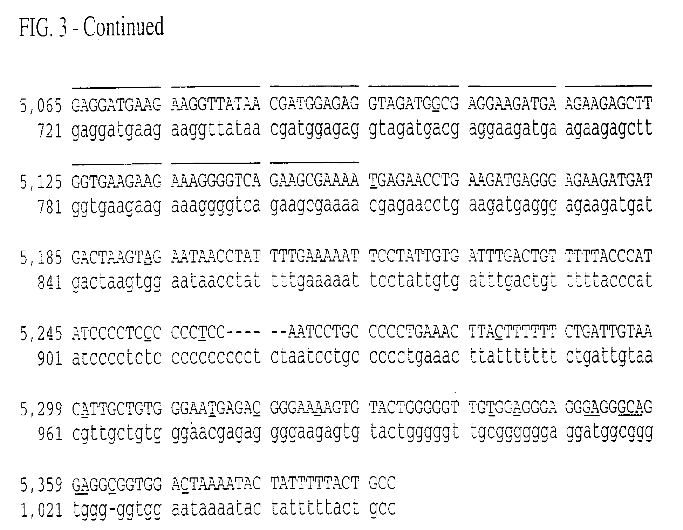

The polypeptides of this invention encompass pp32r1 and pp32r1 analogs, pp32r2 and pp32r2 analogs, along with other variants of pp32 and their analogs, pp32r1 and pp32r2 are naturally occurring, mature proteins, and further encompass all precursors and allelic variations of pp32r1 and pp32r2, as well as including forms of heterogeneous molecular weight that may result from inconsistent processing *in vivo.* An example of the pp32r1 sequence is shown in Figure 3. top line. pp32r1 analogs" are a class oi peptides which includes:
1) "Allelic variations of pp32r1," which are polypeptides which are substantially similar to pp32r1. Preferably the amino acid sequence of the allelic variation is encoded by a nucleic acid sequence that differs from the sequence of pp32rl by one nucleotide in 300;
2) "Truncated pp32r1 peptides,'' which include fragments of either pp32 or allelic variations of pp32r1 that preferably retain either (i) an amino acid sequence unique to pp32r1, (ii) an epitope unique to pp32rl or (iii) pp32r1 activity;
3) "pp32r1 fusion proteins," which include heterologous polypeptides which are made up of one of the above polypeptides (pp32r1, allelic variations of pp32r1 or truncated pp32r1 peptides) fused to any heterologous amino acid sequence.

"Unique" sequences of the pp32r1 variant according to this invention, either amino acid sequences or nucleic acid sequences which encode them, are sequences which are identical to a sequence of a pp32r1 polypeptide, but which differ in at least one amino acid or nucleotide residue from the sequences of human pp32 (Genbank Locus HSU73477), murine pp32 (Genbank Locus MMU73478), human cerebellar leucine rich acidic nuclear protein (LANP) (Genbank Locus AF025684), murine LANP (Genbank Locus AF022957), 11PP2a or human potent heat-stable protein phospatase 2a inhibitor (Genbank Locus HSU60823), SSP29 (Genbank Locus HSU70439), HLA-DR associated protein I (Genbank Locus HSPPHAP1. Accession No. X75090), PHAP12a (EMBL Locus HSPHAP12A, Genbank Accession No. Y07569), PHAP12b (EMBL Locus HSPHAP12B, Genbank Accession No. Y07570), and April (EMBL Locus HSAPRIL), and preferably, are not found elsewhere in the human genome. (A list of these sequences is provided in Table 3A.) Similarly, an epitope is "unique" to pp32r1 polypeptides if it is found on pp32r1 polypeptides but not found on any members of the set of proteins listed above. Analogs of pp32r2 and unique pp32r2 sequences are defined similarly. Of course, unique sequences of pp32r1 are not found in pp32r2 and vice versa.

"Variants of pp32" are homologous proteins which differ from pp32 by at least 2 amino acids. In particular, sequence comparison between pp32 and a variant will demonstrate at least one segment of 10 amino acids in which the sequence differs by at least two (2) amino acids. More typically a variant will exhibit at least two such 10 amino acid segments. Preferably, variants of pp32 in accordance with this invention will exhibit differences in functional activity from pp32. In particular, pp32r1 and pp32r2 are variants of pp32 whose activity includes stimulation of transformation in the rat fibroblast transformation assay described herein.

A composition comprising a selected component A is "substantially free" of another component B when component A makes up at least about 75% by weight of the combined weight of components A and B. Preferably, selected component A comprises at least about 90% by weight of the combined weight, most preferably at least about 99% by weight of the combined weight. In the case of a composition comprising a selected biologically active protein, which is substantially free of contaminating proteins, it is sometimes preferred that the composition having the activity of the protein of interest contain species with only a single molecular weight (i.e., a "homogeneous" composition).

As used herein, a "biological sample" refers to a sample of tissue or fluid isolated from a individual, including but not limited to, for example, plasma, serum, spinal fluid, lymph fluid, the external sections of the skin, respiratory, intestinal, and genitourinary tracts, tears, saliva, milk, blood cells, tumors, organs, and also samples of *in vivo* cell culture constituents (including but not limited to conditioned medium resulting from the growth of cells in cell culture medium, putatively virally infected cells, recombinant cells, and cell components).

"Human tissue" is an aggregate of human cells which may constitute a solid mass. This term also encompasses a suspension of human cells, such as blood cells, or a human cell line.

The term "immunoglobulin molecule" encompasses whole antibodies made up of four immunoglobulin peptide chains, two heavy chains and two light chains, as well as immunoglobulin fragments. "Immunoglobulin fragments" are protein molecules related to antibodies, which are known to retain the epitopic binding specificity of the original antibody, such as Fab, F(ab)'₂, Fv, etc. Two polypeptides are "immunologically cross-reactive" when both polypeptides react with the same polyclonal antiserum.

### General Methods

The practice of the present invention employs, unless otherwise indicated, conventional molecular biology, microbiology, and recombinant DNA techniques within the skill of the art. Such techniques arc well known to the skilled worker and are explained fully in the literature. See, e.g., Maniatis, Fritsch & Sambrook. "Molecular Cloning: A Laboratory Manual" (1982): "DNA Cloning: A Practical Approach," Volumes I and II (D.N. Glover, ed., 1985); "Oligonucleotide Synthesis" (M.J. Gait, ed., 1984); "Nucleic Acid Hybridization" (B.D. Hames & S.J. Higgins, eds., 1985): "Transcription and Translation" (B.D. Hames & S.J. Higgins, eds., 1984); "Animal Cell Culture" (R.I. Freshney, ed., 1986); "Immobilized Cells and Enzymes" (IRL Press, 1986); B. Perbal, "A Practical Guide to Molecular Cloning" (1984), and Sambrook, et al., "Molecular Cloning: a Laboratory Manual" (1989).

### pp32 Related Genomic DNA

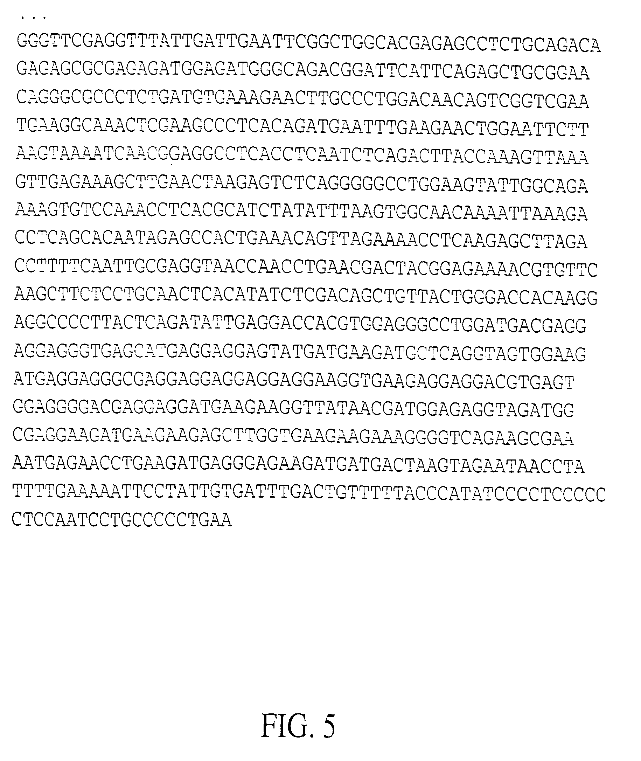

Screening a human genomic library in bacteriophages with probes generated from human pp32 cDNA yielded a new sequence that contained an open reading frame encoding a protein homologous with pp32 (see Example 2: pp32 sequence, reported in Chen, et al., *Mol. Biol. Cell,* **7**:2045-2056, 1996). While the pp32r1 and pp32r2 sequences (see Figures 2 and 5) are substantially homologous to pp32, multiple single nucleotide base changes and short deletions suggest that they are encoded by gene distinct from pp32 gene. The pp32 family also includes substantially homologous polypeptides reported by others: HLA-DR associated protein I (Vaesen, 1994), leucine-rich acidic nuclear protein (Matsuoka. 1994), and protein phosphatase 2A inhibitor (Li. 1996).

DNA segments or oligonucleotides having specific sequences can be synthesized chemically or isolated by one of several approaches. The basic strategies for identifying, amplifying and isolating desired DNA sequences as well as assembling them into larger DNA molecules containing the desired sequence domains in the desired order, are well known to those of ordinary skill in the art. See, e.g., Sambrook, et al., (1989); B. Perbal, (1984). Preferably, DNA segments corresponding to all or a part of the cDNA or genomic sequence of pp32r1 may be isolated individually using the polymerase chain reaction (M.A. Innis, et al.. "PCR Protocols: A Guide To Methods and Applications." Academic Press. 1990). A complete sequence may be assembled from overlapping oligonucleotides prepared by standard methods and assembled into a complete coding sequence. See, e.g., Edge (1981) Nature 292:756: Nambair, et al. (1984) Science 223:1299; Jay, et al. (1984) J. Biol. Chem., 259:6311.

The assembled sequence can be cloned into any suitable vector or replicon and maintained there in a composition which is substantially free of vectors that do not contain the assembled sequence. This provides a reservoir of the assembled sequence, and segments or the entire sequence can be extracted from the reservoir by excising from DNA in the reservoir material with restriction enzymes or by PCR amplification. Numerous cloning vectors are known to those of skill in the art, and the selection of an appropriate cloning vector is a matter of choice (see, e.g., Sambrook, et al., incorporated herein by reference). The construction of vectors containing desired DNA segments linked by appropriate DNA sequences is accomplished by techniques similar to those used to construct the segments. These vectors may be constructed to contain additional DNA segments, such as bacterial origins of replication to make shuttle vectors (for shuttling between prokaryotic hosts and mammalian hosts), etc.

Procedures for construction and expression of proteins of defined sequence are well known in the art. A DNA sequence encoding pp32r1, pp32r2, or an analog of either pp31R1 or pp32r2, can be synthesized chemically or prepared from the wild-type sequence by one of several approaches, including primer extension, linker insertion and PCR (see, e.g., Sambrook, et al.). Mutants can be prepared by these techniques having additions, deletions and substitutions in the wild-type sequence. It is preferable to test the mutants to confirm that they are the desired sequence by sequence analysis and/or the assays described below. Mutant protein for testing may be prepared by placing the coding sequence for the polypeptide in a vector under the control of a promoter, so that the DNA sequence is transcribed into RNA and translated into protein in a host cell transformed by this (expression) vector. The mutant protein may be produced by growing host cells transfected by an expression vector containing the coding sequence for the mutant under conditions whereby the polypeptide is expressed. The selection of the appropriate growth conditions is within the skill of the art.

The assembled sequence can be cloned into any suitable vector or replicon and maintained there in a composition which is substantially free of vectors that do not contain the assembled sequence. This provides a reservoir of the assembled sequence, and segments or the entire sequence can be extracted from the reservoir by excising from DNA in the reservoir material with restriction enzymes or by PCR amplification. Numerous cloning vectors are known to those of skill in the art, and the selection of an appropriate cloning vector is a matter of choice (see, e.g., Sambrook, et al., incorporated herein by reference). The construction of vectors containing desired DNA segments linked by appropriate DNA sequences is accomplished by techniques similar to those used to construct the segments. These vectors may be constructed to contain additional DNA segments, such as bacterial origins of replication to make shuttle vectors (for shuttling between prokaryotic hosts and mammalian hosts), etc.

### Producing the Recombinant Peptide

Preferably, DNA from the selected clones should be subcloned into an expression vector, and the protein expressed by cells transformed with the vector should be tested for immunoreactivity with antibodies against the recombinant protein of this invention prepared as described below. Such subcloning is easily within the skill of the ordinary worker in the art in view of the present disclosure. The amino acid coding region of the DNA sequence of this invention may be longer or shorter than the coding region of the disclosed sequence, so long as the recombinant peptide expressed by the DNA sequence retains at least one epitope cross-reactive with antibodies which are specifically immunoreactive with pp32r1, pp32r2, or other pp32 variant as desired. The preparation of selected clones which contain DNA sequences corresponding to all or part of the sequence of pp32r1 or pp32r2 may be accomplished by those of ordinary skill in the art using conventional molecular biology techniques along with the information provided in this specification.

It is possible to purify a pp32 variant protein, such as pp32r1. which is cross-reactive with antibodies specific for pp32, from an appropriate tissue/fluid source; however, a cross-reactive pp32 variant, or analog thereof, may also be produced by recombinant methods from a DNA sequence encoding such a protein or polypeptide. Polypeptides corresponding to the recombinant protein of this invention may be obtained by transforming cells with an expression vector containing DNA from a clone selected from an mammalian (preferably human) library as described herein. Suitable expression vector and host cell systems are well known to those of ordinary skill in the art, and are taught, for instance, in Sambrook, et al., 1989. The peptide may be obtained by growing the transformed cells in culture under conditions wherein the cloned DNA is expressed. Of course, the peptide expressed by the clone may be longer or shorter than pp32r1 or pp32r2, so long as the peptides are immunologically cross-reactive. Depending on the expression vector chosen, the peptide may be expressed as a fusion protein or a mature protein which is secreted or retained intracellularly, or as an inclusion protein. The desired polypeptides can be recovered from the culture by well-known procedures, such as centrifugation, filtration, extraction, and the like, with or without cell rupture, depending on how the peptide was expressed. The crude aqueous solution or suspension may be enriched for the desired peptide by protein purification techniques well known to those skilled in the art. Preparation of the polypeptides may include biosynthesis of a protein including extraneous sequence which may be removed by post-culture processing.

Using the nucleotide sequences disclosed herein and the polypeptides expressed from them, antibodies can be obtained which have high binding affinity for pp32r1 or pp32r2, but much lower affinity for pp32 and/or other variants of pp32. Such antibodies, whether monoclonal or purified polyclonal antibodies can be used to specifically detect pp32r1 or pp32r2. Techniques for preparing polypeptides, antibodies and nucleic acid probes for use in diagnostic assays, as well as diagnostic procedures suitable for detection of pp32 are described in U.S. Patent Nos. 5,726,018 and 5,734,022, and these techniques may be applied to pp32r1 or pp32r2 by substitution of the nucleic acid sequences disclosed herein. Similar substitution may be applied to other variants of pp32.

### pp32r1 Promoter Sequence

Multiple consensus sequences for binding active steroid receptors found in genomic sequences upstream from the pp32rl coding region are consistent with hormone regulation of gene expression. The consensus sequences were associated with the both induction and repression of expression by steroid hormones. The combination of both positively and negatively acting elements suggests complex regulation of pp32r1 expression.

Possible steroid hormone regulation of pp32r1 expression is important in regard to prostate cancer. While about one-half of treated patients initially respond to androgen ablation, subsequent hormone refraction and continued aggressive tumor growth is common (Gamick. M.B., "Prostate Cancer,'' in Scientific American Medicine, Dale, D.C. and Federman, D.D. Eds., Scientific American Inc., New York. 1995). Many different steroid hormones regulate the growth of prostate cancer cells (Huggins, et al., "Studies on prostate cancer: I. The effect of castration, of estrogen, and of androgen injection on serum phosphatases in metastatic carcinoma of the prostate,'' *Cancer Res.,* 1:293, 1941). These findings established a basis for androgen ablation therapy for the treatment of metastatic prostate cancer.

The present invention provides androgen-activated promoters based on the upstream portion of the genomic sequence in Figure 2. The promoter sequence provided by this invention is bounded at its 3' terminus by the translation start codon of a coding sequence and extends upstream (5' direction) to include at least the number of bases or elements necessary to initiate transcription at levels above background. Within the promoter sequence will be found a transcription initiation site (conveniently defined by mapping with nuclease S1), a protein binding domain (consensus sequence) within about 100 bases upstream of the transcription initiation site generally designated the TATA box (a binding site for TATA box binding proteins and RNA polymerase), and various other protein binding domains (consensus sequences) upstream of the TATA box that modulate the basic transcriptional activity of the transcription initiation site and the TATA box. The various other protein binding domains preferably contain recognition sequences shown in Table 1 for binding (1) androgen receptors, estrogen receptors, glucocorticoid receptors, and progesterone receptors; (2) transcription factors containing the leucine zipper motif including, but not limited to Fos. Jun. JunB. and Myc: and, (3) certain tissue specific transcription factors including, but not limited to GATA-1 and GATA-2. The various other protein binding domains upstream of the TATA box may contribute to specificity (tissue specific expression), accuracy (proper initiation), and strength (transcription frequency) of the promoter. The promoter elements may serve overlapping functions so that the promoter may function in the absence of subsets of these elements.

### Therapy

Inhibition of function of protransforming variants of pp32 by any means would be expected to be an avenue of therapy.

U.S. Patent No. 5,726,018, describes various therapeutic avenues which may be applied by the skilled worker based on the nucleotides and protein sequences disclosed herein. In a particular embodimenl all or a portion of the sequence of pp32r1 or pp32r2 may be supplied in the antisense orientation to block expression of the variants found in carcinomas, particularly prostate cancer. Suitable methods for preparation of antisense expression vectors and administration of antisense therapy may be found in U.S. Patent No. 5,756,676. Prescreening of the patient population using the diagnostic methods described herein to identify patients having tumors expressing the particular pp32 variant is preferred.

Screening for compounds having therapeutic effects in prostate cancer mav also be facilitated by the present invention. Studies which may be used to screen candidate compounds are described in U.S. Patent No. 5,756,676, modified by the use of cell lines which express particular variants of pp32 (see, e.g., Examples below). Compounds which affect steroid dependent protein expression may also be detected according to this invention by similar screening studies using an androgen-activated promoter as provided herein operatively coupled to a DNA sequence whose expression may be detected. (Marker sequences are well known in the art, see, e.g., Sambrook, et al., and selection of an appropriate detectable expression marker is a routine matter for the skilled worker.) Screening by testing the effect of candidate compounds on recombinant cells containing an expression vector having an androgen-activated promoter operatively coupled to an expression marker, with appropriate controls, is within the skill of the art, in view of the promoter sequences provided herein. In one aspect, this invention provides a method for screening candidate compounds for pharmacological activity by (1) culturing a cell transfected with the DNA molecule containing an androgen-activated transcriptional promoter which is operatively linked to an open reading frame comprising at least one exon of a protein coding sequence and (2) determining expression of the open reading frame in the presence and absence of the compound. In a preferred mode the androgen activated promoter may be the portion of the sequence in Figure 2 which is upstream of the translation initiation site, or alternatively the androgen activated promoter may be the 2700 bp upstream from the translation initiation site.

### Diagnostic Methods Based on the pp32 Gene Family

In one aspect, this invention provides methods for detecting and distinguishing among members of the pp32 gene family. As explained herein, the presence of one or more members of the gene family may be detected using assays based on common structures among the members resulting from common or similar sequences. For example, polyclonal antibodies elicited by pp32 will cross-react with pp32r1 and pp32r2, including various alleles of these pp32 variants. Similarly, the full coding region of the pp32 cDNA will hybridize under suitable conditions with nucleic acid encoding any of the variants, as shown by the *in situ* detection of the variants in tumor sections which were subsequently shown to contain either pp32r1 or pp32r2 allelic forms (Example 1). Selection of conditions that promote the immune cross-reactivity or cross-hybridization necessary for such detection is within the skill of the art, in view of the examples provided herein. For example, by using large nucleotide probes in hybridization experiments, the effects of one or a few differences in sequence may be overcome, i.e.. larger probes will bind to more dissimilar target sequences, in contrast to shorter probes for which each nucleotide makes a larger percentage contribution to the affinity, and a single nucleotide alteration will cause a greater relative reduction in hybridization efficiency. Typically probes of 50 or more nucleotides are used to find homologues to a given sequence, and the studies reported in Example 1 used the entire sequence of pp32 as a probe to find cells expressing homologous members of the gene family other than pp32. Likewise, polyclonal antisera elicited to an antigen having multiple epitopes is more likely to cross-react with a second antigen that has a few of the same epitopes along with many different epitopes, while a monoclonal antibody or even a purified polyclonal antiserum might not bind to the second antigen.

In addition to determining the presence of one or more members of the pp32 gene family this invention also provides methods for distinguishing among members. Determining which pp32 variant may be useful, for instance to determine whether a transformation promoting or suppressing variant is present in a tissue sample. Suitable methods for distinguishing include both immunoassay and nucleic acid binding assays. Preferred are methods which can detect a 10-fold difference in the affinity of the detecting ligand (e.g., antibody or oligonucleotide) for the target analyte. Such methods are well documented for other systems, and may be adopted to distinguish between pp32 variants by routine modification of such methods in view of the guidance provided herein.

Protein level assays may rely on monoclonai or purified polyclonal antibodies of relatively greater affinity for one variant compared to another (see, e.g.. Smith, et al. ("Kinetics in interactions between antibodies and haptens.'' Biochemistry, 14(7):1496-1502. 1975. which shows that the major kinetic variable governing antibody-hapten interactions is the rate of dissociation of the complex, and that the strength of antibody-hapten association is determined principally by the activation energy for dissociation), and Pontarotti, et al. ("Monoclonal antibodies to antitumor Vinca alkaloids: thermodynamics and kinetics," Molecular Immunology, 22(3):277-84, 1985, which describes a set of monoclonal antibodies that bind various dimeric alkaloids and can distinguish among the alkaloid haptens due to different relative affinities of the various monoclonal antibodies for particular dimeric alkaloids), each of which is incorporated herein by reference). Suitable modifications of the conditions for immunoassays to emphasize the relative affinity of monoclonal antibodies with different affinity are also discussed in U.S. Patent No. 5.759.791.

A number of methods are available which are capable of distinguishing between nucleic acid sequences which differ in sequence by as little as one nucleotide. For example, the ligase chain reaction has been used to detect point mutations in various genes (see. e.g., Abravaya, et al., "Detection of point mutations with a modified ligase chain reaction (Gap-LCR)," *Nucleic Acids Research,* 23(4):675-82, 1995, or Pfeffer, et al., "A ligase chain reaction targeting two adjacent nucleotides allows the differentiation of cowpox virus from other Orthopoxvirus species," *Journal of Virological Methods*, 49(3):353-60, 1994). Amplification of a sequence by PCR also may be used to distinguish sequences by selection of suitable primers, for example, short primers, preferably 10-15 matching nucleotides, where at least one of the primers has on the 3' end a unique base that matches one variant but not other variants, and using annealing conditions under which the primer having the unique base has at least a ten-fold difference in dissociation rate between the fully matching variants and variants which do not fully match. Similar differentiation may be achieved in other methods dependent on hybridization by using short probes (typically under 50bp, preferably 25bp or less more preferably less than 20bp or even 10-12 bp) by adjusting conditions in hybridization reactions to achieve at least a ten-fold difference in dissociation rate for the probes between the fully matching variants and variants which do not fully match. Cleavase fragment length polymorphism may also be used, and a specific example below provides guidance from which the skilled worker will be able to design similar studies by routine selection of other cleavase enzymes in view of the sequences provided herein.

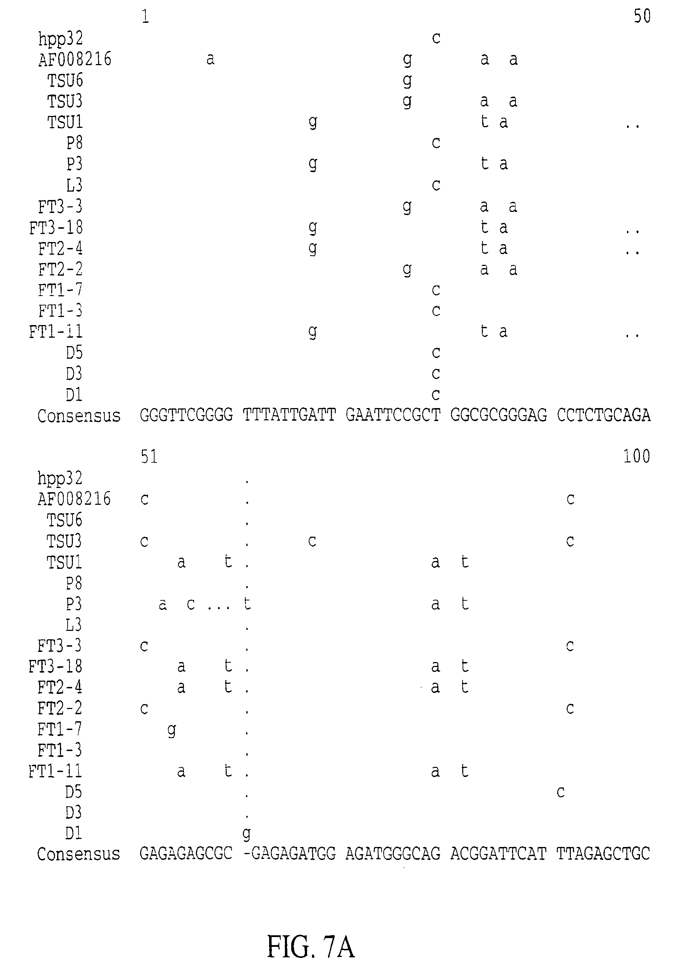

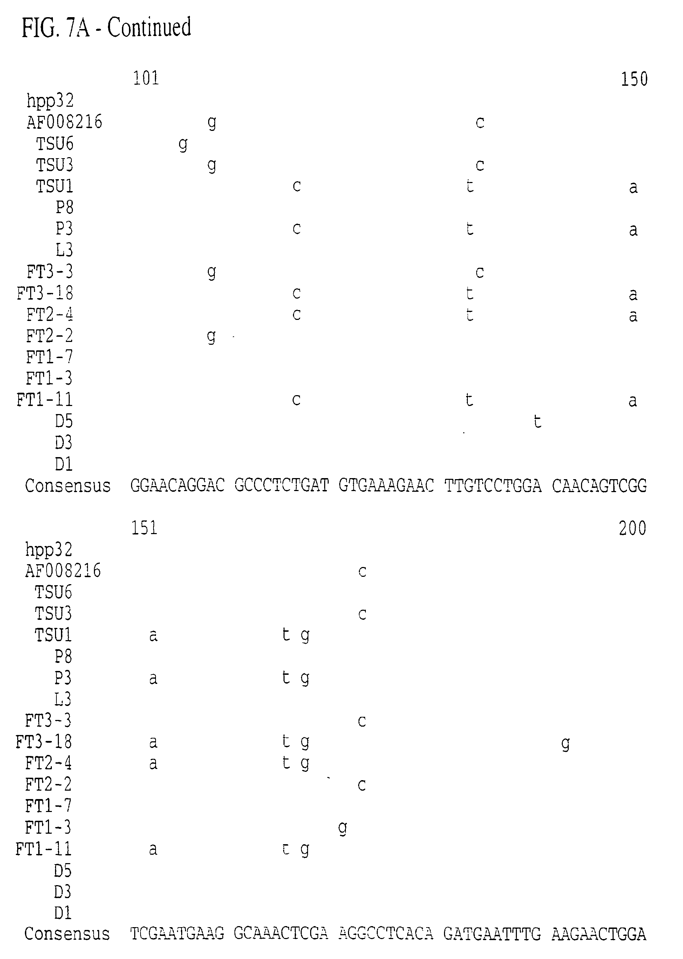

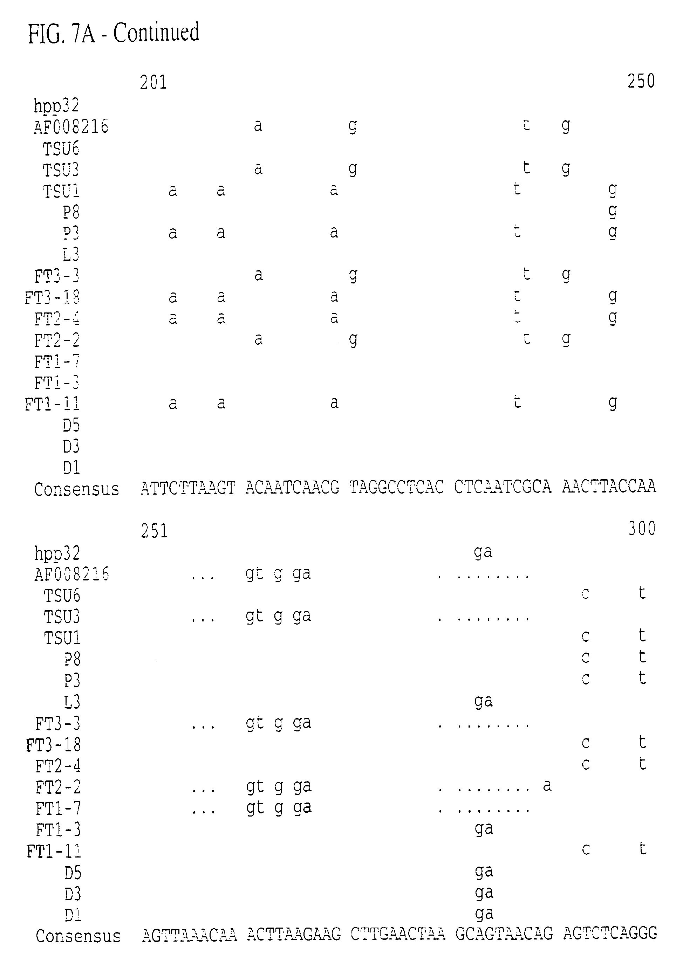

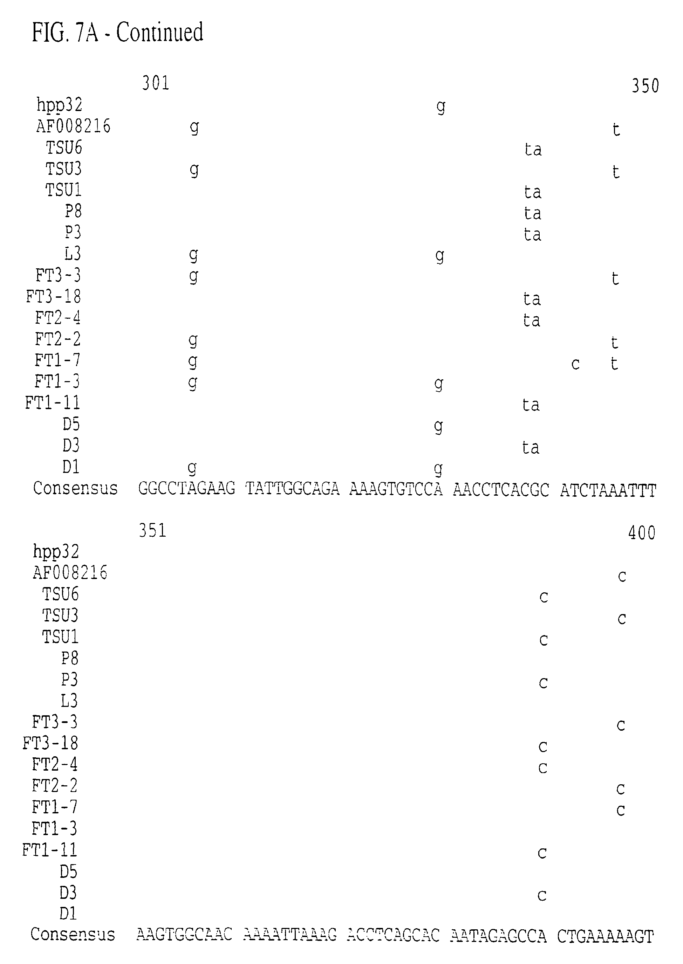

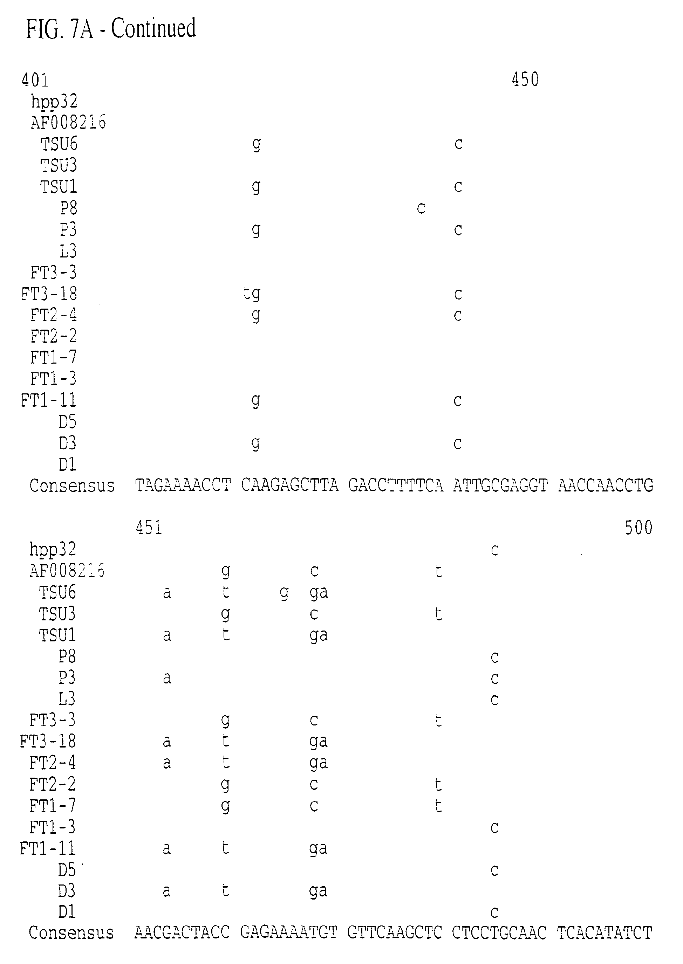

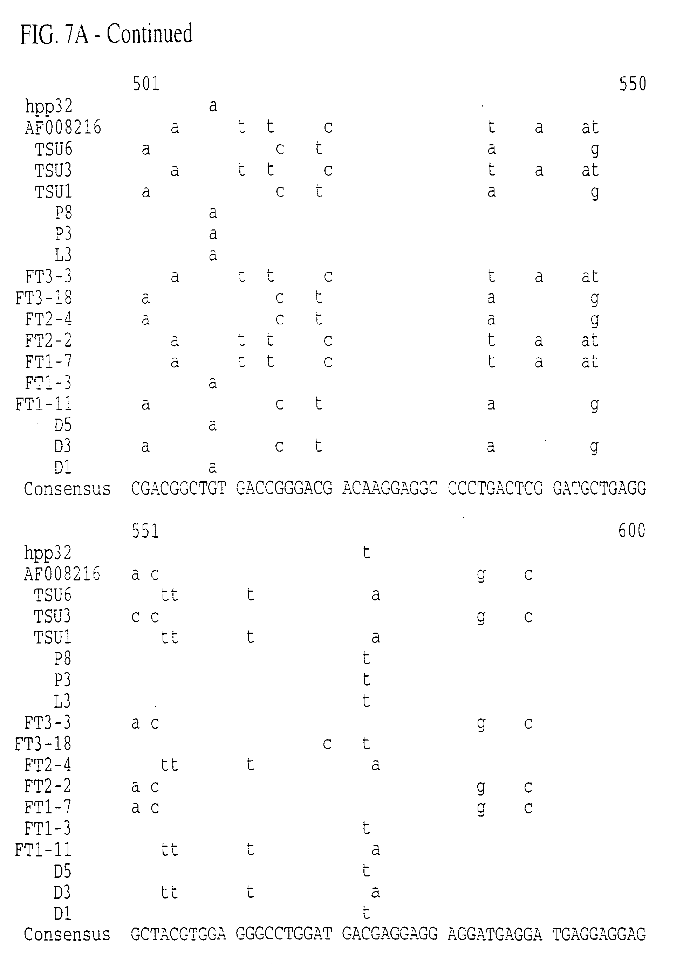

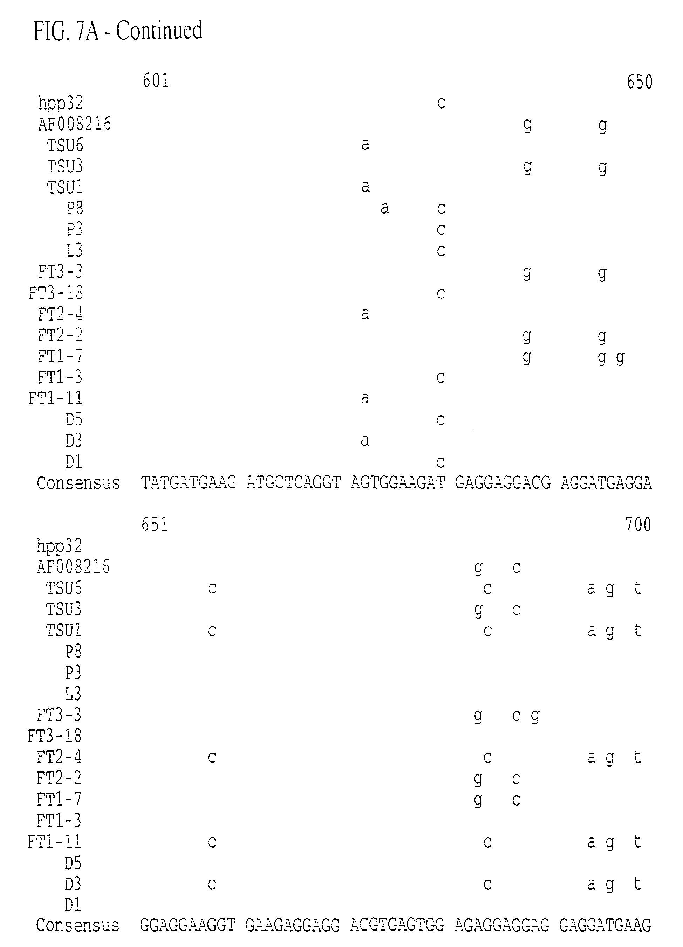

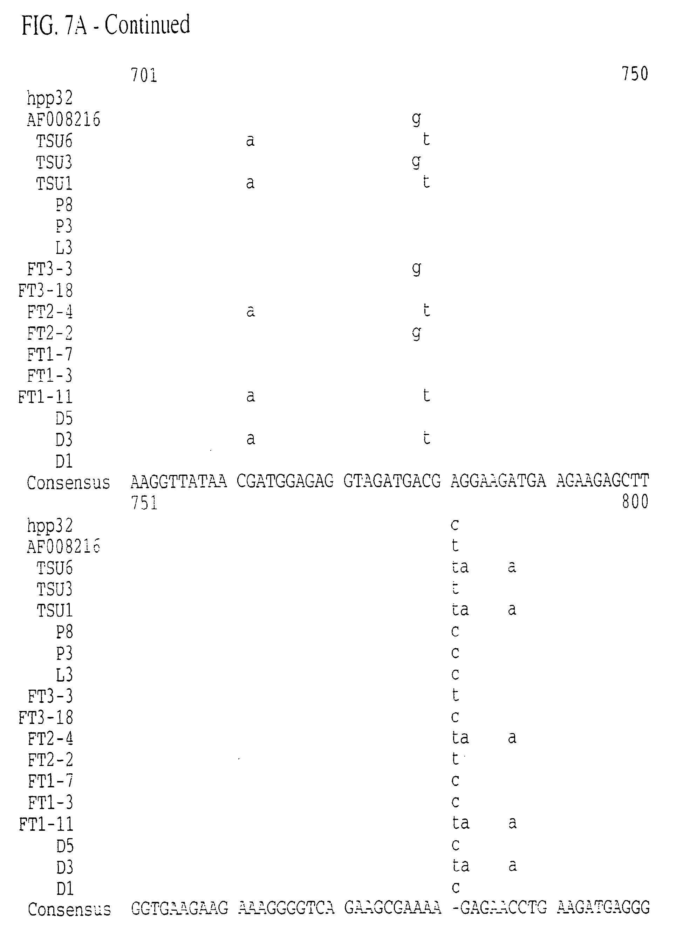

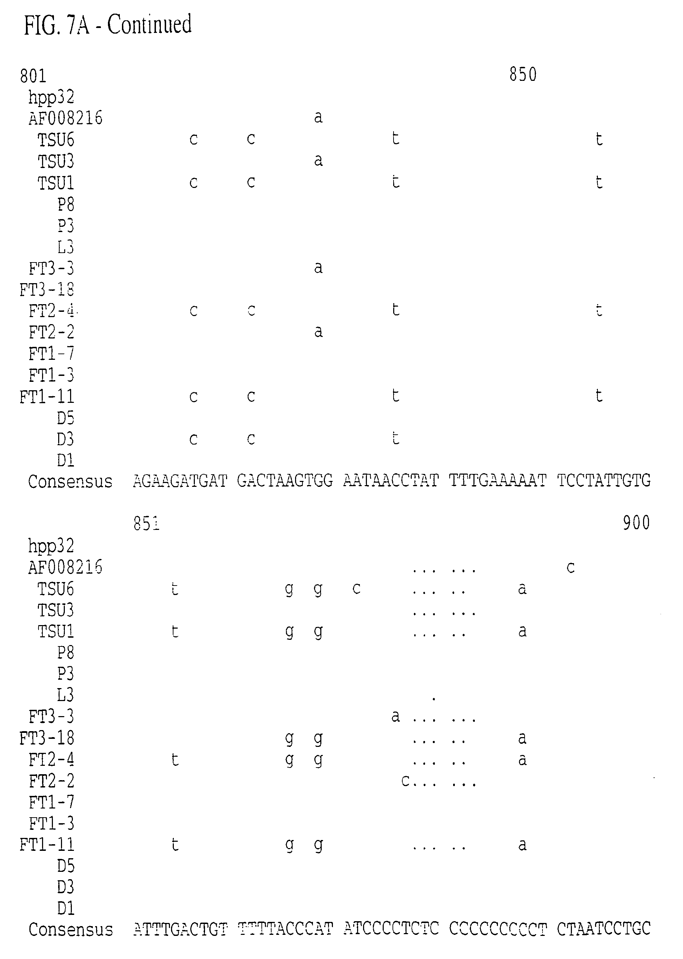

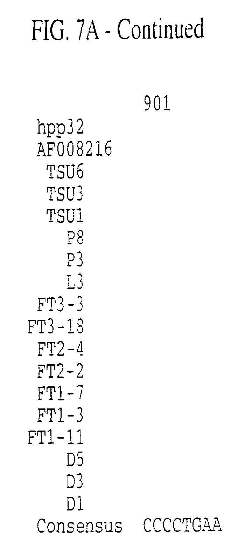

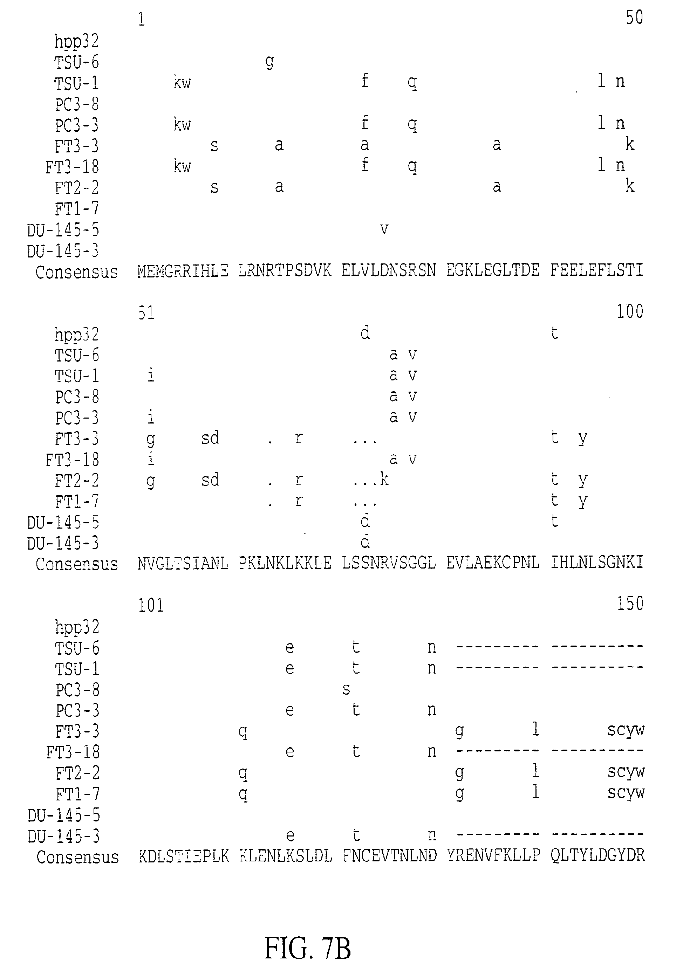

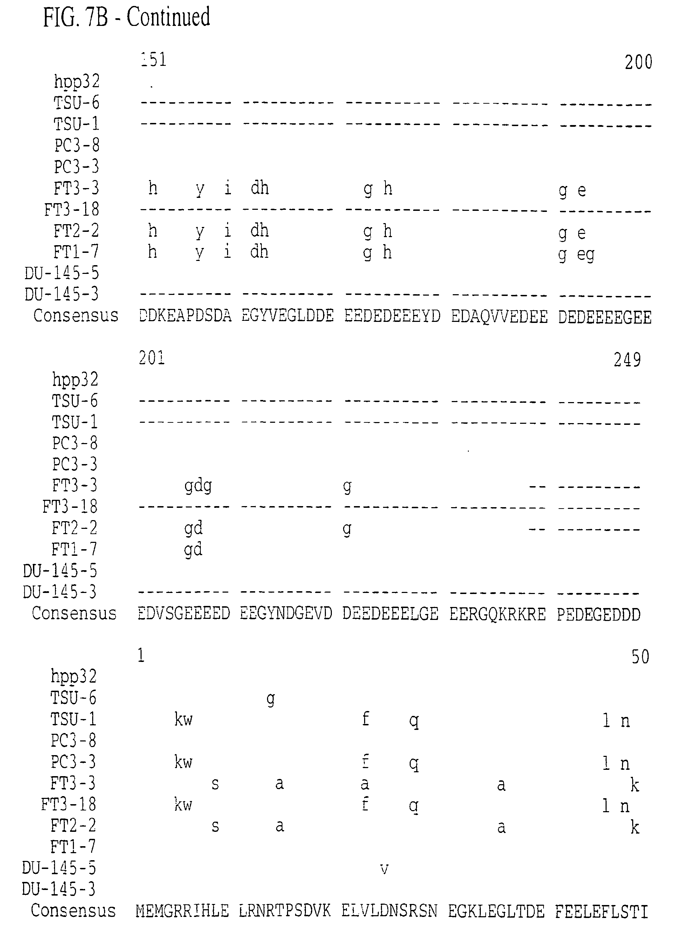

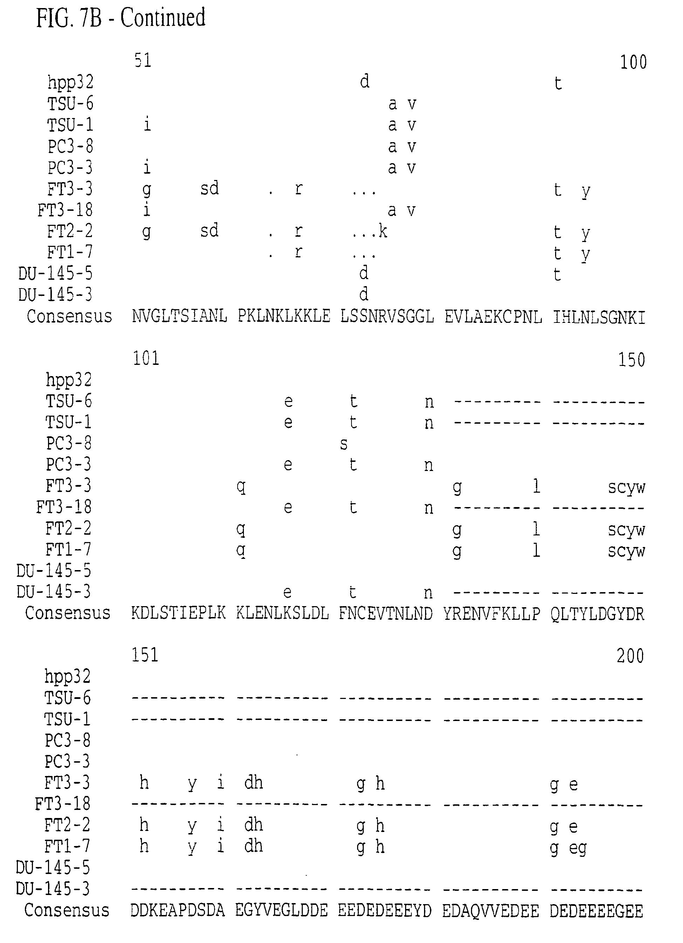

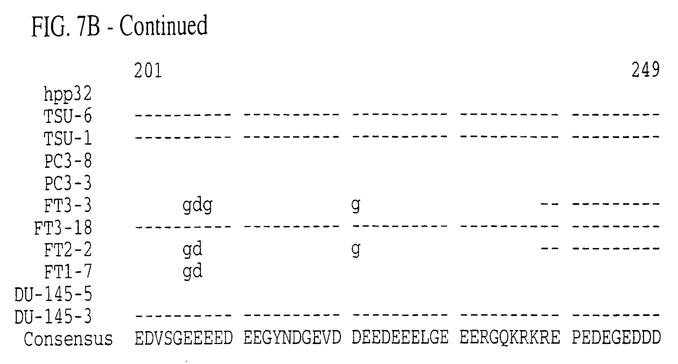

The diagnostic methods of this invention may be used for prognostic purposes and patient differentiation as described herein. In particular, the methods of this invention allow differentiation between products expressed from the various sequences disclosed in Figure 7. Preferred methods are those that detect and/or differentiate between pp32, pp32r1, and/or pp32r2. Situations in which differentiation between pp32 variants will be of benefit will be readily apparent to the skilled clinician in view of the present disclosure. Selection among the diagnostic methods provided by this invention of a suitable technique to achieve the desired benefit is a routine matter for the skilled clinician.

### EXAMPLES

In order to facilitate a more complete understanding of the invention, a number of Examples are provided below. However, the scope of the invention is not limited to specific embodiments disclosed in these Examples, which are for purposes of illustration only.

### Example 1. Cellular Location of pp32 Expression

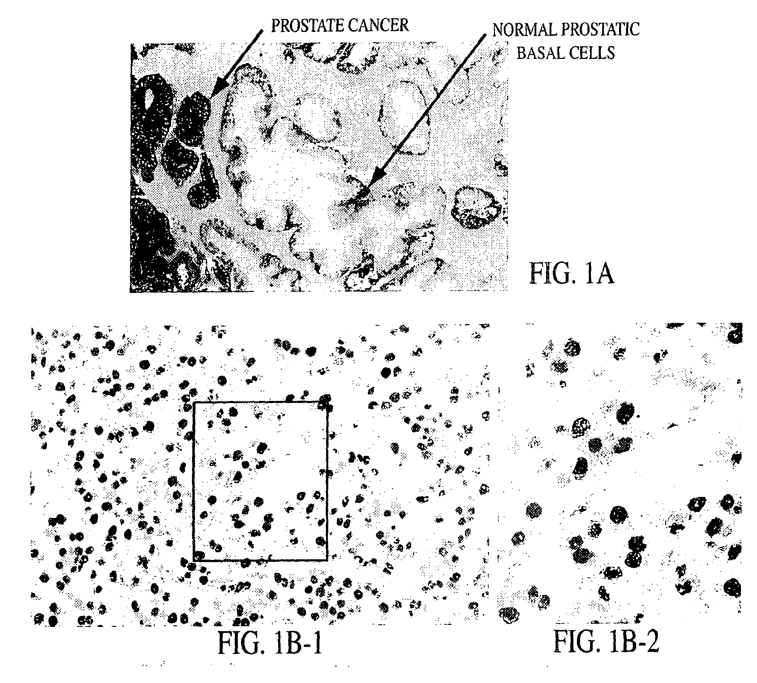

pp32 mRNA can be detected by *in situ* hybridization with a pp32 probe under stringent conditions.
In situ hybridization. Bases 1-298 of the pp32 cDNA sequence (GenBank 11SU73477) were subcloned into the Bluescript vector by standard techniques. Digoxigenin labeled anti-sense and sense RNA probes were generated using a commercially available kit (Boehringer Mannheim). Vector DNA linearized with BamHI and XhoI served as template for antisense and sense probe generation respectively. In vitro transcription was performed for 2 hours at 37° in a final volume of 20 µl which contained 1 µg of template DNA, 2 U/µl of either T3 or T7 RNA polymerase, 1 U/µl ribonuclease inhibitor, mM each of ATP, CTP, GTP, 0.65 mM UTP. 0.35 mM digoxigenin-11-UTP, 40 mM Tris-HCl pH 8.0. 10 mM NaCl, 10 mM DTT. 6 mM MgCl₂ and 2 mM spermidine. The reaction was stopped by adding 2 µl of 0.2M EDTA, pH 8.0 and the synthesized transcripts were precipitated for 30 min at -70° with 2.2 µl of 4 M LiCl and 75 µl of pre-chilled ethanol. RNA was pelleted by centrifugation, washed with 80% ethanol, mildly dried and dissolved in 100 µl of DEPC treated water. Yields of labeled probe were determined by an enzyme linked immunoassay using a commercially available kit (Boehringer Mannheim). Nonradioactive in situ hybridization was performed with anti-sense and sense pp32 RNA probes generated by in vitro transcription (see U.S. Patent No. 5.726.018). Figure 1A shows that normal prostatic basal cells are positive, whereas the clear, differentiated glandular cells are negative. In contrast prostatic adenocarcinoma, shown at left, is strikingly positive. Note that the signal is cytoplasmic since it is mRNA and not the protein that is detected in this assay.

pp32 displays a distinctive pattern of expression *in vivo* (Chen, et al.: Malek, et al., "Identification and preliminary characterization of two related proliferation-associated nuclear phosphoproteins." *Journal of Biological Chemistry,* 265:13400-13409, 1990; Walensky, et al., "A novel M(r) 32,000 nuclear phosphoprotein is selectively expressed in cells competent for self-renewal," *Cancer Research* 53-4720-4726, 1993). In normal peripheral tissues, expression is restricted to stem-like cell populations such as crypt epithelial cells in the gut and basal epithelium in the skin: in the adult central nervous system, cerebral cortical neurons and Purkinje cells also express pp32. In normal prostate, basal cells express pp32, whereas pp32 mRNA is not detectable by *in* situ hybridization in differentiated glandular cells (Figure 1A). In contrast, strong in situ hybridization to pp32 probes is found in nearly all clinically significant human prostatic adenocarcinomas. 87% of human prostatic adenocarcinomas of Gleason Score 5 and above express mRNA that hybridizes strongly with probes to pp32 in contrast to only 11% of prostate cancers of Gleason Score 4 and below in a study of 55 patients.
**Immunohistochemistry.** Formalin-fixed, paraffin-embedded tissue was sectioned at 4 µM, deparaffinized, hydrated, processed for heat-induced antigen retrieval at 95° in 0.01 M citrate buffer, pH 6.0, for 20 min (Cattoretti, et al., "Antigen unmasking on formalin-fixed, paraffin-embedded tissue sections," *Journal of Pathology* 171:83-98, 1993), then incubated overnight at room temperature with a 1/20 dilution of anti-pp32 antibody. Following washing, the slide was sequentially developed with biotinylated swine-anti-rabbit IgG at 1/100 (Dako), strepavidin peroxidase (Dako), and diaminobenzidine. Figure 1B shows a representative high-grade human prostate cancer stained with affinity-purified rabbit polyclonal anti-pp32 antibody (Gusev, et al., "pp32 overexpression induces nuclear pleomorphism in rat prostatic carcinoma cells," *Cell Proliferation* 29:643-653, 1996). The left-hand panel shows a representative field at 250x: the rectangle indicates the area show in computer generated detail in the right-hand panel. Strongly hybridizing tumors show intense immunopositivity with antibodies to pp32. indicating that they express pp32 or immunologically related proteins (Figure 1A and 1B).

### Example 2. ESTs corresponding to pp32

Several potential variant pp32 species have been identified in the prostate cancer expressed sequence tag libraries of the NCI's Cancer Genome Anatomy Project. Clone 588488 encodes a protein that is 96% identical to APRIL, although absent retrieval and sequencing of the full clone, it is impossible to tell whether the entire EST clone encodes a pp32 related sequence; neither is it possible to assess the biologic function of this molecule at this time. Nevertheless, it is apparent that the sequenced portion encodes a protein bearing great similarity to pp32. This EST does not appear in the database for normal prostate. As with the variant pp32 species recovered from prostate cancer, generation of this molecule by mutation would require a complex mechanism.

pp32-related genes are present in other organisms. The existence of a pp32 gene family in rodent would be consistent with the existence of a comparably sized family in human. A murine pp32 (GenBank U73478) has 89% amino acid identity to pp32, but less identity to pp32r1 and APRIL. (The murine cerebellar leucine rich acidic nuclear protein has a single amino acid substitution relative to murine pp32.) We additionally identified a murine EST, GenBank AA066733, with closest identity to APRIL protein at 85% identity over 148 amino acids of a predicted open reading frame. Several other murine EST's. AA212094 and W82526 are closely related to the pp32 family but are not significantly more related to either pp32, pp32r1, or APRIL. A human homoiogue of such a gene would be expected to encode a fourth member of this gene family. We identified EST's predicted to encode pp32-related proteins in *C*. *elegans,* schistosomes, zebrafish, and *Drosophila* (data not shown). However, these sequences may not represent the complete extent of the pp32 gene family in these organisms, and thus are not informative for the likely size of the mammalian pp32 gene family.

### Example 3. The Structure of a Gene Encoding a Relative of the pp32 Family

Screening a human genomic library in bacteriophages with probes generated from human pp32 cDNA yielded a new sequence that contained an open reading frame encoding a protein homologous with pp32.

### Screening a Human Genomic Library in Bacteriophages for pp32 cDNA.

A genomic library from human placenta in the Lambda Fix II vector was expressed in *E. coli* strain XL-1 Blue MRA (Stratagene #946206). Screening for bacteriophage clones containing DNA inserts homologous with pp32 cDNA followed routine procedures (Sambrook, et al.). Briefly, nitrocellulose filters that had overlain bacteriophage plaques were hybridized with P-32 labeled probes for pp32 cDNA. The probes were prepared by the random primer method (Stratagene #300385) using pp32 cDNA as a template (Chen, et al., *Molec. Biol. Cell,* **7**:2045-2056, 1996.). Reactive bacteriophage plaques were plugged and the bacteriophages were eluted, reexpressed, and rescreened with pp32 cDNA probes until pure. Bacteriophage DNA was prepared by the plate lysate method (Sambrook, et al.).

### Identifying Restriction Fragments within Bacteriophage DNA Containing Sequences Homologous with pp32 cDNA.

DNA from a bacteriophage clone containing pp32 cDNA sequences was digested with HindIII. Using routine methods, the restriction fragments were separated by agarose gel electrophoresis, transferred in alkaline buffer to positively charged nylon filters, and hybridized with probes that were selective for the 5' and 3' ends of the pp32 cDNA (Sambrook, et al.). The 5' and 3' probes were prepared as described above except that the products of polymerase chain reactions (PCR) were used as templates for the labeling reactions (Saiki, et al.. *Science,* **239**:487-491, 1988.). One PCR product was a 249 base pair segment of pp32 cDNA containing nucleotides 32 through 279. It was the result of a reaction using a pp32cDNA template and the primers
5'-TATGCTAGCGGGTTCGGGGTTTATTG-3' and
5'-GATTCTAGATGGTAAGTTTGCGATTGAGG-3' (primer set A).
The other product was a 263 base pair segment of pp32 cDNA including nucleotides 677 through 938. It was the result of a reaction using a pp32 cDNA template and the primers
5'-GAATCTAGAAGGAGGAGGAAGGTGAAGAG-3' and
5'-CTATCTAGATTCAGGGGGCAGGATTAGAG-3' (primer set B).
The PCR reactions included 35 cycles of one minute denaturations at 95°C. one minute primer annealings at 50°C. and one minute extensions at 72°C (cycling program A). A 4.7 kb HindIII restriction fragment that hybridized with the 5' probe, but not with the 3' probe and a 0.9 kb HindIII fragment that hybridized with the 3' probe, but not with the 5' probe were subcloned into pBluescript (Gibco) by routine methods (Sambrook, et al.). The nucleotide sequences of both strands of purified plasmid DNA containing the inserts were determined by automated procedures (DNA Analysis Facility, Johns Hopkins University School of Medicine).

**Completion of Sequencing by Direct Sequencing of PCR Products.** Alignment of the sequences of the 4.7 and 0.9 kb HindIII restriction fragments with pp32 cDNA showed about 90% homologies between the 3' end of the 4.7 kb fragment and the 5' region of pp32 cDNA and the 5' end of the 0.9 kb fragment and the 3' region of the pp32 cDNA. There was an unaligned 199 base pair gap of pp32 cDNA sequence between the ends of the restriction fragments. Primers were designed to specifically anneal to relative pp32 sequences on both sides of the sequence gap. The primer sequences were
5'-GAGGTTTATTGATTGAATTCGGCT-3' and
5'-CCCCAGTACACTTTTCCCGTCTCA-3' (primer set C).
Polymerase chain reactions followed cycling program A with primer set C and pure bacteriophage DNA as a template. The 943 base pair products were shown by ethidium bromide staining agarose gels, extracted from excised fragments of low melt agarose (NuSieve) electrophoresis gels, and sequenced by automated procedures as described above.

A sequence of 5,785 bases was obtained from the human placental genomic library bacteriophage clone containing segments homologous with pp32 cDNA (Figure 2). This sequence was deposited in Genbank under Accession No. U71084, Locus HSU71084. The sequence has an open reading frame extending from nucleotides 4.453 to 5.154. Analysis of the nucleotide sequence upstream of the open reading frame revealed consensus sequences for active steroid hormone receptors at over twenty positions (Table 1).

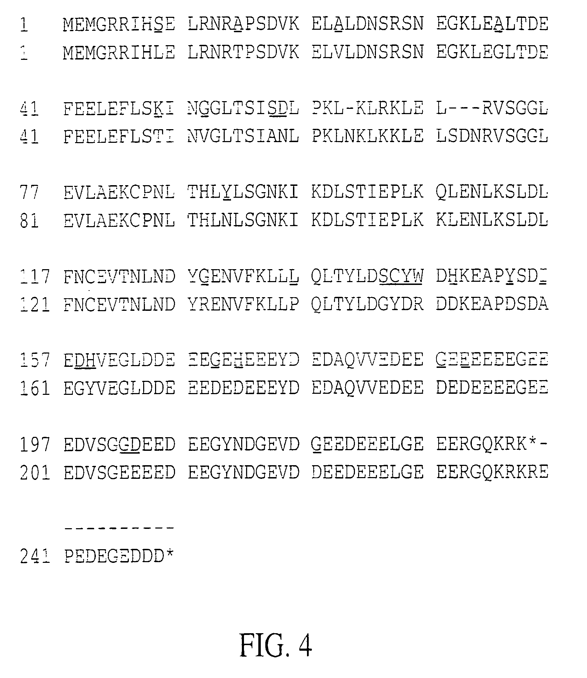

Sequence analysis of the open reading frame showed 94% sequence homology to pp32 (Figure 3). Alignment of the open reading frame sequence to pp32 cDNA revealed 33 scattered, solitary base differences and clustered differences of two and seven bases. There were two internal deletions of three and nine bases. The open reading frame encoded a polypeptide containing 234 amino acid residues with 88% protein-level homology to pp32 (Figure 4). Alignment of the translated sequence to the pp32 amino acid sequence revealed 18 scattered, solitary amino acid residue differences, three differences in clusters of two residues, and one difference in a clusters of four residues. There were two internal deletions of one and three residues and a terminal deletion of eleven residues. The translated sequence contained 69 acidic residues. 26 fewer than pp32.

### Example 4. Chromosome Mapping of pp32r1

The pp32r1 gene maps to chromosome 4 as determined by PCR of the NIGMS monochromosomal panel 2 (Drwinga, et al., "NIGMS human/rodent somatic cell hybrid mapping panels 1 and 2," Genomics 16:311314, 1993) followed by sequencing of the PCR product. Interestingly, the full sequence of pp32r1 including 4364 nucleotides of sequence 5' to the start ATG contained over 400 matches in a blastn search of the non-redundant GenBank database. These matches were to two short regions of about 278 and 252 base pairs (nucleotides 674-952 and 2542-2794) that represent repeats in opposite orientations. The repeats are significantly related to elements on many chromosomes.

The human pp32 gene has been mapped to chromosome 15q22.3-q23 by fluorescence in situ hybridization (Fink, et al.). A Unigene entry for pp32 (Hs. 76689; HLA-DR associated protein 1) lists 93 EST sequences corresponding to this gene. 12 of which contain a mapped sequence-tagged site (STS). These STS sites are all reported to map to chromosome 15. as are many of the pp32 EST's analyzed by electronic PCR (http://www.ncbi.nlm.nih.gov). APRIL protein was also mapped to chromosome 15q25 (Mencinger, et al.: GenBank Y07969).

### Example 5. Sequence Analysis of pp32r2

A pp32-related sequence (designated pp32r2) has been identified on chromosome 12 by methods analogous to those described in Example 2 for isolation of the unique intronless pp32-related gene pp32r1, found on chromosome 4. It was initially thought that the chromosome 12 sequence, encoding a truncated protein, might represent a pseudogene; however that interpretation has been reassessed in view of the present findings. The sequence has been designated pp32r2, and is recorded in Genbank as locus AF008216; the sequence of pp32r2 is shown in Figure 5. By BESTFIT analysis (Genetics Computer Group. Inc., Wisconsin Package, version 9.1. Madison, WI, 1997), pp32r2 is 99.5% identical to FT1.11, FT2.4 and T1, showing four nucleotide differences over the 875 nucleotide overlap of the sequences; this level of variation is consistent with a polymorphism. Similarly. BESTFIT analysis shows that PP32R1 is 99.6 % identical to FT3.3 and 99.4% identical to FT2.2. displaying four and five nucleotide differences, respectively (see Figure 7 below).

### Example 6. Sequence Comparison of Multiple Clones

Screening of a human placental genomic library in Lambda Fix 11 vector (Stratagene #946206) with P-32 labeled probes for pp32 cDNA yielded a clone of approximately 23 kb. 4.7 kb and 0.9 kb HindIII restriction fragments of this clone hybridized with probes for pp32 cDNA. The 4.7 kb clone aligned with the 5' portion of the pp32 cDNA sequence, and the 0.9 kb fragment aligned with the 3' end. A small discontinuity of 0.2 kb was sequenced from a bridging PCR product. No introns were identified.

Cultured cells including the whole human embryonic line FSH173WF and the prostatic cancer cell lines PC-3 and LNCaP (American Type Culture Collection) were grown under recommended tissue culture conditions. Poly A+ RNA was prepared by oligo dT adsorption (MicroFasTrack. Invitrogen) and used as a template for the generation of cDNA through reactions with reverse transcriptase and random hexamers (GeneAmp RNA PCR Kit, Perkin Elmer). The cDNA sequences encoding the open reading frame were amplified by nested PCR using primers specifically selective for the genomic sequence over pp32 sequences. The final 298 base pair products were seen by ethidium bromide staining agarose electrophoretic gels.

Using procedures similar to those described in Example 3. except without the need for nested primers in most cases, transcripts from DU-145 cells and from numerous patients were sequenced for comparison to the transcripts from the above samples. The results are shown in Table 2. A summary of the degree of identity between various transcripts is provided in Table 3.

### Example 7. Sequence Variation for Individual Isolates of Different Cell Lines and Tumor Tissue

The explanation for the apparent discordant expression of pp32 in cancer is that prostate tumors do not generally express pp32, but rather express variant pp32 species that promote transformation, instead of inhibiting it.
**RT-PCR and CFLP.** Sequences were reverse-transcribed and amplified using bases 32 to 52 of HSU73477 as a forward primer and 919 to 938 of the same sequence as a reverse primer in conjuriction with the Titan One-Tube RT-PCR kit (Boehringer). Reverse transcription was carried out at 50° for 45 min followed by incubation at 94° for min; the subsequent PCR utilized 45 cycles of 92° for 45, 55° for 45 sec. and 68° for 1 min with a final extension at 68° for 10 min in a PTC 100 thermocycler (MJ Research). Template RNA was isolated from cell lines or frozen tumor samples using RNAzol B (Tel-Test) according to the manufacturer's instructions, then digested with RNAse-free DNAse I (Boehringer). pCMV32 was used as a positive control without reverse transcription. The cleavage assay was performed according to the manufacturer's specifications (Life Technologies) with digestion at 55° for 10 min at 0.2 mM MnCl₂ and electrophoresed on a 6% denaturing polyacrylamide sequencing gel.

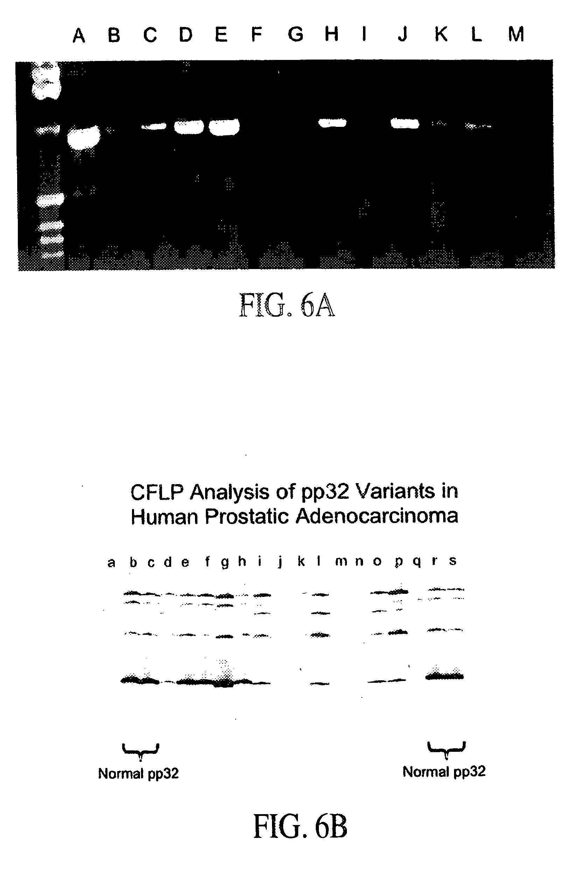

At the level of RTPCR, paired normal prostate and prostatic adenocarcinoma from three patients yielded amplification products (Figure 6A) ranging from 889 to 909 bp. The reaction employed consensus primers capable of ampliring the full-length coding sequence from pp32 and the two closely-related intronless genomic sequences pp32r1 and pp32r2. The sole difference noted was a diminished amplicon yield from normal tissue as compared to neoplastic. Four human prostatic adenocarcinoma cell lines. DU-145, LNCaP, PC-3, and TSUPR-1, also yielded similar products.

Figure 6A shows RT-PCR amplified DNA from human prostate and prostate cancer cell lines. Lane a is an undigested control whose band migrated substantially slower than the digestion produces; samples in all other lanes were digested with cleavage as described. The lanes show: 1 kb ladder (Lifé Technologies), A; pCMV32, B; DU-145, C; LNCaP, D; PC-3. E; TSUPr-1, F; a representative sample, FT-1, without reverse transcription. G; FN-1, H; FT-1, I; FN-2, J; FT-2, K; FN-3, L; FT-3, M; negative control with template omitted. FN indicates frozen benign prostate and the number indicates the patient: FT indicates frozen prostatic adenocarcinoma and the number indicates the patient. Numbers on the left-hand side of the figure indicate the size in kb of a reference 1 kb DNA ladder (Life Technologies).

Qualitative differences between normal and neoplastic tissue began to emerge when the RT-PCR products were subcloned and analyzed by cleavage fragment length polymorphism analysis (CFLP) and sequence analysis. Figure 6B shows a cleavase fragment length polymorphism analysis of cloned cDNA amplified by RT-PCR from human prostatic adenocarcinoma, adjacent normal prostate, and human prostatic adenocarcinoma cell lines using primers derived from the normal pp32 cDNA sequence. The lanes show individual RT-PCR-derived clones from the DU-145, LNCaP, PC-3 and TSUPr1 cell lines, from frozen prostate cancer (FT), and from frozen normal prostate (FN): a, undigested normal pp32 cDNA; b, normal pp32 cDNA: c, DU-145-1; d, DU-145-3; e, DU-145-5; f, LNCaP-3; g, PC3-3; h, PC3-8; i, TSUPrI. -I; j, TSUPrl-3; k, TSUPrl-6; 1, FT1.11; m, FT1.7; n, FT2.2; o, FT2.4; p, FT3.18; q, FT3.3; r, FN3.17; s, FN2.1. LNCaP expresses normal pp32. The band shifts correspond to sequence differences. All clones of RT-PCR product from normal prostate tissue displayed a normal CFLP pattern that corresponded precisely to that obtained from cloned pp32 cDNA template (GenBank HSU73477. Figure 6B). Prostatic adenocarcinomas yielded four distinct CFLP patterns upon similar analysis, of which three were unique and one mimicked the normal pp32 pattern. Examination of DU-145. PC-3, and TSUPR-1 cell lines yielded substantially similar results whereas LnCaP yielded only a normal pp32 CFLP pattern. Further analysis at the sequence level confirmed that normal prostate and LnCaP contained solely normal pp32 transcripts.

Transcripts obtained from prostatic adenocarcinomas and from most cell lines represented closely-related variant species of pp32, summarized in Table I. These transcripts varied from 92.4% to 95.9% nucleotide identity to normal pp32 cDNA (Genetics Computer Group, Inc., Wisconsin Package, version 9.1. Madison, WI, 1997). Of the sixteen variant transcripts obtained, fifteen had open reading frames encoding proteins ranging from 89.3% to 99.6% identity to normal pp32. The table summarizes data obtained for variant pp32 transcripts obtained from human prostatic adenocarcinoma and prostate cancer cell lines. Sequences falling into closely related groups are indicated by the group letters (A,B,C); U indicates unassigned sequences not clearly falling into a group. The origin of each sequence is: FT, frozen tumor followed by patient number, decimal point, and clone number; D, DU-145 followed by clone number (as are all cell line sequences); P, PC3; and T, TSUPrl. Nucleotide identity, gaps in the nucleotide sequence aligninent, and protein identity were determined from BESTFIT alignments with the normal pp32 cDNA and protein sequences. The effect on transformation is described as: stimulates, more foci obtained when transfected with ras+myc than with ras+myc+vector control: inactive, equivalent foci obtained as with ras + myc + vector control; and suppresses, fewer foci obtained as with ras + myc + vector control.

The predicted protein sequences fell into three discrete groups: [1] truncated sequences spanning the N-terminal 131 amino acids of pp32, of which one such sequence substantially equivalent to pp32r2 was obtained identically from two of three patients and from the TSUPR-1 cell line; [2] sequences more closely homologous to a distinct pp32-related gene, pp32r1 than to pp32. and [3] heterogeneous pp32-related sequences. Tumors from two of the three patients analyzed contained no detectable normal pp32 transcripts. Two of twelve cloned transcripts from the third patient tumor were normal by CFLP pattern, with sequence confirmation of normality on one clone. Two clones from cell lines were normal by CFLP screening, but were later shown to represent variant sequences.

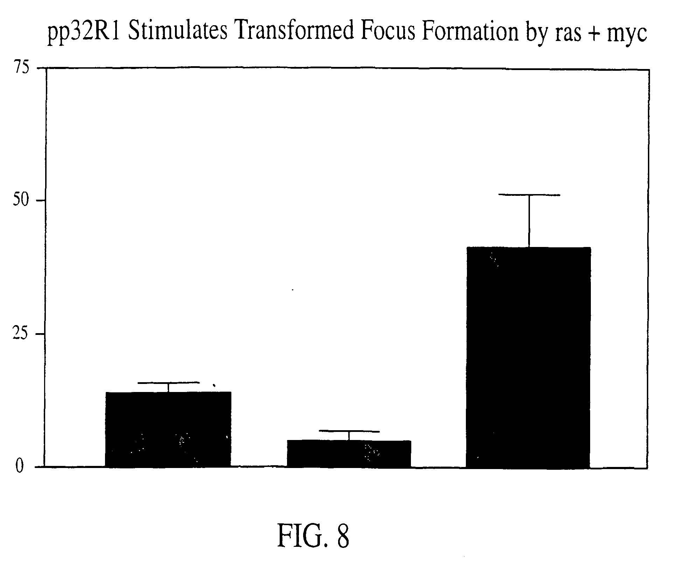

Figures 7A and 7B show a multiple pairwise alignment of nucleotide and predicted protein sequences for all transcripts (Smith, et al., "Identification of common molecular subsequences." *J. Mol. Biol.,* 147:195-197 1981). The figures were compiled with the GCG Pileup and Pretty programs (Smith, et al.). Differences from the consensus sequences are shown as indicated; agreement with the consensus sequence is shown as a blank. Normal human pp32 is designated hpp32. Sequences from the TSUPrl. PC3. and DU-145 cell lines are as indicated. The designation FT indicates sequence derived from a frozen human prostatic adenocarcinoma. Only the normal pp32 sequence, hpp32. was obtained from normal prostate adjacent to tumor tissue. Figure 8A shows alignment of the amplicon nucleotide sequences with pp32 and the predicted amplicon from pp32rl: Figure 8B shows alignment of the predicted protein sequences. One sequence (FT 1.11), independently obtained three times from two separate patients and the TSUPR-1 cell line, is shown only once in the diagram. The pileup and pairwise alignments illustrate several important points: [1] there is a high degree of sequence conservation at both the nucleotide and predicted amino acid levels; [2] the sequence differences are distributed throughout the length of the sequence without obvious hotspots: [3] there is no obvious clustering or segmentation of sequence differences; and [4] the variant sequences fall into the previously described groups. These points are detailed in Figures 8A and 8B.

### Example 8. Diagnostic method to distinguish among family members

The three members of the pp32 family which are expressed in human prostate cancer are pp32, pp32r1 and pp32r2. Whereas pp32 suppresses in vitro transformation and in vivo tumorigenesis in model systems, pp32r1 and pp32r2 are pro-transforming and are tumorigenic in the same systems. It is possible to determine which of the three members is expressed in a tissue sample by using a protocol similar to that described in Example 7.
**Analysis from freshly frozen human tissue and cell lines.** Total RNA is extracted from freshly frozen human tissues or human cancer cell lines and subjected to reverse transcription and polymerase chain reaction amplification with single set of primers capable of amplifying the entire coding region of the cDNA of all the three genes. A suitable set of primers is:
Upper: 5'GGGTTCGGGGTTTATTG3' - This corresponds to bp32 to bp48 of the pp32 cDNA sequence (Genbank U73477)
Lower: 5'CTCTAATCCTGCCCCCTGAA3' - This corresponds to bp919 to bp938 of the pp32 cDNA sequence (Genbank U73477)
The observed amplicon sizes with this primer set are pp32 - 907bp, pp32r1 - 889bp and pp32r2 - 900bp. The three cDNAs are distinguished from each other by restriction enzyme digestion with the following enzymes - EcoR I. Hind III and Xho I. The resultant digest is run on a 2.5% agarose gel to positively identify the three different cDNAs. The table below lists the sizes of the bands observed The bolded numbers indicate the band sizes useful for identification of the three cDNAs.

**Table 4A Expected band sizes upon restriction digestion of the RT-PCR product from fresh tissue and cell lines**

| | Undigested | EcoR I | EcoR I/Hind III | EcoR I/Xho I |
|---|---|---|---|---|
| | | | Double digest | Double digest |
| hpp32 | 907 | 21,**177,709** | 21,**177**.69,**640** | 21,**177,709** |
| pp32r1 | 889 | 21,**177,691** | 21,19,66,**198,427** | 21,**177,691** |
| pp32r2 | 900 | 21,**879** | 21,**244,635** | 21,**385,494** |

**Analysis from formalin fixed and paraffin embedded tissue.** A similar approach is followed for identification of pp32, pp32r1 and pp32r2 transcripts from formalin fixed and paraffin embedded tissues. Total RNA is extracted and subjected to reverse transcription and PCR amplification with a single set of primers capable of amplifying a stretch of 200bp from all the three cDNAs. A suitable set of primers is:
Upper primer - from bp394 to bp414 of the pp32 cDNA sequence (Genbank U73477)
Lower primer - from bp609 to bp629 of the pp32 cDNA sequence (Genbank U73477)
The three cDNAs are distinguished from each other by restriction enzyme digestion with the following enzymes - Hind III, Xho I and BseR I. The resultant digest is run on a 3% agarose gel to positively identify the three different cDNAs. The table below lists the sizes of the bands observed. The bolded numbers indicate the band sizes useful for identification of the three cDNAs.

**Table 5A Expected band sizes upon restriction digestion of the RT-PCR product from formalin fixed and paraffin embedded tissues**

| | Undigested | Hind III | Xho I | BseR I |
|---|---|---|---|---|
| hpp32 | 200 | 200 | 200 | **80,120** |
| pp32r1 | 200 | **100,100** | 200 | 200 |
| pp32r2 | 200 | 200 | **44,156** | 80.120 |

### Example 9. pp32r1 Augments Oncogene-Mediated Transformation of Rat Embryo Fibroblasts.

pp32r1 was subcloned into a eukaryotic expression vector under the CMV promoter and analyzed for its effect on ras + myc-mediated formation of transformed foci in rat embryo fibroblasts. Genomic sequences including the entire coding region for pp32r1 were amplified by PCR and subcloned into the eukaryotic TA cloning and expression vector pCR3.1 vector (Invitrogen) which contains a CMV promoter. The assay was performed as described (Chen et al. Mol Biol Cell. 7:2045-56, 1996) with each T75 flask receiving 5 micrograms of pEJ-ras, and/or 10 micrograms of pMLV-c-mvc, pCMV32, pp32r1 in PCR3.1, or PCR 3.1 alone. After 14 days, transformed colonies were enumerated. Figure 8 shows the results. The data represent the average of seven replicates from two separate experiments in duplicate and one in triplicate. The error bars indicate standard error of the mean. In contrast to pp32, which consistently suppresses focus formation induced by ras + myc and other oncogene pairs, pp32r1 caused a statistically significant stimulation of focus formation with p=.004 by an unpaired t-test.

### Example 10. Effect of Transcripts from Various Cell Lines on Rat Fibroblast Transformation Assays

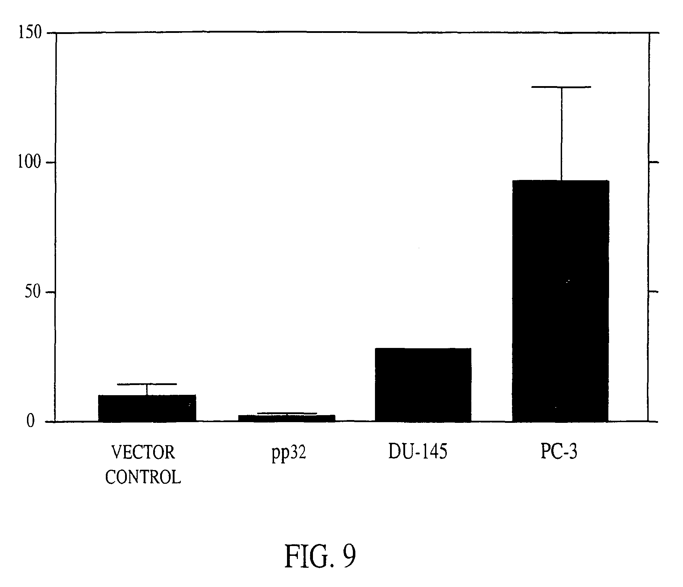

Expression constructs prepared as described above from PC-3 and DU-145 cells were tested in the rat embryo fibroblast transformation assay described by Chen, et al., *Mol Biol Cell.,* **7**:2045-56, 1996, incorporated herein by reference. The results are shown in Figure 9. Transcripts from the two cell lines stimulated ras+myc induction of transformed rat embryo fibroblast foci, in contrast to normal pp32, which suppressed transformation. The figure shows the mean +/- the standard deviation, except for DU-145, which represents a single determination.

### Example 11. Transformation Activity of Various Isolates from Patient Tumors

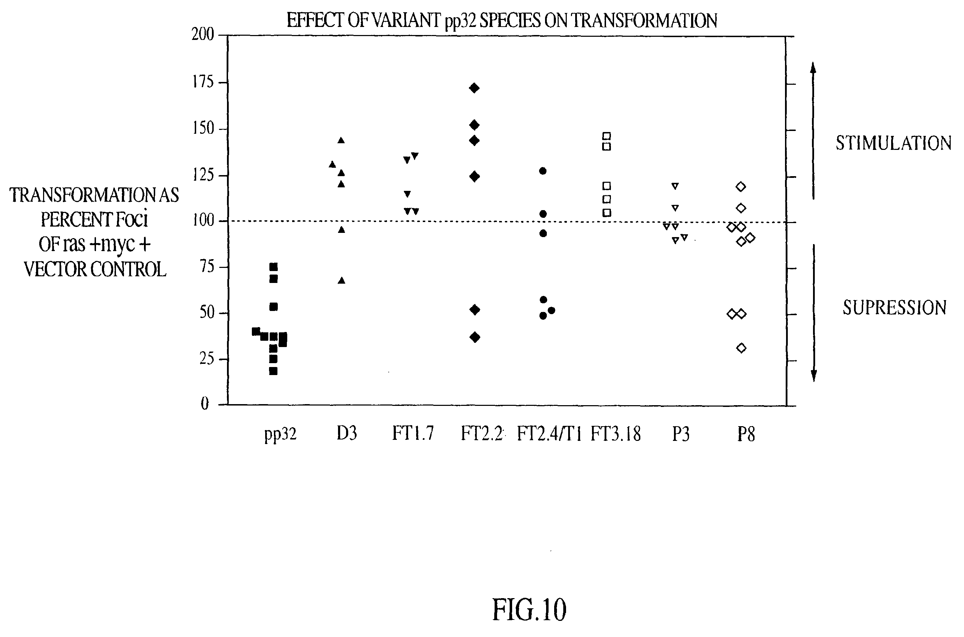

The variant transcripts isolated from prostate cancer patients differ significantly from pp32 in sequence. The isolated transcripts were found to stimulate transformation. **Transformation assay.** Rat embryo fibroblasts were transfected with the indicated constructs as described (Chen, et al.) and transformed foci enumerated. For each experiment, approximately 1 x 10⁶ cells were plated per T75 flask and incubated for 2 to 3 d prior to transfection to achieve approximately 40% confluency. For each flask of primary rat embryo fibroblasts, the plasmids indicated in each experiment were added in the following amounts: pEJ-ras, 5 µg; and pMLV-c-myc, pCMV32, pCMVneo, or variant pp32 constructs in pCR3.1 (Invitrogen), 10 µg. Plasmids were prepared in two volumes Lipofectin (2 µl lipofectin per µg DNA) then gently mixed by inversion in 1.5 ml OPTIMEM in sterile 15 ml polystyrene tubes and allowed to incubate at room temperature for > 15 min. For experiments with more than one flask, mixtures of all reagents were increased in proportion to the numbers of flasks required for each transfection. Cells were washed once with OPTIMEM (Gibco-BRL), and then fed with 6 ml of OPTIMEM and 1.5 ml of the DNA/Lipofectin mix. After overnight incubation, the cells were grown in standard media and refed with fresh media twice weekly. Foci were counted fourteen days post-transfection. Figure 10 summarizes four separate experiments. Each data point represents the results from an individual flask expressed as the percent foci obtained in the contemporaneous control of ras+myc+vector.

Figure 10 shows that expressed variant transcripts from prostate cancer cell lines and from human prostatic adenocarcinoma generally produce increased numbers of transformed foci when co-transfected with ras and myc, as compared to the number of foci obtained when ras and myc are transfected with blank vector. Variant pp32 transcripts from DU-145 (D3), and from three prostate cancers (FT 1.7, FT 2.2, and FT3.18) yield increased numbers of transformed foci over those produced by ras and myc alone with blank vector. This stands in marked contrast to normal pp32. which consistently suppresses transformation. These activities are also summarized in Table I.

### Example 12. Effect of pp32 Variants on Tumorigenesis In Vivo

Experiments testing the effect of transfection ofNIH3T3 cells on tumorigenesis *in vivo* are consistent with *in vitro* results in rat embryo fibroblasts. NIH3T3 cells were stably transfected by lipofection with the pp32 species indicated in Table 6A carried in the pCR3.1-Uni CMV-driven mammalian expression vector (Invitrogen). The G418-resistant clones employed in these experiments were all shown by genomic PCR to carry the indicated pp32 species. For analysis of tumorigenesis, 5 x 10⁶ cells in 100 microliters of unsupplemented Dulbecco's modified Eagle's medium without phenol red were injected into the flanks of female athymic nude mice on an outbred background of greater than six weeks in age (Harlan). For logistical reasons, inoculations of the various groups were staggered over a seven day period. Each group of mice was euthanized precisely seven weeks after inoculation. Where a mouse had a tumor, the tumor was dissected, measured, and weighed, and Table 6A reports the average weight of tumors in mice injected with cells carrying various vectors. One tumor from each group was examined histologically. All tumors were fibrosarcomas without noteworthy inflammation present. Data obtained with NIH3T3 cells indicate that NIH3T3 cells stably transfected with the variant pp32 species P3, P8, FT1.7, FT2.2, and FT2.4 form tumors when inoculated into nude mice. In contrast, NIH3T3 cells stably transfected to express human pp32 fail to form tumors in vivo even when further transfected with ras. Lines of NIH3T3 cells were also established that were stably transfected with expression constructs encoding pp32 or pp32. antisense. Basal expression of pp32 is essential for maintenance of contact inhibition and serum-dependent cell growth; antisense ablation of endogenous pp32 synthesis permitted cells to grow normally following serum withdrawal. Constitutive over-expression of pp32 potently suppressed ras-mediated transformation of NIH3T3 cells *in vitro* and tumorigenesis *in vivo.* In contrast, antisense ablation of endogenous pp32 dramatically increased the number and size of ras-transformed foci; *in vivo*, tumors obtained from ras-transformed antisense pp32 cells were approximately 50-fold greater in mass than tumors obtained from ras-transformed control cells.

For purposes of clarity of understanding, the foregoing invention has been described in some detail by way of illustration and example in conjunction with specific embodiments, although other aspects, advantages and modifications will be apparent to those skilled in the an to which the invention pertains. The foregoing description and examples are intended to illustrate, but not limit the scope of the invention. Modifications of the above-described modes for carrying out the invention that are apparent to persons of skill in medicine, immunology, hybridoma technology, pharmacology, pathology, and/or related fields are intended to be within the scope of the invention, which is limited only by the appended claims.

**TABLE 1**

| Position | Strand | Consensus Sequence | Factor |
|---|---|---|---|
| 4 | C | TTTCCT | PEA3 |
| 21 | N | CAAGGTCA | ELP |
| 23 | N | AGGTCA | PPAR |
| 32 | C | CCCTAA | TBF1 |
| 41 | N | CTTGGC | NF-1 (-like proteins) |
| 81 | N | TAAACAC | Pit-1 |
| 82 | N | AAACACA | HiNF-A |
| 113 | C | CTTCCC | c-Ets-2 |
| 118 | N | CTATCA | GATA-1 |
| 122 | N | CAGTTG | c-Myc |
| 212 | C | AATAAATA | TFIID |
| 213 | N | ATAAATA | ETF |
| 247 | N | TATCTA | NIT2 |
| 261 | C | AAGGAA | c-Ets-2 |
| 262 | N | AGGAAA | PEA3 |
| 283 | C | TTTTTCTTTTTC | Hb |
| 320 | C | TTATAT | GAL4 |
| 333 | N | TAAAAAA | TBP |
| 349 | N | TTATACATT | TBP |
| 363 | C | AAGGAA | c-Ets-2 |
| 394 | C | TTTCTATA | TBP |
| 398 | N | TATAAA | TBP |
| 398 | N | TATAAA | TFIID |
| 411 | C | CTGAATT | Pit-1 |
| 420 | N | TGTCCC | GR |
| 423 | C | CCCTAA | TBF1 |
| 434 | N | TTCCTT | c-Ets-2 |
| 447 | C | CTTCCC | c-Ets-2 |
| 514 | N | TTATCTCT | GATA-1 |
| 514 | C | TTATCT | GATA-2 |
| 515 | N | TATCTC | NIT2 |
| 537 | N | TATGCA | EFII |
| 553 | N | AAGTCA | GCN4 |
| 608 | N | TGACTA | GCN4 |
| 628 | N | CCTCCCAAC | LyF-1 |
| 640 | N | TGTCCT | GR |
| 648 | N | TTAAAATTCA | 1-Oct |
| 648 | N | TTAAAATTCA | 4-Oct |
| 649 | N | TAAAAT | F2F |
| 649 | N | TAAAAT | Pit-1 |
| 661 | N | TAAAAAA | TBP |
| 673 | N | CTTGGC | NF-1 (-like proteins) |
| 725 | N | AGGCGG | Sp1 |
| 729 | N | GGGCGG | ETF |
| 729 | N | GGGCGG | Sp1 |
| 729 | C | GGGCGG | Sp1 |
| 741 | N | AGGTCA | PPAR |
| 793 | N | TATAAATA | B factor |
| 793 | N | TATAAA | TBP |
| 793 | N | TATAAATA | TFIID |
| 793 | N | TATAAAT | TMF |
| 794 | N | ATAAATA | ETF |
| 809 | N | TTATCT | GATA-1 |
| 809 | C | TTATCT | GATA-2 |
| 815 | N | GGGTGTGG | TEF-2 |
| 826 | C | CACATG | muEBP-C2 |
| 826 | C | CACATG | TFE3-S |
| 826 | N | CACATG | USF |
| 978 | N | ATGTAAAACA | 1-Oct |
| 978 | N | ATGTAAAACA | 2-Oct |
| 978 | N | ATGTAAAACA | NF-IL-2A |
| 1000 | N | ATGTCAGA | CSBP-1 |
| 1006 | N | GATTTC | H4TF-1 |
| 1034 | C | TTTTCAT | Pit-1 |
| 1047 | N | AAGATAAAACC | RVF |
| 1048 | C | AGATAA | GATA-1 |
| 1048 | N | AGATAA | GATA-2 |
| 1049 | N | GATAAA | TFIID |
| 1083 | C | GCCAAG | NF-1 (-like proteins) |
| 1124 | N | CGCCAT | UCRF-L |
| 1163 | C | GACCTG | TGT3 |
| 1307 | N | CAGTCA | GCN4 |
| 1347 | C | TGCATA | EFII |
| 1373 | C | AGAACA | AR |
| 1373 | N | AGAACAT | GR |
| 1373 | N | AGAACA | GR |
| 1373 | C | AGAACA | GR |
| 1373 | N | AGAACA | PR |
| 1373 | C | AGAACA | PR |
| 1373 | N | AGAACA | PR A |
| 1373 | C | AGAACA | PR A |
| 1393 | C | TCACTT | IRF-1 |
| 1393 | C | TCACTT | IRF-2 |
| 1395 | C | ACTTCCT | E1A-F |
| 1423 | N | TTATCT | GATA-1 |
| 1423 | C | TTATCT | GATA-2 |
| 1424 | N | TATCTA | NIT2 |
| 1452 | N | TTACTC | GCN4 |
| 1471 | N | TGGGTCA | c-Fos |
| 1471 | N | TGGGTCA | c-Jun |
| 1471 | N | TGGGTCA | ER |
| 1496 | N | TCTCTTA | c-Myc |
| 1511 | N | TATAAA | TBP |
| 1511 | N | TATAAA | TFIID |
| 1549 | C | TTTGAA | TFIID |
| 1568 | C | AATGTATAA | TBP |
| 1581 | C | TTTGAA | TFIID |
| 1590 | C | AGATAA | GATA-1 |
| 1590 | N | AGATAA | GATA-2 |
| 1591 | C | GATAATTG | Dfd |
| 1657 | C | AGGACA | GR |
| 1670 | C | ATTTTA | F2F |
| 1670 | C | ATTTTA | Pit-1 |
| 1671 | C | TTTTATA | B factor |
| 1671 | C | TTTTATA | Dr1 |
| 1671 | C | TTTTATA | En |
| 1671 | C | TTTTATA | TBP |
| 1671 | C | TTTTATA | TBP-1 |
| 1671 | C | TTTTATA | TFIIA |
| 1671 | C | TTTTATA | TFIIB |
| 1671 | C | TTTTATA | TFIID |
| 1671 | C | TTTTATA | TFIIE |
| 1671 | C | TTTTATA | TFIIF |
| 1671 | C | TTTTATA | TRF |
| 1672 | C | TTTATA | TBP |
| 1694 | C | AATAAATA | TFIID |
| 1695 | N | ATAAATA | ETF |
| 1733 | N | AGGAAA | PEA3 |
| 1749 | C | TTATAT | GAL4 |
| 1783 | N | TAACTCA | AP-1 |
| 1829 | C | TAGATA | NIT2 |
| 1857 | N | CGCCAT | UCRF-L |
| 1875 | N | TTCTGGGAA | IL-6 RE-BP |
| 1895 | N | TGACTA | GCN4 |
| 1899 | N | TATTTAA | TBP |
| 1942 | N | ATATAA | GAL4 |
| 1985 | C | TTTATA | TBP |
| 1985 | C | TTTATA | TFIID |
| 2010 | C | AATAAATA | TFIID |
| 2011 | N | ATAAATA | ETF |
| 2058 | C | TGCATA | EFII |
| 2095 | N | CAGTCA | GCN4 |
| 2146 | C | AAGGAA | c-Ets-2 |
| 2147 | N | AGGAAA | PEA3 |
| 2190 | N | AGGAAA | PEA3 |
| 2220 | C | GGCACA | GR |
| 2252 | N | CCAATAG | gammaCAAT |
| 2286 | N | TGTGCC | GR |
| 2292 | N | ATGGGA | PTF1-beta |
| 2314 | N | TATGCA | EFII |
| 2328 | C | GGCACA | GR |
| 2350 | C | ATGATAAG | GATA-1 |
| 2351 | N | TGATAAG | GATA-1 |
| 2363 | N | GGGAAG | c-Ets-2 |
| 2367 | N | AGCCACT | CP2 |
| 2369 | C | CCACTGGGGA | AP-2 |
| 2404 | N | TAAAAT | F2F |
| 2404 | N | TAAAAT | F2F |
| 2404 | N | TAAAAT | Pit-1 |
| 2409 | N | TTGTCATA | 77+82K protein |
| 2409 | N | TTGTCATA | VETF |
| 2415 | N | TATCTA | NIT2 |
| 2451 | C | TTTATC | TFIID |
| 2452 | N | TTATCT | GATA-1 |
| 2452 | C | TTATCT | GATA-2 |
| 2486 | N | CTCTCTCTCTCTC | GAGA factor |
| 2644 | N | AGGCGG | Spl |
| 2658 | N | ACAGCTG | GT-IIBalpha |
| 2658 | N | ACAGCTG | GT-IIBbeta |
| 2709 | C | GGCCAGGC | AP-2 |
| 2723 | N | TGAACT | GR |
| 2731 | C | TGACCT | PPAR |
| 2731 | C | TGACCTCA | URTF |
| 2753 | N | CTTGGC | NF-1 (-like proteins) |
| 2818 | C | TGATGTCA | AP-1 |
| 2818 | C | TGATGTCA | c-Fos |
| 2818 | C | TGATGTCA | c-Jun |
| 2818 | C | TGATGTCA | CREB |
| 2845 | N | GGGAAG | c-Ets-2 |
| 2858 | N | AGATAG | GATA-1 |
| 2858 | C | AGATAG | GATA-1 |
| 2864 | C | AGTTCA | GR |
| 2899 | N | ATATAA | GAL4 |
| 2900 | N | TATAAAA | B factor |
| 2900 | N | TATAAAA | Dr1 |
| 2900 | N | TATAAAA | En |
| 2900 | N | TATAAAA | TBP |
| 2900 | N | TATAAA | TBP |
| 2900 | N | TATAAAA | TBP-1 |
| 2900 | N | TATAAAA | TFIIA |
| 2900 | N | TATAAAA | TFIIB |
| 2900 | N | TATAAAA | TFIID |
| 2900 | N | TATAAAA | TFIIE |
| 2900 | N | TATAAAA | TFIIF |
| 2900 | N | TATAAAA | TRF |
| 2921 | C | TTTGAA | TFIID |
| 2924 | C | GAAATC | H4TF-1 |
| 2930 | C | CATTAG | Isl-1 |
| 2948 | C | TGTACA | GR |
| 2948 | C | TGTACA | PR |
| 2948 | C | TGTACA | PR A |
| 2964 | C | ATTTGAGAA | VITF |
| 3030 | N | AGTGTTCT | GR |
| 3032 | N | TGTTCT | AR |
| 3032 | N | TGTTCT | GR |
| 3032 | C | TGTTCT | GR |
| 3032 | N | TGTTCT | PR |
| 3032 | C | TGTTCT | PR |
| 3032 | N | TGTTCT | PR A |
| 3032 C | | TGTTCT | PR A |
| 3104 | C | GGATTATT | Tll |
| 3106 | C | ATTATTAA | AFP 1 |
| 3111 | N | TAAAAT | F2F |
| 3111 | N | TAAAAT | Pit-1 |
| 3125 | C | ATTITA | F2F |
| 3125 | C | ATTA | Pit-1 |
| 3142 | N | TGTGAT | GR |
| 3169 | N | GTTTTATT | HOXD10 |
| 3169 | N | GTTTTATT | HOXD8 |
| 3169 | N | GTTTTATT | HOXD9 |
| 3175 | C | TTTGAA | TFIID |
| 3185 | N | TTGCTCA | Zta |
| 3206 | N | GATTTC | H4TF-1 |
| 3212 | N | AGGAAA | PEA3 |
| 3238 | C | ATTTTA | F2F |
| 3238 | C | ATTTTA | Pit-1 |
| 3256 | C | TTTGAA | TFIID |
| 3266 | N | TTGCTCA | Zta |
| 3320 | C | ATTTTA | F2F |
| 3320 | C | ATTTTA | Pit-1 |
| 3358 | N | ATGGGA | PTF 1-beta |
| 3360 | C | GGGACA | GR |
| 3440 | C | CACTCA | GCN4 |
| 3460 | C | TTTCCT | PEA3 |
| 3483 | N | GACACA | GR |
| 3491 | C | TTTCCT | PEA3 |
| 3495 | N | CTAATG | Isl-1 |
| 3523 | C | AGAACA | AR |
| 3523 | N | AGAACA | GR |
| 3523 | C | AGAACACT | GR |
| 3523 | C | AGAACA | GR |
| 3523 | N | AGAACA | PR |
| 3523 | C | AGAACA | PR |
| 3523 | N | AGAACA | PR A |
| 3523 | C | AGAACA | PR A |
| 3538 | C | TTTATC | TFIID |
| 3539 | N | TTATCT | GATA-1 |
| 3539 | C | TTATCT | GATA-2 |
| 3551 | N | TGAGTG | GCN4 |
| 3569 | C | TCCCAT | PTF1-beta |
| 3594 | N | TTAGGG | TBF1 |
| 3653 | C | CCTGCTGAA | LyF-1 |
| 3668 | N | CTCATGA | 1-Oct |
| 3668 | N | CTCATGA | 2-Oct |
| 3668 | N | CTCATGA | Oct-2B |
| 3668 | N | CTCATGA | Oct-2B |
| 3668 | N | CTCATGA | Oct-2C |
| 3679 | C | TGTGTAA | Zta |
| 3685 | C | AGAACT | GR |
| 3712 | C | TTTCCT | PEA3 |
| 3713 | N | TTCCTT | c-Ets-2 |
| 3717 | N | TTGCTCA | Zta |
| 3727 | C | AAAACATAAAT | ssARS-T |
| 3749 | N | TAAAAAA | TBP |
| 3784 | C | CACTCA | GCN4 |
| 3791 | C | ATTTTA | F2F |
| 3791 | C | ATTTTA | Pit-1 |
| 3815 | N | TATCTA | NIT2 |
| 3829 | C | TAGATA | NIT2 |
| 3859 | C | AGAACA | AR |
| 3859 | N | AGAACAG | GR |
| 3859 | N | AGAACA | GR |
| 3859 | C | AGAACA | GR |
| 3859 | N | AGAACA | PR |
| 3859 | C | AGAACA | PR |
| 3859 | N | AGAACA | PR A |
| 3859 | C | AGAACA | PR A |
| 3860 | N | GAACAG | LVa |
| 3877 | C | ATCACA | GR |
| 3886 | N | TGAGTCA | AP-1 |
| 3886 | C | TGAGTCA | AP-1 |
| 3886 | C | TGAGTCA | c-Fos |
| 3886 | C | TGAGTCA | c-Jun |
| 3886 | C | TGAGTCA | FraI |
| 3886 | C | TGAGTCA | NF-E2 |
| 3887 | C | GAGTCA | GCN4 |
| 3931 | N | AGATAG | GATA-1 |
| 3931 | C | AGATAG | GATA-1 |
| 3960 | N | TTGGCA | NF-1/L |
| 3965 | C | ATTTTA | F2F |
| 3965 | C | ATTTTA | Pit-1 |
| 4026 | N | TATTTAA | TBP |
| 4037 | N | TGTGAT | GR |
| 4040 | N | GATGCAT | Pit.1 |
| 4042 | C | TGCATA | EFII |
| 4079 | N | TTCAAAG | SRY |
| 4079 | N | TTCAAAG | TCF-1A |
| 4079 | N | TTCAAA | TFIID |
| 4097 | N | CAGGTC | TGT3 |
| 4140 | N | TGATTCA | AP-1 |
| 4140 | C | TGATTCA | AP-1 |
| 4140 | N | TGATTC | GCN4 |
| 4164 | N | GGGAGTG | p300 |
| 4205 | C | AGATAA | GATA-1 |
| 4205 | N | AGATAA | GATA-2 |
| 4219 | C | TTAGTCAC | AP-1 |
| 4219 | C | TTAGTCA | AP-1 |
| 4219 | C | TTAGTCAC | c-Fos |
| 4219 | C | TTAGTCAC | c-Jun |
| 4219 | C | TTAGTCA | c-Jun |
| 4219 | C | TTAGTCA | Jun-D |
| 4220 | C | TAGTCA | GCN4 |
| 4271 | N | TGTTCT | AR |
| 4271 | N | TGTTCT | GR |
| 4271 | C | TGTTCT | GR |
| 4271 | N | TGTTCT | PR |
| 4271 | C | TGTTCT | PR |
| 4271 | N | TGTTCT | PR A |
| 4271 | C | TGTTCT | PR A |
| 4280 | C | TGACCCA | c-Fos |
| 4280 | C | TGACCCA | c-Jun |
| 4280 | C | TGACCCA | ER |
| 4292 | C | CTTATCAG | GATA-1 |
| 4292 | C | CTTATCA | GATA-1 |
| 4361 | N | TTCAAAG | SRY |
| 4361 | N | TTCAAAG | TCF-1A |
| 4361 | N | TTCAAA | TFIID |

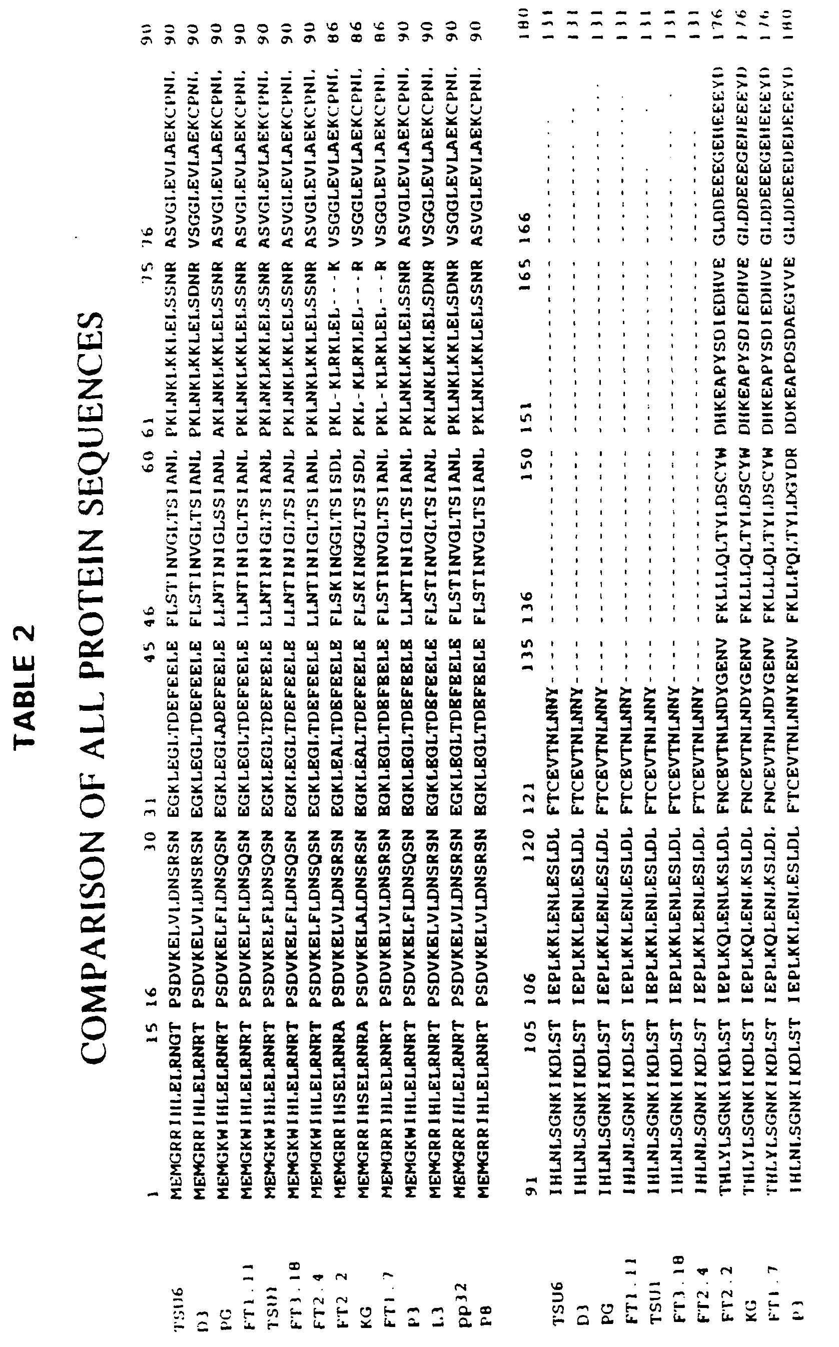

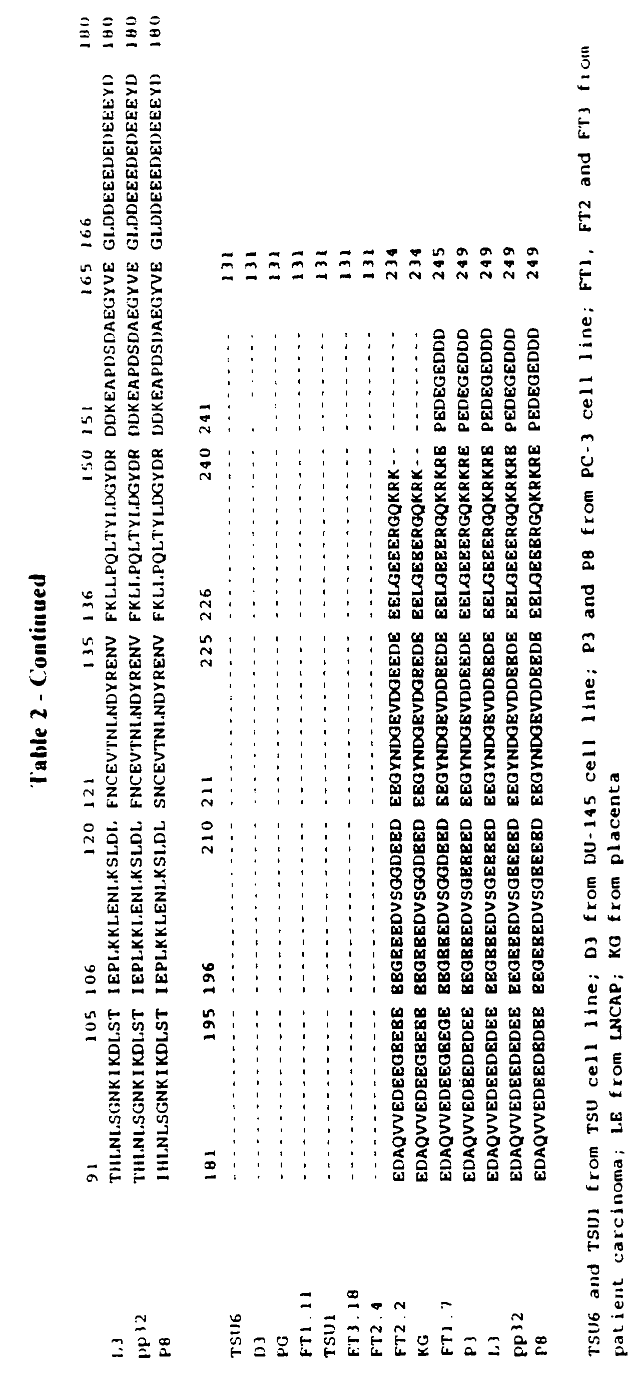

**TABLE 3**

| | Comparison to pp32 Sequences | | |
|---|---|---|---|
| | % Identity | | % Similarity |
| CLONE | cDNA | Protein | Protein |
| D3, DU-145 cells | 95 | 90 | 95 |
| P3, PC-3 | 86 | 94 | 96 |
| P8, PC-3 | 98 | 97 | 97 |
| | | | |
| FT1.11 | 97 | 86 | 92 |
| FT1.7 | 95 | 95 | 95 |
| FT2.2 | 94 | 85 | 88 |
| FT2.4 | 99 | 86 | 92 |
| FT3.18 | 99 | 90 | 94 |

**TABLE 1A**

| Sequence | Sequence Group | Nucleotide identity with pp32 | Gaps | Protein Identity with pp32 | Effect on Oncogene-Mediated Transformation | Comment |
|---|---|---|---|---|---|---|
| FT 1. 3 | A | 99. 8 | | 100 | Not Tested | Identical to pp32 |
| D1 | A | 99. 9 | | 100 | Not tested | identical to pp32 with 2 silent nt changes |
| L3 | A | 99. 9 | | 100 | Not Tested | |
| D3 | U | 95. 8 | 0 | 96. 9 | Generally Stimulatory | Encodes truncated variant pp32 |
| D5 | U | 99. 6 | 0 | 99. 6 | Not Tested | |
| FT 1. 2 | U | 92. 9 | 1 | | Not tested | No ORF |
| P3 | U | 96. 5 | 1 | 94. 4 | Slightly Stimulatory | |
| P8 | U | 98. 7 | 0 | 98. 0 | Variable | |
| FT 1. 11 | B | 92. 4 | 2 | 89. 3 | Not Tested | All sequences identical: appears to be product of pp32r2 |
| FT 2. 4 | B | 92. 4 | 2 | 89. 3 | Variable | |
| TI | B | 92. 4 | 2 | 89. 3 | | |
| T6 | U | 94. 2 | 1 | 93. 9 | Not Tested | Encodes truncated variant pp32 |
| FT 3 18 | U | 94. 7 | 2 | 89. 3 | Stimulatory | Encodes truncated variant pp32 |
| FT 2. 2 | C | 94. 4 | 3 | 87.6 | Stimulatory | Sequences differ by 1 nt. appears to be product of pp32rl |
| FT 3. 3 | C | 94. 4 | 3 | 87. 6 | not tested | |
| FT 1. 7 | U | 95. 9 | 2 | 91. 4 | Stimulatory | |

**Table 2A**

| Protein | Genbank Accession | Length | Human pp32 | Human pp32r1 | Human pp32r2 | Human April | Murine pp32 |
|---|---|---|---|---|---|---|---|
| Human p32 | HSU73477 | 249 | 100% | 88% Identity 2 gaps; Z=77 | 84% Identity 0 gaps; Z=73 | 71% Identity 3 gaps; Z=58 | 89% Identity 1 gap; Z= 87 |
| Human pp32r1 | AF008216 | 234 | | 100% Identity | 785 Identity 2 gaps; Z=65 | 61% Identity 5 gaps; Z= 15 | 90% identity 3 gaps; Z=64 |
| Human pp32r2 | HSU71084 | 131 | | | 100% Identify | 61% Identify 3 gaps; Z=52 | 77% Identity I gap; Z=80 |
| Human April | Y07969 | 249 | | | | 100% | 71% Identity 4 gaps; Z=68 |
| Murine pp32 | U734778 | 247 | | | | | 100% Identity |

| | | | | | | | |
|---|---|---|---|---|---|---|---|
| Percent amino acid identity of pp32 and related proteins. Sequences were aligned using the GAP program (7). The number of gaps in the alignment is indicated as well as the Z score, a statistical measure of protein relatedness derived from 50 comparisons of randomized protein sequences | | | | | | | |

**Table 3A. pp32 Homologs**

| |
|---|
| human pp32 (Genbank Locus HSU73477) |
| murine pp32 (Genbank Locus MMU73478) |
| human cerebellar leucine rich acidic nuclear protein (LANP) (Genbank Locus AF025684) |
| murine LANP (Genbank Locus AF022957) |
| murine RFC1 (Genbank Locus MUSMRFC, Accession NO. L23755) |
| I1PP2a or human potent heat-stable protein phospatase 2a inhibitor (Genbank Locus HSU60823) |
| SSP29 (Genbank Locus HSU70439) |
| HLA-DR associated protein I (Genbank Locus HSPPHAPI, Accession No. X75090) |
| PHAPI2a (EMBL Locus HSPHAP12A, Genbank Accession No. Y07569) |
| PHAPI2b (EMBL Locus HSPHAPI2B, Genbank Accession No. Y07570) |
| April (EMBL Locus HSAPRIL) |

**Table 6A. Tumorigenicity in Nude Mice of NIH3T3 Cells Transfected with pp32 and pp32 Variants**

| pp32 Species | Clone | Tumors/ | Average Tumor Weight |
|---|---|---|---|
| FT1.7 | 1 | 3/3 | 14.9 ± 2.1 |
| | 2' | 3/3 | 13.3 ± 3.7 |
| FT2.2 | 1 | 3/3 | 10.5 ± 2.8 |
| | 2 | 3/3 | 3.8 ± 2.1 |
| FT2.4 | 1 | 3/3⁶ | 1.3 ± 0.9 |
| | 2 | 3/3 | 13.8 ± 3.3 |
| D3 | 5² | 0/3 | |
| | 6² | 0/3 | |
| P3 | 11 | 3/3 | 5.7 ± 0.5 |
| | 14³ | 3/3 | 2.1 ± 1.2 |
| P8 | 1⁴ | 3/3 | 6.4 ± 5.3 |
| | 2 | 3/3 | 11.3 ± 3.9 |
| | 4⁵ | 3/3 | 10.1 ± 4.8 |
| L3 (pp32) | 5⁵ | 0/3 | |
| | 6⁴ | 0/3 | |
| Vector Control | 2³ | 0/3 | |
| | 3¹ | 0/3 | |

| | | | |
|---|---|---|---|
| ¹FT1.7, clone 2 and Vector Control, clone 3 were tested on contralateral sides of a single group of animals. | | | |
| ²D3 clone 5 was tested on the contralateral sides of a group of animals simultaneously injected with NIH3T3 cells transfected with a clone of pp32r1 (data not shown). D3 clone 6 was tested on the contralateral sides of a group of animals simultaneously injected with a second clone of NIH3T3 cells transfected with pp32r1 (data not show). | | | |
| ³P3, clone 14 and Vector Control, clone 2 were tested on contralateral sides of a single group of animals. | | | |
| ⁴P8, clone 1 and pp32, clone 6 were tested on contralateral sides of a single group of animals. | | | |
| ⁵P8, clone 4 and pp32, clone 5 were tested on contralateral sides of a single group of animals. | | | |
| ⁶One tumor in this group, weighing 0.5 gm, was detected only upon post mortem dissection. | | | |

## Claims

1. An isolated DNA molecule comprising a sequence of 18 contiguous nucleotides selected from the sequence consisting of base pairs 4894-4942 of the sequence in Figure 2, or a sequence complementary thereto.

2. An isolated DNA molecule comprising the DNA molecule of claim 1 which encodes the amino acids from residue 146-163 of the amino acid sequence of pp32r1, encoded by the sequence of figure 2.

3. An isolated nucleic acid probe of at least 15 nucleotides which specifically hybridizes on Northern blot with nucleic acid encoding the amino acids from residue 146-163 of the amino acid sequence of pp32r1 encoded by the sequence of figure 2.

4. The isolated DNA molecule of any one of the preceding claims, wherein said isolated DNA molecule is producing using recombinant methods.

5. The isolated DNA molecule according to claim 4, wherein said isolated DNA molecule is contained in an expression vector.

6. A recombinant cell containing the expression vector of claim 5.

7. The isolated DNA molecule of claim 1, wherein said DNA molecule is operatively linked to a promoter in antisense orientation.

8. A method of producing a pp32r1 encoded by the sequence of figure 2 nucleic acid sequence encoding at least residues 146-163 of the pp32r1 protein sequence, comprising amplifying a suitable source of human nucleic acid using a pair of nucleic acid primers each comprising at least 10 nucleotides wherein at least one of the primers binds specifically to the pp32r1 sequence such that the amplified nucleic acid comprises the DNA molecule of claim 1.

9. A diagnostic method for predicting malignant potential of neuroendocrine, neural, mesenchymal, lymphoid, epithelial or germ cell derived tumours in a sample of human neuroendocrine, neural, mesenchymal, lymphoid, epithelial or germ cell derived tissue comprising determining, in the sample, levels or intracellular sites of expression of a gene product expressed from a gene sequence which encodes residues 146-163 of the sequence of pp32r1 encoded by the sequence of figure 2.

10. The method of claim 9, wherein the gene product is mRNA.

11. The method of claim 10, wherein the mRNA is extracted from the sample and quantitated.

12. The method of claim 10, wherein the level of mRNA is determined by *in situ* hybridization to a section of the tissue sample.

13. The method of claim 10, wherein the mRNA is quantitated by polymerase chain reaction.

14. The method according to claim 9, wherein the gene product is protein.

15. The method according to claim 14, wherein the method further comprises reacting the sample with an antibody that specifically binds to a polypeptide consisting of the sequence of pp32r1 encoded by the sequence of figure 2, but does not specifically bind to a polypeptide consisting of the sequence of pp32 or pp32r2 encoded by the sequence of figure 5 or an antibody that specifically binds to a polypeptide consisting of the sequence of pp32r2, but does not specifically bind to a polypeptide consisting of the sequence of pp32 or pp32r1.

16. The method according to claim 9, wherein the tissue is a carcinoma.

17. The method according to claim 9, wherein the tissue is a carcinoma or sarcoma of a tissue selected from the group consisting of epithelial, lymphoid, hematopoietic, mesenchymal, central nervous system and peripheral nervous system tissues.

18. The method according to claim 17, wherein the tissue is selected from the group consisting of colon carcinoma, prostate carcinoma and non-Hodgkin's lymphoma.

19. An antibody that specifically binds to a polypeptide consisting of the sequence of pp32r1 encoded by the sequence of figure 2 or pp32r2 encoded by the sequence of figure 5, but does not specifically bind to a polypeptide consisting of the sequence of pp32.

20. The antibody of claim 19, wherein the antibody is a monoclonal antibody.

## Patentansprüche

1. Isoliertes DNA-Molekül, umfassend eine Sequenz aus 18 aufeinanderfolgenden Nukleotiden, die ausgewählt sind aus der aus den Basenpaaren 4894-4942 bestehenden Sequenz der Sequenz in Figur 2, oder eine dazu komplementäre Sequenz.

2. Isoliertes DNA-Molekül, umfassend das DNA-Molekül gemäß Anspruch 1, das für die Aminosäuren von Rest 146 bis Rest 163 der von der Sequenz in Figur 2 codierten pp32r1-Aminosäuresequenz codiert.

3. Isolierte Nukleinsäuresonde aus wenigstens 15 Nukleotiden, die bei einem Northern Blot spezifisch mit Nukleinsäure hybridisiert, die für die Aminosäuren von Rest 146 bis Rest 163 der von der Sequenz in Figur 2 codierten pp32r1-Aminosäuresequenz codiert.

4. Isoliertes DNA-Molekül nach irgendeinem der vorhergehenden Ansprüche, wobei das isolierte DNA-Molekül mit rekombinanten Verfahren hergestellt wird.

5. Isoliertes DNA-Molekül nach Anspruch 4, wobei das isolierte DNA-Molekül in einem Expressionsvektor enthalten ist.

6. Rekombinante Zelle, enthaltend den Expressionsvektor gemäß Anspruch 5.

7. Isoliertes DNA-Molekül nach Anspruch 1, wobei das DNA-Molekül in *antisense-*Orientierung funktionsfähig mit einem Promotor verknüpft ist.

8. Verfahren zur Herstellung einer Nukleinsäuresequenz für pp32r1, das von der Sequenz in Figur 2 codiert wird, die wenigstens für die Reste 146-163 der pp32r1-Proteinsequenz codiert, umfassend das Amplifizieren einer geeigneten Quelle humaner Nukleinsäure mittels eines Nukleinsäureprimerpaares, wobei jeder Primer wenigstens 10 Nukleotide umfasst und wenigstens einer der Primer spezifisch so an die pp32r1-Sequenz bindet, dass die amplifizierte Nukleinsäure das DNA-Molekül gemäß Anspruch 1 umfasst.

9. Diagnostisches Verfahren zur Vorhersage des malignen Potentials von neuroendokrinen, neuralen, mesenchymalen, lymphoiden, epithelialen oder von Keimzellen abstammenden Tumoren in einer Probe von humanem neuroendokrinem, neuralem, mesenchymalem, lymphoidem, epithelialem oder von Keimzellen stammendem Gewebe, umfassend das Bestimmen, in der Probe, von Expressionsmengen oder intrazellulären Expressionsorten eines Genprodukts, das aus einer Gensequenz exprimiert wird, die für die Reste 146-163 der von der Sequenz in Figur 2 codierten pp32r1-Sequenz codiert.

10. Verfahren nach Anspruch 9, wobei das Genprodukt mRNA ist.

11. Verfahren nach Anspruch 10, wobei die mRNA aus der Probe extrahiert und quantifiziert wird.

12. Verfahren nach Anspruch 10, wobei die Menge an mRNA durch *in situ-*Hybridisierung an einen Schnitt der Gewebeprobe bestimmt wird.

13. Verfahren nach Anspruch 10, wobei die mRNA über Polymerasekettenreaktion quantifiziert wird.

14. Verfahren nach Anspruch 9, wobei das Genprodukt Protein ist.

15. Verfahren nach Anspruch 14, wobei das Verfahren ferner das Reagierenlassen der Probe mit einem Antikörper umfasst, der spezifisch an ein Polypeptid bindet, das aus der von der Sequenz in Figur 2 codierten pp32r1-Sequenz besteht, der aber nicht spezifisch an ein Polypeptid bindet, das aus der von der Sequenz in Figur 5 codierten pp32- oder pp32r2-Sequenz besteht, oder mit einem Antikörper, der spezifisch an ein Polypeptid bindet, das aus der pp32r2-Sequenz besteht, der aber nicht spezifisch an ein Polypeptid bindet, das aus der pp32- oder der pp32r1-Sequenz besteht.

16. Verfahren nach Anspruch 9, wobei das Gewebe ein Karzinom ist.

17. Verfahren nach Anspruch 9, wobei das Gewebe ein Karzinom oder Sarkom eines Gewebes ist, das ausgewählt ist aus der Gruppe bestehend aus epithelialem, lymphoidem, hämatopoietischem und mesenchymalem Gewebe und Gewebe des zentralen und des peripheren Nervensystems.

18. Verfahren nach Anspruch 17, wobei das Gewebe ausgewählt ist aus der Gruppe bestehend aus Kolonkarzinom, Prostatakarzinom und Non-Hodgkin-Lymphom.

19. Antikörper, der spezifisch an ein Polypeptid bindet, das aus der von der Sequenz in Figur 2 codierten pp32r1-Sequenz oder aus der von der Sequenz in Figur 5 codierten pp32r2-Sequenz besteht, aber nicht spezifisch an ein Polypeptid bindet, das aus der pp32-Sequenz besteht.

20. Antikörper nach Anspruch 19, wobei der Antikörper ein monoklonaler Antikörper ist.

## Revendications

1. Molécule d'ADN isolée comprenant une séquence de 18 nucléotides contigus choisie parmi la séquence consistant en les paires de bases 4894-4942 de la séquence de la figure 2 ou une séquence complémentaire de celle-ci.

2. Molécule d'ADN isolée comprenant là molécule d'ADN selon la revendication 1 qui code pour les acides aminés des résidus 146-163 de la séquence d'acides aminés de la pp32r1, codée par la séquence de la figure 2.

3. Sonde d'acide nucléique isolée d'au moins 15 nucléotides qui s'hybride spécifiquement lors d'un transfert de type Northern avec l'acide nucléique codant les acides aminés des résidus 146-163 de la séquence d'acides aminés de la pp32r1, codée par la séquence de la figure 2.

4. Molécule d'ADN isolée selon l'une quelconque des revendications précédentes, dans laquelle ladite molécule d'ADN isolée est produite en utilisant des procédés recombinants.

5. Molécule d'ADN isolée selon la revendication 4, dans laquelle ladite molécule d'ADN isolée est contenue dans un vecteur d'expression.

6. Cellule recombinante contenant le vecteur d'expression selon la revendication 5.

7. Molécule d'ADN isolée selon la revendication 1, dans laquelle ladite molécule d'ADN isolée est fonctionnellement liée à un promoteur dans une orientation anti-sens.

8. Procédé de production d'une séquence d'acide nucléique de la pp32r1, codée par la séquence de la figure 2, codant au moins les résidus 146-163 de la séquence de la protéine pp32r1, comprenant l'amplification d'une source appropriée d'acide nucléique humain en utilisant une paire d'amorces d'acide nucléique comprenant chacune au moins 10 nucléotides, dans lequel au moins une des amorces se lie spécifiquement à la séquence de la pp32r1 de sorte que l'acide nucléique amplifié comprend la molécule d'ADN selon la revendication 1.

9. Procédé de diagnostic destiné à prédire le potentiel de malignité de tumeurs dérivées de cellules neuroendocriniennes, neurales, mésenchymateuses, lymphoïdes, épithéliales ou germinales dans un échantillon de tissu dérivé de cellules neuroendocriniennes, neurales, mésenchymateuses, lymphoïdes, épithéliales ou germinales comprenant la détermination dans l'échantillon des niveaux ou des sites intracellulaires d'expression d'un produit génique exprimé à partir d'une séquence génique qui code les résidus 146-163 de la séquence de la pp32r1, codée par la séquence de la figure 2.

10. Procédé selon la revendication 9, dans lequel le produit génique est un ARNm.

11. Procédé selon la revendication 10, dans lequel l'ARNm est extrait de l'échantillon et quantifié.

12. Procédé selon la revendication 10, dans lequel le niveau de l'ARNm est déterminé par une hybridation *in situ* à une coupe de l'échantillon de tissu.

13. Procédé selon la revendication 10, dans lequel l'ARNm est quantifié par une amplification en chaîne par polymérase.

14. Procédé selon la revendication 9, dans lequel le produit génique est une protéine.

15. Procédé selon la revendication 14, dans lequel le procédé comprend en outre la mise en réaction de l'échantillon avec un anticorps qui se lie spécifiquement à un polypeptide consistant en la séquence de la pp32r1, codée par la séquence de la figure 2, mais qui ne se lie pas spécifiquement à un polypeptide consistant en la séquence de la pp32 ou de la pp32r2, codée par la séquence de la figure 5, ou avec un anticorps qui se lie spécifiquement à un polypeptide consistant en la séquence de la pp32r2, mais qui ne se lie pas spécifiquement à un polypeptide consistant en la séquence de la pp32 ou de la pp32r1.

16. Procédé selon la revendication 9, dans lequel le tissu est un carcinome.

17. Procédé selon la revendication 9, dans lequel le tissu est un carcinome ou un sarcome d'un tissu choisi dans le groupe consistant en des tissus épithéliaux, lymphoïdes, hématopoïétiques, mésenchymateux, du système nerveux central et du système nerveux périphérique.

18. Procédé selon la revendication 17, dans lequel le tissu est choisi dans le groupe consistant en un cancer du côlon, un cancer de la prostate et un lymphome non hodgkinien.

19. Anticorps qui se lie spécifiquement à un polypeptide consistant en la séquence de la pp32r1, codée par la séquence de la figure 2, ou de la pp32r2, codée par la séquence de la figure 5, mais qui ne se lie pas spécifiquement à un polypeptide consistant en la séquence de la pp32.

20. Anticorps selon la revendication 19, dans lequel l'anticorps est un anticorps monoclonal.
